# EUROPEAN PATENT APPLICATION

(11) **EP 4 471 054 A2**
(43) Date of publication of application: **04.12.2024**
(21) Application number: 23746487.0
(22) Date of filing: 30.01.2023
(51) Int. Cl.: C07K 14/775, C12N 15/12, A61P 25/28, A61P 35/00

(54) **C-TERMINAL FRAGMENT OF APOLIPOPROTEIN E AND APPLICATION THEREOF IN INHIBITING ?-SECRETASE ACTIVITY**

(30) Priority: 30.01.2022 CN 202210113320
(71) Applicant: Synphatec (Shanghai) Biopharmaceutical Technology Co., Ltd., Shanghai 201204 (CN)
(72) Inventor: CHEN, Yelin, Shanghai 200032 (CN); HOU, Xianglong, Shanghai 200032 (CN); GENG, Yang, Shanghai 200032 (CN); ZHANG, Xuexin, Shanghai 200032 (CN)
(74) Representative: Impact Intellectual Property LLP
(86) International application number: PCT/CN2023/073849
(87) International publication number: WO 2023/143610

(57) **Abstract**

The present invention relates to a C-terminal fragment (such as ApoE CT) of apolipoprotein E (ApoE) and modified ApoE2 and an application of the fragment in inhibiting γ-secretase activity. In one aspect, the present invention provides an isolated ApoE construct, which contains an ApoE polypeptide, the ApoE polypeptide: (1) contains a fragment from an amino acid sequence as shown by SEQ ID NO: 12, a first amino acid residue at a N-terminal of the fragment being an amino acid residue at any position between positions 215-221 of SEQ ID NO: 12, and a last amino acid residue at a C-terminal of the fragment being an amino acid residue at any position between positions 274-299 of SEQ ID NO: 12; or (2) the amino acid sequence has at least about 70% sequence identity with the fragment of (1), and retains at least about 50% of inhibition of the fragment of (1) against γ-secretase enzymatic digestion activity. The present invention further provides an encoding sequence, nucleic acid construct, host cell, construction method, pharmaceutical composition, and application of a protein portion of the ApoE construct.

## Description

### Cross References to Related Applications

This patent application claims priority to Chinese Patent application No. CN202210113320.2 filed on January 30, 2022, which is incorporated herein in its entirety by reference.

### References to Electronic Sequence Listings

The contents of the Electronic Sequence Listing (217334 IPCWO.xml; Size: 92,558 bytes; Date Created: January 30, 2023) are hereby incorporated by reference in their entirety.

### TECHNICAL FIELD

The present description relates to the C-terminal fragment of apolipoprotein E (ApoE) and application thereof in inhibiting γ-secretase activity.

### BACKGROUND

γ-secretase is a cleavage enzyme for more than 100 type I transmembrane proteins and is involved in a variety of biological processes. Abnormal differentiation of T-cells and B-cells, hemorrhagic diarrhea, skin and hair defects, Alzheimer's disease (AD), etc. are all associated with the abnormality of γ-secretase. γ-secretase is a complex of four proteins: NCT, APH1, PEN2 and presenilin1 (PS1) or presenilin2 (PS2). The center of γ-secretase enzymatic digestion activity is PS1 or PS2. Now it has been found that a variety of PS1 and PS2 mutations can lead to AD, and both PS1 and PS2 are pathogenic genes of AD, therefore γ-secretase is a very important drug target, especially for the treatment of AD.

AD is the most common neurodegenerative disease in humans. AD patients have symptoms such as progressive memory impairment, cognitive dysfunction, personality changes, and language barriers, which further develop into loss of self-care ability and eventually death. China has the largest number of AD patients in the world. As of 2019, there are more than 10 million AD patients in China, showing an increasing trend every year. People over the age of 80 have an over 40% chance of developing AD. And the cost of treating AD is very high, causing great burden to families and society.

In the past few decades, the research on the pathogenesis of AD has made great progress, and the Aβ cascade hypothesis has been reported. This hypothesis holds that the excessive production and abnormal aggregation of Aβ in the brain is the initial cause of a series of serious and slow lesions, causing a series of abnormal biological processes in the brain, leading to synaptic degeneration on neurons and neuron death. In the brain, amyloid precursor protein (APP) is mainly expressed by neurons, and Aβ is produced by sequentially cutting APP by β-secretase and γ-secretase, and the generated Aβ is released outside the cell. This process is called the amyloidogenic pathway of APP. Under normal physiological conditions, most of the Aβ produced in the brain is soluble. However, after the lesion occurs, a large amount of Aβ in the brain accumulates abnormally, forming amyloid plaques deposited around neurons, causing damages to neurons and brain tissue failure, and eventually leading to AD. In addition, abnormal aggregation and precipitation of amyloid protein in cerebral blood vessels can cause amyloid cerebrovascular disease. In addition to being cleaved by β-secretase and γ-secretase to generate toxic Aβ, APP can also be cleaved sequentially by α-secretase and γ-secretase, which is the non-amyloidogenic pathway. Since the cleavage site of α-secretase is located inside Aβ, the cleavage product of α-cleavage will not generate Aβ, and the cleaved fragment is called α-CTF, which has no neurotoxicity and will not cause AD. A-cleavage and β-cleavage compete with each other, where increasing the cleavage by α-secretase will reduce the production of Aβ; and similarly, reducing the cleavage by γ-secretase will reduce the production of Aβ, slow down the formation of amyloid plaques, and eventually slow down or even prevent the occurrence of AD and amyloid cerebrovascular disease. A variety of medicaments targeting γ-secretase have been tested in clinical trials.

ApoE is a protein widely secreted in the human body and can be co-expressed with APP in neurons in the brain. There are three isomers of human ApoE: ApoE2, ApoE3, and ApoE4, which are highly similar in structure and function, and can be roughly divided into three functional domains: the N-terminal domain comprising receptor binding sites, the C-terminal domain comprising lipid binding sites, and the intermediate flexible hinge region connecting the two functional domains. ApoE2, ApoE3 and ApoE4 differ by only two amino acid residues in the N-terminal domain, and have identical amino acid sequences in the C-terminal domain. ApoE2 has a protective effect and can reduce the risk of AD; ApoE4 is the most important risk factor for AD, which can not only significantly increase the risk of AD, but also significantly advance the age of onset of AD and aggravate AD.

So far, it is still unclear how ApoE2 and ApoE4 alter the risk of AD.

### SUMMARY

In one aspect, the present description provides an isolated ApoE construct, which comprises an ApoE polypeptide, wherein the ApoE polypeptide: (1) comprises a fragment of an amino acid sequence as shown by SEQ ID NO: 12, the first amino acid residue at the N-terminus of the fragment (or the ApoE polypeptide) being an amino acid residue at any position of positions 215-221 of SEQ ID NO: 12, and the last amino acid residue at the C-terminus of the fragment (or the ApoE polypeptide) being an amino acid residue at any position of positions 274-299 (e.g., 279-299) of SEQ ID NO: 12; or (2) is of a amino acid sequence having at least about 70% (e.g., at least about 75%, 80%, 85%, 90%, 95%, 96%, 97%, 98%, 99% or higher) identity with the fragment of (1), and retains at least about 50% (e.g., at least about 60%, 70%, 80%, 85%, 90%, 95%, 96%, 97%, 98%, 99%, 100%, 1.5 times, 2 times, 5 times, 10 times or more) of inhibition of the fragment of (1) against γ-secretase enzymatic digestion activity.

In some embodiments of the isolated ApoE construct according to any of the above, the ApoE polypeptide or the fragment of the amino acid sequence as shown by SEQ ID NO: 12 of (1) comprises (or consists of, or substantially consists of) any of the following sequences: (i) the amino acid sequence of amino acid residues of positions 215-299 of SEQ ID NO: 12; (ii) the amino acid sequence of amino acid residues of positions 216-299 of SEQ ID NO: 12; (iii) the amino acid sequence of amino acid residues of positions 217-299 of SEQ ID NO: 12; (iv) the amino acid sequence of amino acid residues of positions 218-299 of SEQ ID NO: 12; (v) the amino acid sequence of amino acid residues of positions 219-299 of SEQ ID NO: 12; (vi) the amino acid sequence of amino acid residues of positions 220-299 of SEQ ID NO: 12; (vii) the amino acid sequence of amino acid residues of positions 221-299 of SEQ ID NO: 12; (viii) the amino acid sequence of amino acid residues of positions 216-294 of SEQ ID NO: 12; (ix) the amino acid sequence of amino acid residues of positions 216-289 of SEQ ID NO: 12; (x) the amino acid sequence of amino acid residues of positions 219-289 of SEQ ID NO: 12; (xi) the amino acid sequence of amino acid residues of positions 219-288 of SEQ ID NO: 12; (xii) the amino acid sequence of amino acid residues of positions 219-287 of SEQ ID NO: 12; (xiii) the amino acid sequence of amino acid residues of positions 219-286 of SEQ ID NO: 12; (xiv) the amino acid sequence of amino acid residues of positions 219-285 of SEQ ID NO: 12; (xv) the amino acid sequence of amino acid residues of positions 219-284 of SEQ ID NO: 12; (xvi) the amino acid sequence of amino acid residues of positions 219-279 of SEQ ID NO: 12; (xvii) the amino acid sequence of amino acid residues of positions 219-274 of SEQ ID NO: 12; (xviii) the amino acid sequence of amino acid residues of positions 220-274 of SEQ ID NO: 12; (xix) the amino acid sequence of amino acid residues of positions 220-279 of SEQ ID NO: 12; or (xx) the amino acid sequence of amino acid residues of positions 221-274 of SEQ ID NO: 12.

In some embodiments of the isolated ApoE construct according to any of the above, the ApoE polypeptide or the fragment of the amino acid sequence as shown by SEQ ID NO: 12 of (1) comprises (or consists of, or substantially consists of) any amino acid sequence of SEQ ID NOs: 13, 18-34, 78 and 87. In some embodiments, the ApoE polypeptide or the fragment of the amino acid sequence as shown by SEQ ID NO: 12 of (1) comprises (or consists of, or essentially consists of) any amino acid sequence of SEQ ID NOs: 31-34, 78 and 87 (e.g., SEQ ID NO: 33).

In some embodiments of the isolated ApoE construct according to any of the above, the ApoE polypeptide comprises (or consists of, or essentially consists of) an amino acid sequence that has at least about 70% (e.g., at least about 75%, 80%, 85%, 90%, 95%, 96%, 97%, 98%, 99% or more) sequence identity with any amino acid sequence of SEQ ID NOs: 13, 18-34, 78 and 87. In some embodiments, the ApoE polypeptide comprises a sequence from a non-human primate, such as a sequence that has high homology and conservation with any of the amino acid sequences of SEQ ID NOs: 13, 18-34, 78 and 87.

In some embodiments of the isolated ApoE construct according to any of the above, the ApoE polypeptide comprises mutations at one or more amino acid positions compared to the amino acid sequence as shown by SEQ ID NO: 12 (e.g., addition, deletion, or substitution): R226, D227, R228, L229, D230, Q235, E238, V239, R240, K242, E244, E245, Q246, A247, Q248, Q249, 1250, R251, L252, Q253, A254, E255, Q275, V280, V287, T289, S290, A291, P293, V294, P295, S296, D297, N298, H299, R251+T289, R251+T289+A291, S290+P293+V294+P295+S296+D297+N298+H299, R226+D227+R228+L229+D230, E238+V239+R240+K242, or E244+E245+Q246+A247+Q248+Q249+I250+R251+L252+Q253+A254. In some embodiments, the ApoE polypeptide comprises mutations at one or more amino acid positions compared to the amino acid sequence as shown by SEQ ID NO: 12: R226A, D227A, R228A, L229A, D230A, Q235A, E238A, V239A, R240A, K242A, E244del, E245del, Q246del, A247del, Q248del, Q249del, I250del, R251S, L252del, Q253del, A254del, E255A, Q275A, V280A, V287A, T289A, S290A, A291T, P293A, V294A, P295A, S296A, D297A, N298A , H299A, R251A+T289A, R251S+T289A+A291T, S290A+P293A+V294A+P295A+S296A+D297A+N298A+H299A, R226A+D227A+R228A+L229A+D230A, E238A+V239A+R240A+K242A, or E244del+ E245del+Q246del+A247del+Q248del+Q249del+I250del+R251del+L252del+Q253del+A 254del.

In some embodiments of the isolated ApoE construct according to any of the above, the ApoE polypeptide comprises (or consists of, or essentially consists of) any one of the amino acid sequence of SEQ ID NOs: 46-52, 57-59, 62 and 76.

In some embodiments of the isolated ApoE construct according to any of the above, the ApoE construct further comprises an amino acid sequence of amino acid residues at positions 1-167 or 1-205 or 1-214 of SEQ ID NO: 12 at the N-terminus of the ApoE polypeptide, or a sequence that has at least about 70% (e.g. at least 75%, 80%, 85%, 90%, 95%, 96%, 97%, 98%, 99%, or higher) identity with amino acids at positions 1-167, or 1-205, or 1-214 of SEQ ID NO: 12. In some embodiments, the ApoE construct comprises (or consists of, or essentially consists of) the amino acid sequence as shown by SEQ ID NO: 60 or 61.

In some embodiments of the isolated ApoE construct according to any of the above, the ApoE construct further comprises a cell-penetrating peptide at the N-terminus or C-terminus of the ApoE polypeptide. In some embodiments, the cell-penetrating peptide comprises (or consists of, or essentially consists of) any amino acid sequence of SEQ ID NOs: 35-45, 63-65, and 84-86; for example, the cell-penetrating peptide comprises (or consists of, or essentially consists of) the amino acid sequence as shown by SEQ ID NO: 63 or 65. In some embodiments, the cell-penetrating peptide (such as the C-terminus) is linked to the ApoE polypeptide (such as the N-terminus), or the amino acid sequence comprising amino acid residues at positions 1-167, 1-205, or 1-214 of SEQ ID NO: 12 (e.g. N-terminal), or the sequence that has at least about 70% identity with amino acids at positions 1-167 or 1-205 or 1-214 of SEQ ID NO: 12 (e.g. N-terminal) by an adapter sequence.

In some embodiments of the isolated ApoE construct according to any of the above, the ApoE construct further comprises a signal peptide at the N-terminus of the ApoE polypeptide. In some embodiments, the signal peptide comprises (or consists of, or essentially consists of) any one of the amino acid sequences selected from SEQ ID NOs: 66-68.

In another aspect, the present description provides an isolated ApoE construct, the isolated ApoE construct comprising element (1): a cell-penetrating peptide, and element (2): the full-length sequence of ApoE2 (e.g., SEQ ID NO: 12) or the full-length sequence of ApoE3 (such as SEQ ID NO: 89) or a fragment at least comprising amino acid residues of positions 221-274 of SEQ ID NO: 12; wherein, the element (1) and the element (2) are directly linked by peptide bonds or linked by an adapter sequence. In some embodiments, the cell-penetrating peptide is selected from any one of the amino acid sequences of SEQ ID NOs: 35-45, 63-65 and 84-86, such as the amino acid sequence as shown by SEQ ID NO: 63 or 65. In some embodiments, the fragment comprising at least amino acid residues at positions 221-274 of SEQ ID NO: 12 of element (2) is selected from any one of the ApoE peptides described in the present description that comprises amino acid residues at positions 221-274 of SEQ ID NO: 12. In some embodiments, the amino acid sequence of the fragment comprising at least amino acid residues at positions 221-274 of SEQ ID NO: 12 as described in element (2), is as shown by any one of SEQ ID NOs: 13, 18-34, and 78. In some embodiments, the fragment comprising at least amino acid residues at positions 221-274 of SEQ ID NO: 12 of element (2) comprises the N-terminal domain (amino acid residues at positions 1-167 of SEQ ID NO: 12) and hinge region (amino acid residues at positions 168-205 of SEQ ID NO: 12) of ApoE2, and the C-terminus comprises at least a fragment of amino acid residues at positions 221-274 of SEQ ID NO: 12. In some embodiments, the element (2) comprises any one of the following sequences: amino acid residues of positions 1-274, amino acid residues of positions 1-279, amino acid residues of positions 1-280, amino acid residues of positions 1-281, amino acid residues of positions 1-282, amino acid residues of positions 1-283, amino acid residues of positions 1-284, amino acid residues of positions 1-285, amino acid residues of positions 1-286, amino acid residues of positions 1-287, amino acid residues of positions 1-288, amino acid residues of positions 1-289, amino acid residues of positions 1-290 , amino acid residues of positions 1-291, amino acid residues of positions 1-292, amino acid residues of positions 1-293, amino acid residues of positions 1-294, amino acid residues of positions 1-295, amino acid residues of positions 1-296, amino acid residues of positions 1-297, amino acid residues of positions 1-298, or amino acid residues of positions 1-299 of SEQ ID NO: 12.

The present description also provides an isolated nucleic acid and vector (for example, cloning vector or expression vector) encoding the protein portion of any one of the aforementioned isolated ApoE constructs, and a host cell expressing the protein portion of any one of the aforementioned isolated ApoE constructs, or a host cell comprising the nucleic acid or vector. In some embodiments, the vector comprises a neuron-specific promoter at the 5' end of the isolated nucleic acid. In some embodiments, the neuron-specific promoter is selected from the synapsin promoter, the thy-1 promoter, and the calmodulin-dependent protein kinase II-α promoter. In some embodiments, the vector is a recombinant AAV or lentivirus expression vector. In some embodiments, the host cell is a neuron (e.g., a human neuron, a non-human primate neuron, or a rodent neuron). In some embodiments, the ApoE construct comprises (or consists of, or essentially consists of) the full-length amino acid sequence of ApoE2 (e.g., SEQ ID NO: 12) or ApoE3 (e.g., SEQ ID NO: 89).

The present description also provides a pharmaceutical composition, which comprises (i) the isolated ApoE construct, isolated nucleic acid, vector, or host cell described in any embodiment herein, and (ii) a pharmaceutically acceptable carrier.

The present description also provides use of the isolated ApoE construct, the isolated nucleic acid, the vector, the host cell, or the pharmaceutical composition according to any of the embodiments herein in the manufacture of a medicament for treating or preventing a disease associated with γ-secretase enzymatic digestion activity in an individual (such as a human). The present description also provides a method for treating or preventing a disease associated with γ-secretase enzymatic digestion activity in an individual (such as a human), comprising administering to the individual an effective dose of the isolated ApoE construct, the isolated nucleic acid, the vector, the host cell or the pharmaceutical composition described in any embodiment herein. In some embodiments, the medicament or method inhibits the γ-secretase enzymatic digestion activity, thereby treating or preventing the disease associated with γ-secretase enzymatic digestion activity. In one or more embodiments, the disease is selected from the group consisting of: a neurodegenerative disease, tumor, cancer, inflammatory disease and renal disease. In one or more embodiments, the disease is selected from the group consisting of: Alzheimer's disease and related diseases, amyloid cerebrovascular disease, Down's syndrome, other neurodegenerative diseases associated with aging (such as frontotemporal dementia), head and neck cancers (such as head and neck squamous cell carcinoma and oral squamous cell carcinoma), breast cancer, liver cancer, pancreatic cancer (including metastatic pancreatic cancer), ovarian cancer, lung cancer (such as non-small cell lung cancer, lung adenocarcinoma and small cell lung cancer), glioma (e.g., malignant glioma), fibroma, lymphoma (e.g., B-cell lymphoma), osteosarcoma, gastric cancer, bladder cancer, asthma (e.g., allergic asthma), pneumonia, airway inflammation, acute kidney injury, clear cell renal cell carcinoma, renal fibrosis, and obstructive nephropathy.

### BRIEF DESCRIPTION OF DRAWINGS

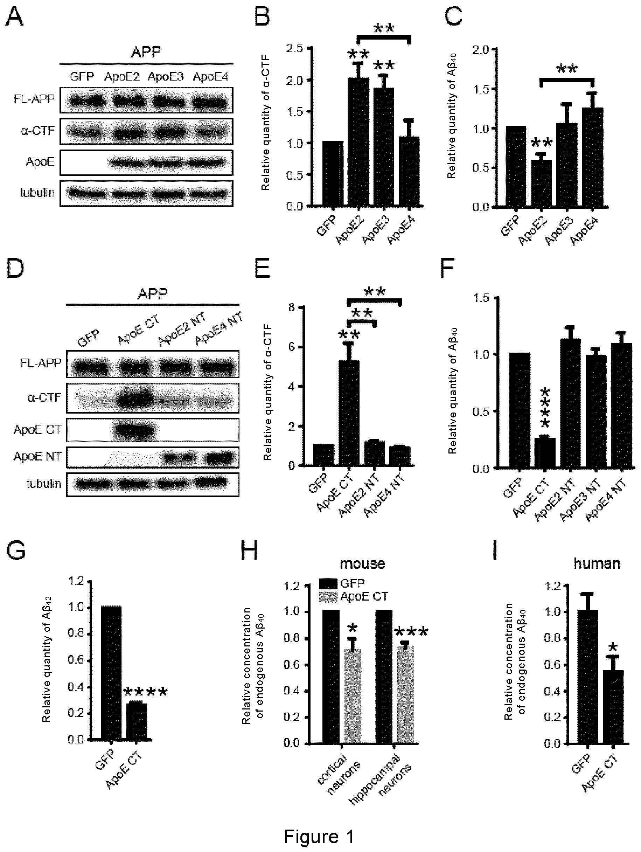

FIG. 1: ApoE attenuates the amyloidogenic pathway of APP and increases the metabolite of non-amyloidogenic pathway (α-CTF) through its C-terminal fragment (ApoE CT). Panels A and B: overexpression of ApoE2 and ApoE3 in HEK 293T cells can significantly increase α-CTF produced by co-expressed APP via non-amyloidogenic pathway (α cleavage), while ApoE4 cannot. Statistical results are in panel B, N=8, paired or one sample t-test. Panel C: total Aβ40 produced by co-expressed APP is reduced by transient transfection of overexpressed ApoE2, N=8, paired or one sample t-test. Panels D and E: Western blot experiments showed that overexpression of ApoE CT increased the α-CTF produced by co-expressed APP, while overexpression of the N-terminal fragment of ApoE2 or the N-terminal fragment of ApoE4 (ApoE2 NT, ApoE4 NT) did not change the α-CTF generated; the statistical results are in panel E, N=8, paired or one sample t-test. Panels F and G: overexpression of ApoE CT decreased Aβ40 and Aβ42 produced by APP, while overexpression of ApoE2 NT, ApoE3 NT or ApoE4 NT did not change Aβ, N=5, one sample t-test. Panels A-G: HEK 293T cells. Panel H: ApoE CT expressed using lentivirus in primary cultured neurons of mouse cerebral cortex and hippocampus can reduce the endogenous production of Aβ40 in neurons. The promoter is hSyn. N=7, one sample t-test. Panel I: ApoE CT expressed in human neurons produced from a pluripotent stem cell line using lentivirus also reduces endogenous Aβ40 produced by neurons. Promoter is hSyn, N=5, unpaired t-test. The APP, α-CTF and ApoE proteins were detected by Western blot, and Aβ was detected by ELISA.

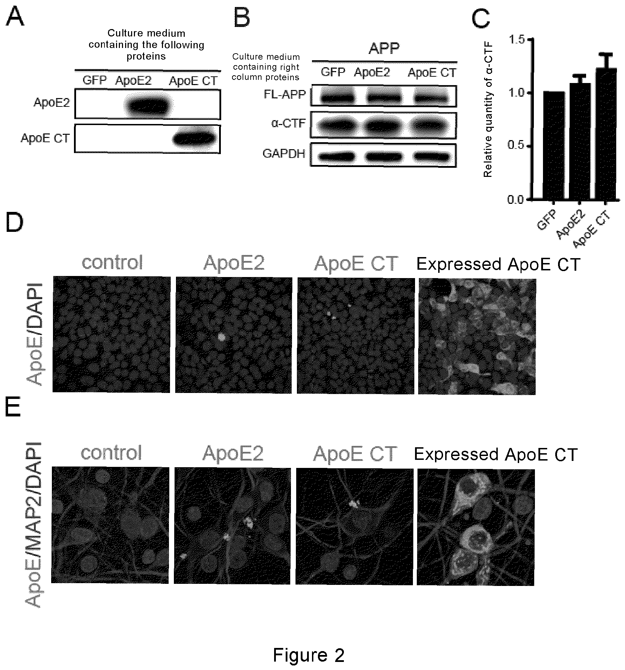

FIG. 2: ApoE2 and ApoE CT in culture do not increase the metabolite of non-amyloidogenic pathway in cells. Panel A: ApoE2 and ApoE CT proteins contained in culture medium were detected by Western blot. Panels B and C: co-incubating with cell culture medium comprising ApoE2 or ApoE CT protein does not change the α-CTF produced by APP in cells. The statistical results are in panel C, N=9, one sample t-test. Panel D: Immunofluorescence experiments showed that after HEK 293T cells were co-incubated with cell culture medium comprising ApoE2 or ApoE CT protein, ApoE2 or ApoE CT protein largely could not be detected inside the cells, and only a small amount was attached to the surface of some cells. However, ApoE CT transiently expressed in HEK 293T cells by liposomes exists in large quantities inside the cells. Panel E: Immunofluorescence experiments showed that after co-incubating with cell culture medium comprising ApoE2 or ApoE CT protein, ApoE2 or ApoE CT protein largely could not be detected inside the primary cultured neurons of mice, and only a small amount of ApoE was attached to the surface of some cells. However, ApoE CT expressed by lentivirus is abundantly present inside the neurons. MAP2 marks neurons and DAPI marks nuclei.

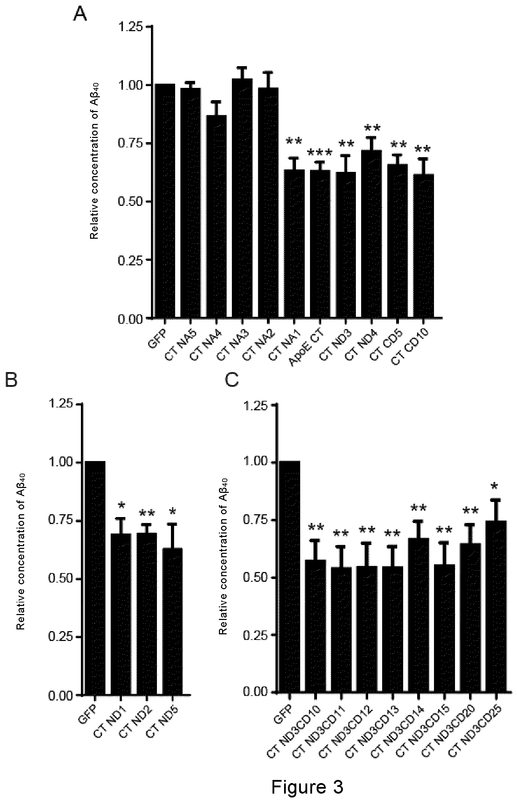

FIG. 3: ApoE CT reduces the core sequence of Aβ. Panels A, B and C: effects of overexpression of different ApoE CT mutants on production of Aβ by APP in HEK 293T cells. Panel A: CT NA1/2/3/4//5: 1, 2, 3, 4 or 5 amino acid residues of the original ApoE were added to the N-terminus of ApoE CT. CT ND3/4: 3 or 4 amino acid residues at the N-terminus of ApoE CT were deleted. CT CD5/10: 5 or 10 amino acid residues at the C-terminus of ApoE CT were deleted. The effect of different ApoE CT mutants on Aβ40 was measured by ELISA. Panel B: CT ND 1/2//5: 1, 2 or 5 amino acid residues at the N-terminus of ApoE CT were deleted. The effect of different ApoE CT mutants on Aβ40 was measured by ELISA. Panel C: CT ND3CD10/11/12/13/14/15/20/25: 10, 11, 12, 13, 14, 15, 20 or 25 amino acids at the C-terminus were deleted, in addition to the deletion of 3 amino acid residues at the N-terminus of ApoE CT. The effect of different ApoE CT mutants on Aβ40 was measured by ELISA. N=5 (panels A and B) or 7 (panel C), one sample t-test.

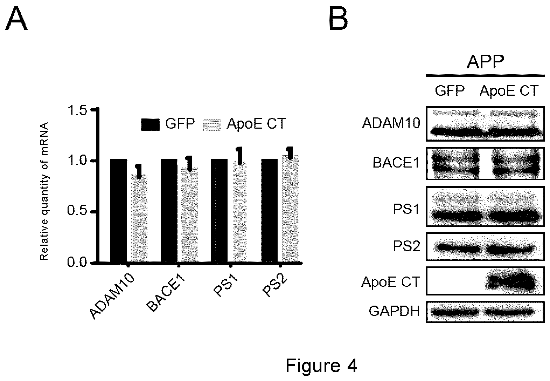

FIG. 4: ApoE CT does not change the expression level of APP secretase. Panel A: ApoE CT does not affect the mRNA expression of APP metabolizing enzymes. Panel B: ApoE CT does not affect the protein expression of APP metabolizing enzymes. ADAM10: α-secretase of APP; BACE1: β-secretase of APP; PS1 and PS2: core active subunit proteins of γ-secretase. mRNA and protein levels were detected by qRT-PCR and Western blot, respectively. Cells expressing GFP served as negative controls. N=9, one sample t-test.

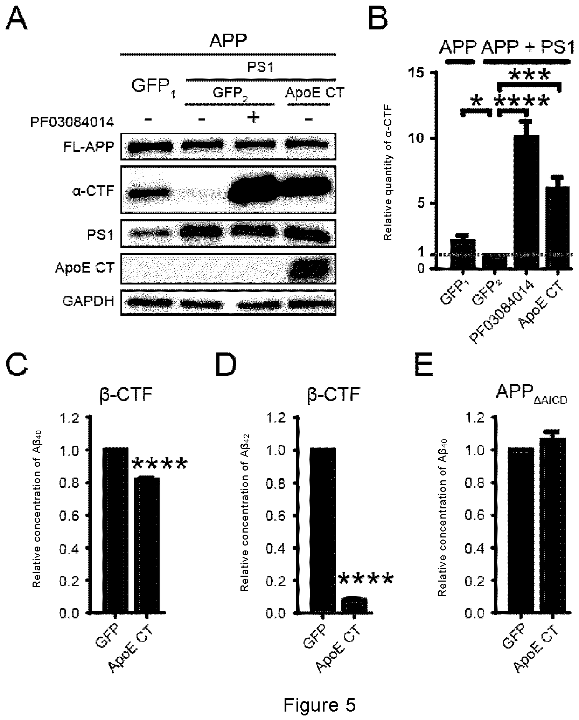

FIG. 5: ApoE CT mimics the effect of γ-secretase inhibitors on APP cleavage and inhibits γ-cleavage of β-CTF. Panels A and B: overexpression of γ-secretase active center protein PS1 can reduce the production of α-CTF, while expression of ApoE CT or treatment with 1 µM PF03084014 (γ-secretase inhibitor) can increase the production of α-CTF. Statistical results are in panel B, N=11, paired or one sample t-test. Panels C and D: overexpressed ApoE CT can inhibit the production of Aβ40 (N=4) and Aβ42 (N=6) from β-CTF, one sample t-test. Panel E: overexpressed ApoE CT does not affect Aβ40 produced by β-cleavage of APP_{ΔAICD}, N=4, one sample t-test. Protein was detected by Western blot, and Aβ was detected by ELISA.

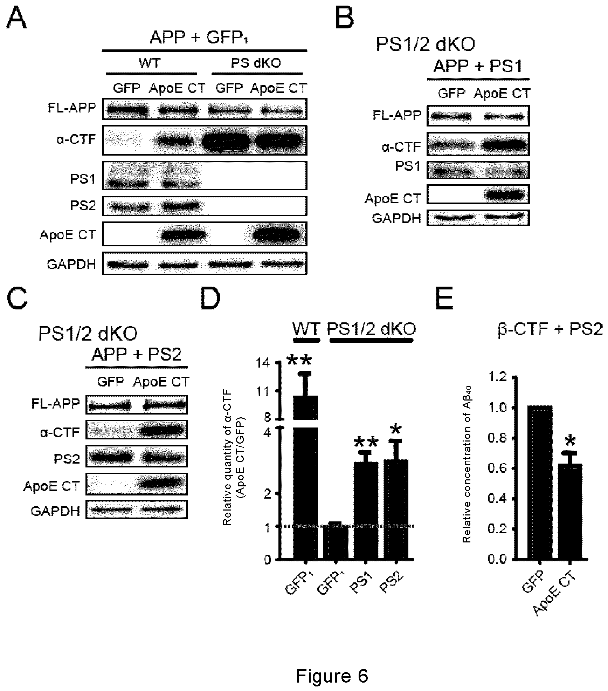

FIG. 6: the effect of ApoE CT on APP cleavage requires the presence of γ-secretase. Panels A and D: In HEK 293T cells in which the γ-secretase active center proteins PS1 and PS2 were knocked out (PS1/2 dKO), α-CTF was greatly increased. At this time, the overexpressed ApoE CT did not further increase α-CTF. The statistical results are in panel D (the two columns labeled GFP₁). Panels B, C, and D: In PS1/2 dKO cells with rescued expression of PS1 or PS2, overexpressed ApoE CT increased α-CTF again. The statistical results are in panel D, N=8, paired t-test. Panel E: In PS1/2 dKO cells with rescued expression of PS2, overexpression of ApoE CT can reduce Aβ40 generated from APP, N=4, one sample t-test. Protein was detected by Western blot, and Aβ40 was detected by ELISA.

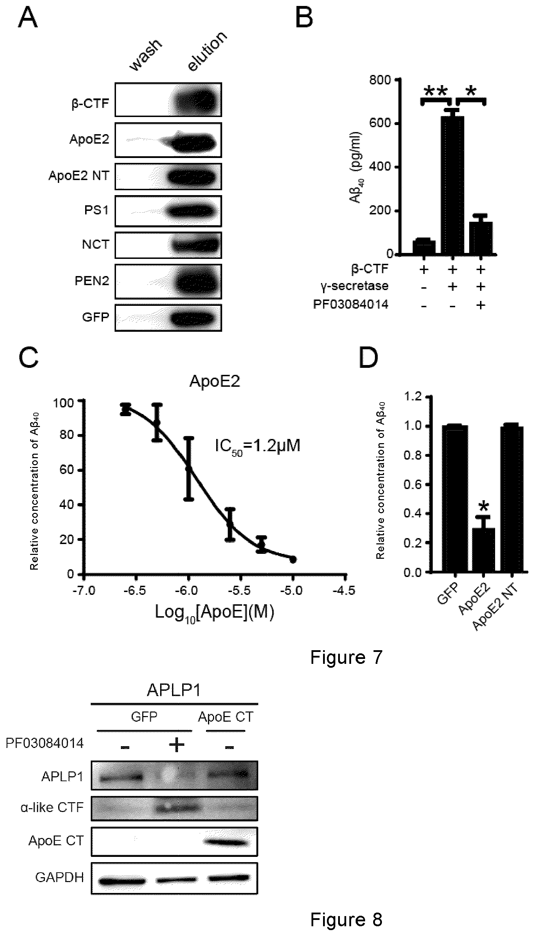

FIG. 7: ApoE2 purified in vitro directly inhibits γ-cleavage of APP in a concentration-dependent manner. Panel A: Western blot showed isolated and purified β-CTF, ApoE2, ApoE2 NT, PS1, NCT, PEN2 and GFP. Panel B: In the purified system, the reaction of γ-secretase cleaving β-CTF to generate Aβ40 can be inhibited by 10 µM PF03084014 (γ-secretase inhibitor), N=3, paired t-tests. Panel C: purified ApoE2 inhibits cleavage of β-CTF by γ-secretase in a dose-dependent manner, reducing Aβ40 production. The half-inhibitory concentration of ApoE2 was about 1.2 µM, N=3. Panel D: purified ApoE2 can inhibit Aβ40 generated from β-CTF after γ-cleavage, but ApoE2 NT cannot inhibit cleavage of β-CTF by γ-secretase, N=3, paired t-tests. Protein was detected by Western blot, and Aβ40 was detected by ELISA.
FIG. 8: ApoE CT does not inhibit γ-cleavage of APLP1. 1 µM PF03084014 (γ-secretase inhibitor) inhibited the γ-cleavage of exogenously expressed APLP1 in HEK 293T cells, and increased the α-like CTF generated by APLP1 through α-cleavage; however, ApoE CT did not increase the α-like CTF produced by APLP1.

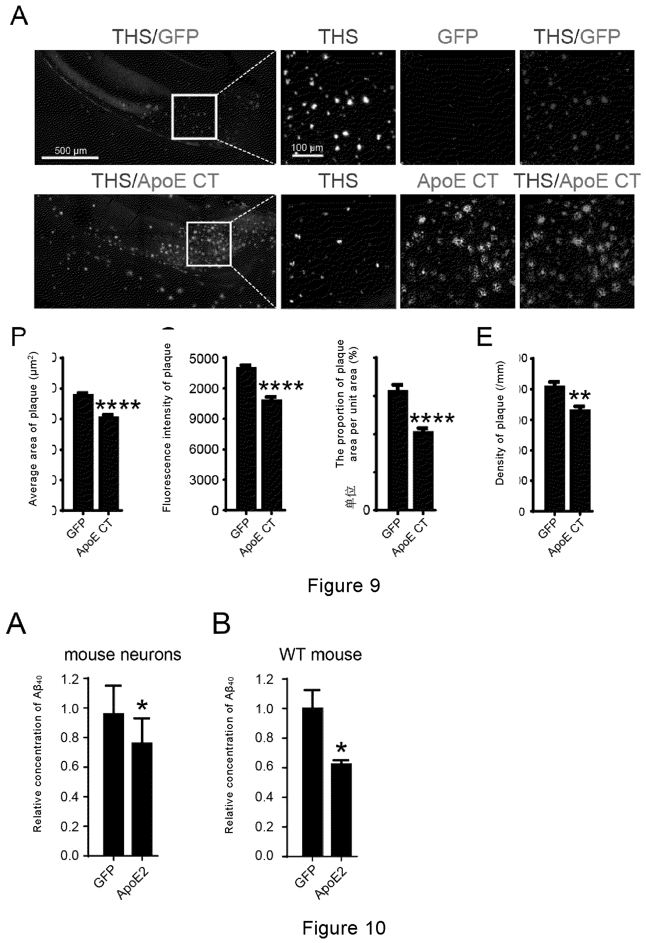

FIG. 9: overexpressed ApoE CT in neurons reduces amyloid plaques in the brain of a 5xFAD mouse model. Panel A: representative images of co-staining of amyloid plaques (THS markers) and ApoE CT or GFP in the subiculum region of the 5xFAD mouse model. ApoE CT was expressed in neurons using the adeno-associated virus (AAV) expression system in 5xFAD mice (injection at one month old, analysis at seven months old); GFP was used as a control, and the promoter was hSyn (human synapsin). The nucleic acid sequence encoding the polypeptide is preceded by a signal peptide encoding sequence. Statistics are in panels B, C, D and E. Panel B: The average area of each plaque in the subiculum region of mice expressing ApoE CT is smaller (panel B). The average fluorescence intensity is weaker (panel C), and the proportion of the total area is smaller (panel D). The density of plaques is also lower (panel E). THS is used to label amyloid plaques. N=33, unpaired t-test.
FIG. 10: ApoE2 expressed in neurons can inhibit the endogenous production of Aβ40 in mouse neurons. Panel A: ApoE2 expressed using a lentivirus in primary cultured neurons of mice could reduce the endogenous Aβ40 produced by mouse neurons, and GFP was used as a control. N=7, paired t-test. Panel B: ApoE2 expressed by AAV in the brain of wild-type mice (WT mouse) reduced endogenous Aβ40 in the brain of mice, and GFP was used as a control. N=5, unpaired t-test. The hsyn promoter was used to ensure neuron-specific expression. Aβ detected by ELISA.

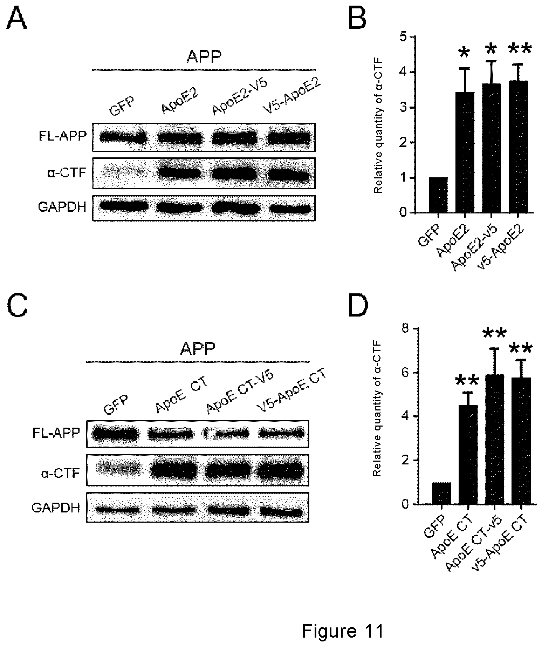

FIG. 11: ApoE2 or ApoE CT with tagged proteins added at both ends still have the activity of regulating APP cleavage. Panels A and B: ApoE2-V5 (C-terminus tagged protein) and V5-ApoE2 (N-terminus tagged protein), both of which comprise tagged protein V5, can significantly increase the α-CTF produced from APP when overexpressed in HEK 293T cells both. Statistics are in panel B. N=6, one sample t-test. Panels C and D: ApoE CT-V5 and V5-ApoE CT with tagged protein V5 at the C-terminus or N-terminus of ApoE CT can significantly increase the α-CTF produced from APP when overexpressed in HEK 293T cells. Statistical results are in panel D. N=6, one sample t-test. APP and its cleavage fragments were detected by Western blot.

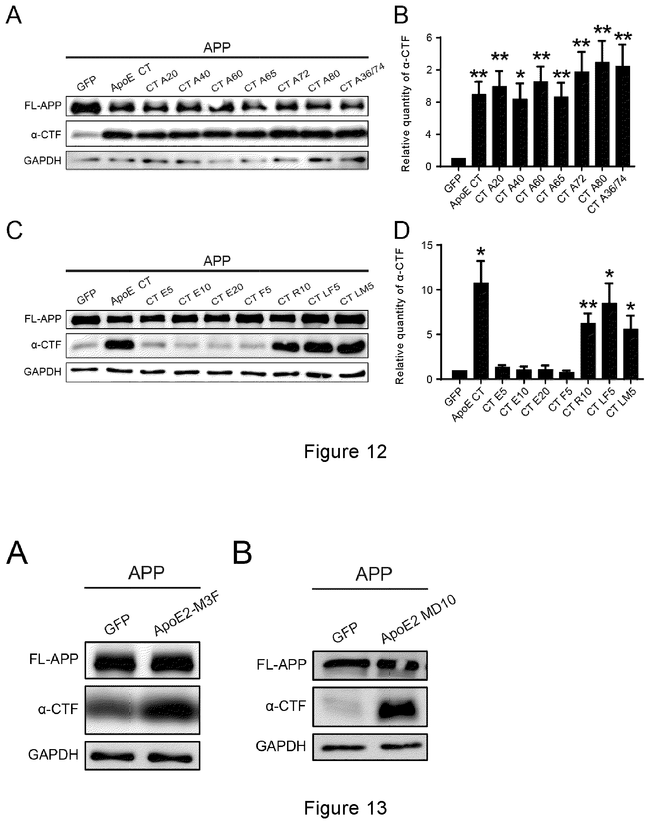

FIG. 12: mutants of ApoE CT can still regulate the cleavage of APP. Panels A and B: ApoE CT, single or double mutations of ApoE CT, CT A20, CT A40, CT A60, CT A65, CT A72, CT A80 and CT A36/74 all significantly increased α-CTF when overexpressed in HEK 293T cells. Statistics are in panel B. N=6, one sample t-test. Panels C and D: similar to ApoE CT, overexpression of CT R10, CT LF5 and CT LM5 with multiple mutations can also significantly increase α-CTF in HEK 293T cells; but CT E5, CT E10, CT E20 and CT F5 failed to increase the activity of α-CTF. Statistics are in panel D. N=4, one sample t-test. Protein was detected by Western blot.
FIG. 13: ApoE2 and ApoE2 with addition or deletion of amino acids can still regulate APP metabolism. Panel A: ApoE2-M3F co-expressed with APP increased α-CTF in HEK 293T cells. Panel B: ApoE2 MD10 co-expressed with APP also increased α-CTF in HEK 293T cells.

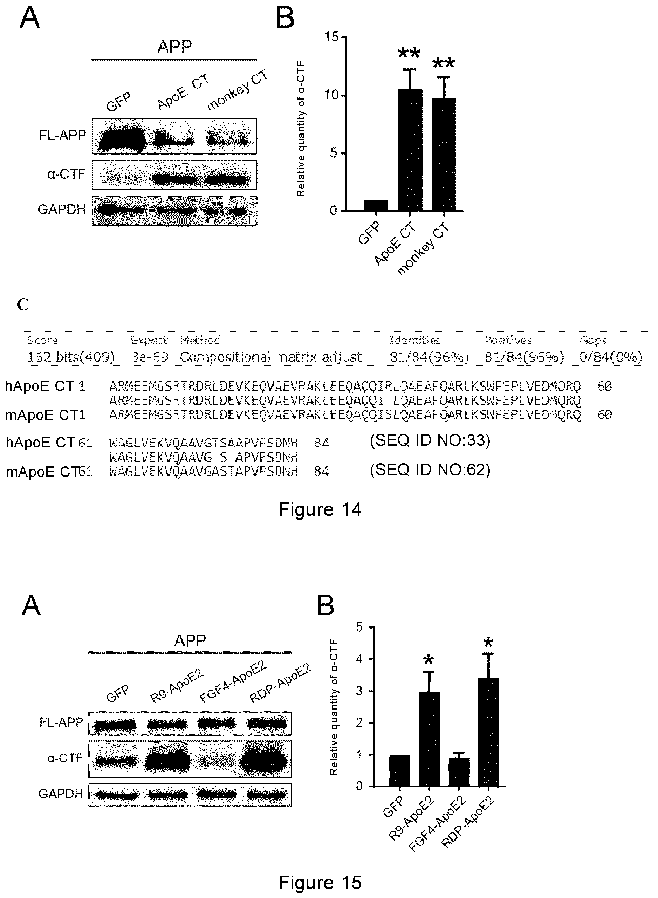

FIG. 14: non-human ApoE CT also regulates the cleavage of APP. Panels A and B: overexpression of ApoE CT from a non-human primate with a high sequence similarity to human ApoE CT significantly increased α-CTF in HEK 293T cells, and its activity was identical to that of human ApoE CT. Statistics are in panel B. N=6, one sample t-test. Protein was detected by Western blot. The human and monkey ApoE CT sequences are aligned in panel C.
FIG. 15: ApoE linked to a cell-penetrating peptide still has the activity of regulating APP cleavage. Panels A and B: APP was co-expressed with R9-ApoE2, FGF4-ApoE2 or RDP-ApoE2 in HEK 293T cells, and GFP was used as a negative control. Co-expression of R9-ApoE2 and RDP-ApoE2 significantly increased α-CTF. Statistics are in panel B. N=5, one sample t-test. Protein was detected by Western blot.

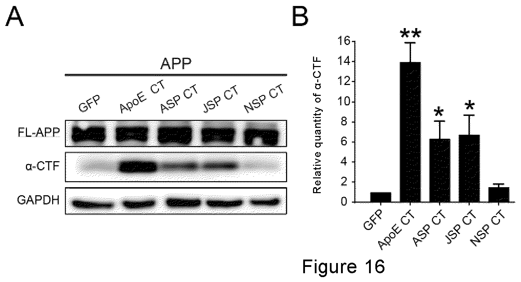

FIG. 16: ApoE CT expressed in cells requires a signal peptide to have the activity of regulating APP cleavage. Panels A and B: ApoE CT mutants with apolipoprotein A1 signal peptide at the N-terminus (ASP CT) and ApoE CT mutants with apolipoprotein J signal peptide (JSP CT) still can significantly increase α-CTF when overexpressed in HEK 293T cells. Expression of NSP CT without a signal peptide in cells could not regulate APP cleavage or increase α-CTF. Statistics are in panel B. N=6, one sample t-test. Protein was detected by Western blot.

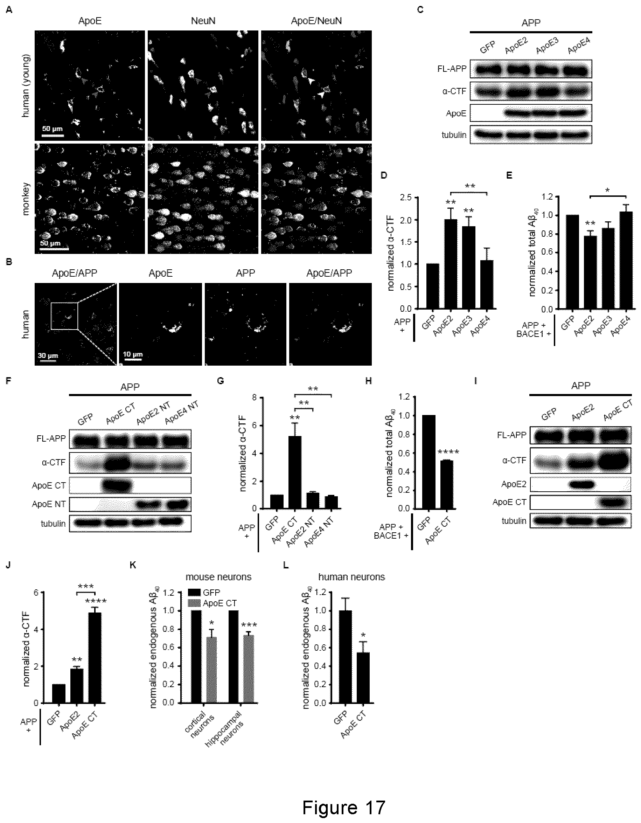

FIG. 17: ApoE2 increases α-CTF and reduces Aβ production through its C-terminal region. Panel A: representative images of co-immunofluorescence staining for ApoE and NeuN (neuronal markers) in human (top) or monkey (bottom) cortex. Arrows indicate ApoE-positive neurons. Panel B: representative images of co-immunofluorescence staining for ApoE and APP in human cerebral cortex. Panel C and D: Western blot and corresponding statistical analysis of full-length APP (FL-APP), α-CTF and ApoE signals of N2A cells transiently co-expressing APP and GFP, ApoE2, ApoE3 or ApoE4. N=8, one sample t-test. Panel E: normalized statistical analysis of Aβ40 of HEK 293T cells transiently co-expressing APP, BACE1 and GFP, ApoE2, ApoE3 or ApoE4. N=8, one sample t-test. Panel F and G: Western blot and corresponding statistical analysis of full-length APP (FL-APP), α-CTF and ApoE signals of N2A cells transiently co-expressing APP and GFP, ApoE CT, ApoE2 NT or ApoE4 NT. N=8, one sample t-test. Panel H: normalized statistical analysis of Aβ40 of N2A cells transiently co-expressing APP, BACE1 and GFP, ApoE CT. N=4, one sample t-test. Panel I and J: Western blot and corresponding statistical analysis of full-length APP (FL-APP), α-CTF and ApoE signals of N2A cells transiently co-expressing APP and GFP, ApoE2 or ApoE CT. N=6, one sample t-test. Panel K: normalized statistical analysis of Aβ40 in mouse cortical neurons or hippocampal neurons transiently expressing GFP or ApoE CT using lentivirus. The promoter is hSyn. N=7, one sample t-test. Panel L: normalized statistical analysis of Aβ40 in human neurons produced from human pluripotent stem cells transiently expressing GFP or ApoE CT using a lentivirus. The promoter is hSyn. N=5, unpaired t-test.

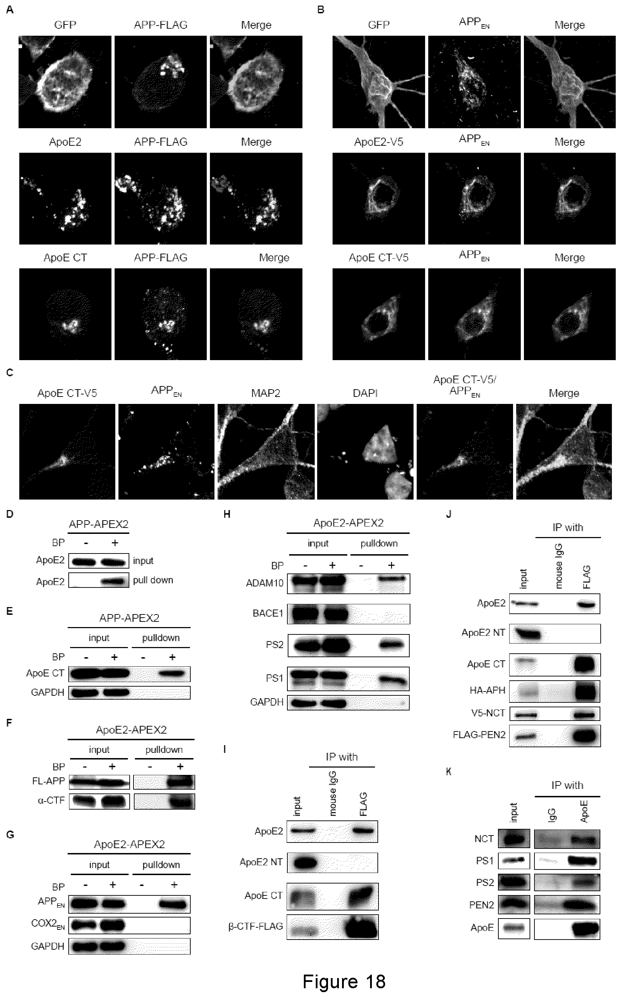

FIG. 18: ApoE CT interacts with APP and γ-secretase. Panel A: representative images of immunofluorescence staining for ApoE and APP of N2A cells transiently co-expressing APP-FLAG and GFP, ApoE2 or ApoE CT. Panel B: representative images of immunofluorescent staining of ApoE and endogenously expressed APP in primary cultured neurons transiently expressing GFP, ApoE2-V5, or ApoE CT-V5. Panel C: representative images of immunofluorescence staining of ApoE CT and endogenously expressed APP in human neurons produced from human pluripotent stem cells transiently expressing ApoE CT-V5. MAP2 staining indicates neurons and DAPI staining indicates nuclei. Panel D: Western blot detection of APP-APEX2-labeled ApoE2 co-expressed in HEK 293T cells. Panel E: Western blot detection of APP-APEX2-labeled ApoE CT co-expressed in HEK 293T cells. Panel F: Western blot detection of ApoE2-APEX2-labeled APP co-expressed in HEK 293T cells. Panel G: Western blot detection of endogenously expressed APP in ApoE-APEX2-labeled neurons expressed in mouse neuronal cells and negative control COX2. Panel H: Western blot detection of ApoE2-APEX2-labeled ADAM10, BACE1, PS1 andPS2 transiently co-expressed with ADAM10, BACE1, PS1 or PS2, respectively, in HEK 293T cells. Panel I: co-immunoprecipitation detection of ApoE interacting with β-CTF-FLAG transiently co-expressed with ApoE2, ApoE2 NT or ApoE CT respectively in HEK 293T cells. Panel J: co-immunoprecipitation detection of ApoE interacting with γ-secretase (HA-APH, V5-NCT, FLAG-PEN2) transiently co-expressed with ApoE2, ApoE2 NT or ApoE CT respectively in HEK 293T cells. Panel K: co-immunoprecipitation detection of the interaction of endogenous γ-secretase (NCT, PS1, PS2 and PEN2) and endogenous ApoE in human brain tissue.

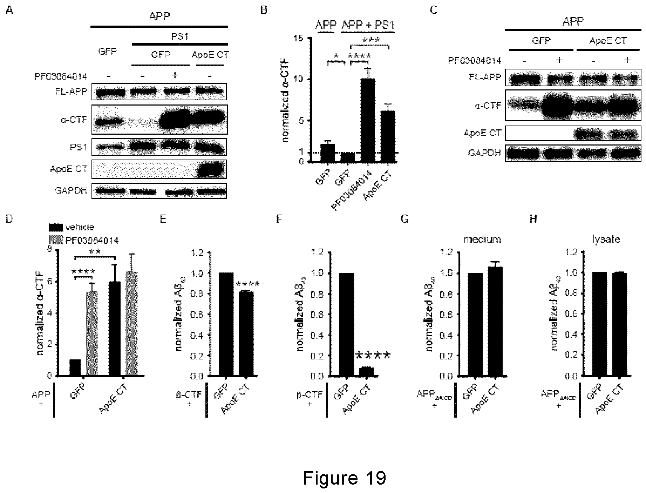

FIG. 19: ApoE CT increased α-CTF and decreased Aβ by inhibiting γ-cleavage of APP. Panels A and B: Western blot detection and statistical analysis of α-CTF production by APP under the following expression or drug treatment conditions in HEK 293T cells: transient expression of APP and GFP (lane 1); with APP and PS1 co-expressed, with or without treatment using 1 uM γ-secretase inhibitor PF03084014 (lane 2, lane 3); co-expression of APP, PS1 and ApoE CT (lane 4). N=11. Panels C and D, Western blot detection and statistical analysis of the effects of GFP or ApoE CT on the production of α-CTF from co-expressed APP in HEK 293T cells when treated with 1 uM γ-secretase inhibitor PF03084014, and the control group was not treated with inhibitors. N=7. Panel E: ELISA detection of Aβ40 produced in HEK 293T cells transiently co-expressing β-CTF and GFP, or β-CTF and ApoE CT, respectively. N=4. Panel F: ELISA detection of Aβ40 produced in HEK 293T cells transiently co-expressing β-CTF and GFP, or β-CTF and ApoE CT, respectively. N=6. Panel G and Panel H: ELISA detection results of Aβ40 produced by HEK 293T co-expressing APP_{ΔAICD} and GFP, or APP_{ΔAICD} and ApoE CT, respectively. Panel G, cell culture medium. Panel H, cell lysates. Panels B, D, E, F, G and H, one sample t-test.

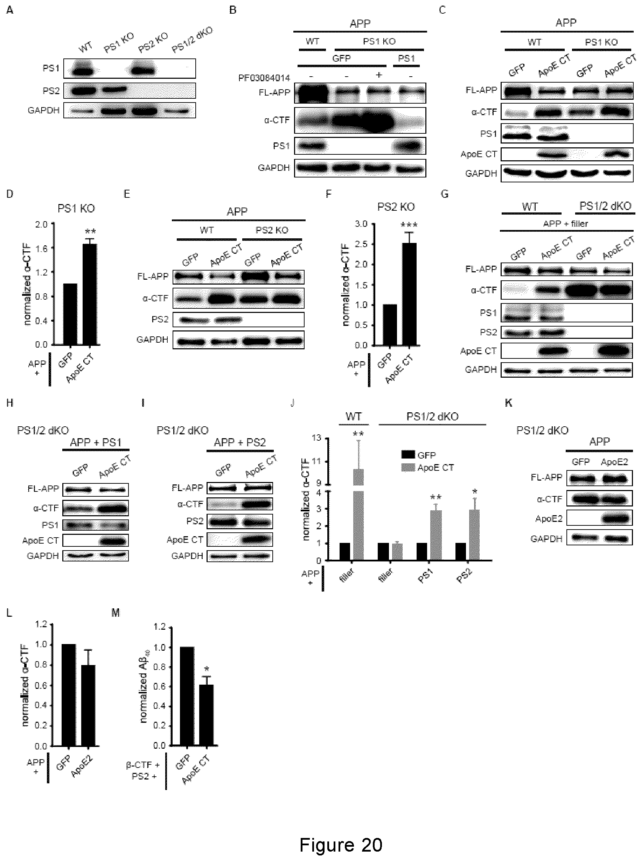

FIG. 20: γ-secretase is essential for ApoE CT to regulate the metabolism of APP. Panel A: Western blot detection of protein expression of PS1 and PS2 in wild-type (WT), PS1 or PS2 single knockout (PS1 KO, PS2 KO), or PS1 and PS2 double knockout (PS1/2 dKO) HEK 293T cell lines. Panel B: APP expression in wild-type HEK 293T cell line (lane 1); APP expression in PS1 single knockout HEK 293T cell line (lane 2); APP expression in PS1 single knockout HEK 293T cell line treated with 1 uM γ-secretase inhibitor PF03084014 (lane 3); APP and PS1 co-expressed in the PS1 single knockout HEK 293T cell line (lane 4). Western blotting was used to detect the content of α-CTF in cells under different conditions. Panels C and D: Western blot detection and statistical analysis of the effect of ApoE CT on α-CTF produced by enzymatic digestion of co-expressed APP in wild-type (WT) and PS1 single knockout (PS1 KO) HEK 293T cell lines, respectively, with GFP serving as a control. N=9. Panels E and F: Western blot detection and statistical analysis of the effect of ApoE CT on α-CTF produced by enzymatic digestion of co-expressed APP in wild-type and PS2 single knockout (PS2 KO) HEK 293T cell lines, respectively, with GFP serving as a control. N=9. Panels G and J: Western blot detection and statistical analysis of the effect of ApoE CT on α-CTF produced by enzymatic digestion of co-expressed APP in wild-type (WT) and PS1 and PS2 double knockout (PS1/2 dKO) HEK 293T cell lines, respectively, with GFP serving as a control. N=8. Panels H and J: Western blot detection and statistical analysis of α-CTF production of PS1 and PS2 double knockout (PS1/2 dKO) HEK 293T cells co-expressed with APP, PS1 and GFP or ApoE CT, respectively. N=8. Panels I and J: Western blot detection and statistical analysis of α-CTF production of PS1 and PS2 double knockout (PS1/2 dKO) HEK 293T cells co-expressing APP and PS2 with either GFP or ApoE CT, respectively. N=8. Panels K and L: Western blot detection and statistical analysis of α-CTF production of PS1 and PS2 double knockout (PS1/2 dKO) HEK 293T cells co-expressing APP with either GFP or ApoE2, respectively. N=9. Panel M: ELISA detection of Aβ40 production of PS1 and PS2 double knockout (PS1/2 dKO) HEK 293T cells co-expressing β-CTF and PS2 with either GFP or ApoE CT, respectively. N=4. Panels D, F, J, L and M, one sample t-test.

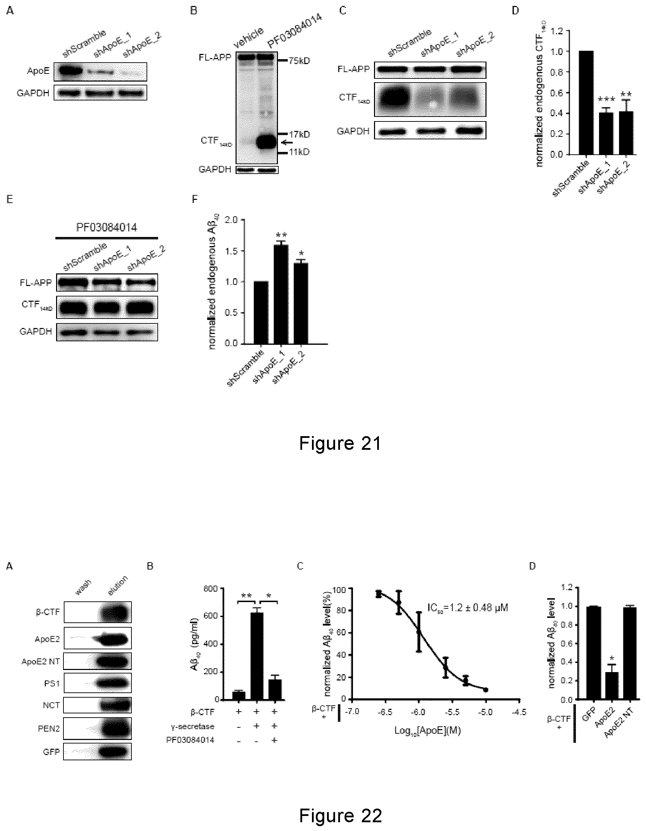

FIG. 21: knockdown of endogenous ApoE in mouse neurons leads to decreased content of APP metabolite CTF_{14kD} and increased Aβ. Panel A: Western blot detection of endogenously expressed ApoE in primary cultured mouse neurons transiently expressing shScramble (negative control), shApoE_1 (shRNA_1 of ApoE) or shApoE _2 (shRNA_2 of ApoE) using a lentivirus. Panel B: relative levels of APP metabolite CTF_{14kD} in mouse neurons treated with 1 uM γ-secretase inhibitor PF03084014. Panels C and D: Western blot detection and statistical analysis of the relative content of metabolite CTF_{14kD} of endogenously expressed APP in primary cultured mouse neurons transiently expressing shScramble, shApoE_1 or shApoE _2 using lentivirus. N=5, one sample t-test. Panels E: Western blot detection of relative content of metabolite CTF_{14kD} of endogenously expressed APP in primary cultured mouse neurons transiently expressing shScramble, shApoE_1 or shApoE _2 using lentivirus when treated with 1 uM γ-secretase inhibitor PF03084014. Panel F: ELISA analysis of Aβ40 produced by endogenously expressed APP in primary cultured mouse neurons transiently expressing shScramble, shApoE_1 or shApoE _2 using a lentivirus. N=5, one sample t-test.
FIG. 22: ApoE2 directly inhibits the γ-cleavage of APP. Panel A: Western blot analysis of expressed and purified β-CTF, ApoE2, ApoE2 NT, PS1, NCT, PEN2 and GFP. Panel B: ELISA detection and statistical analysis of Aβ levels generated under three different conditions: purified β-CTF, or co-incubation of purified β-CTF and γ-secretase, or co-incubation of purified β-CTF and γ-secretase and with treatment with 1 uM γ-secretase inhibitor PF03084014. N=3, student's t test. Panel C: ELISA detection and statistical analysis of Aβ produced by co-incubation of purified β-CTF and a series of concentration gradients of ApoE2. The IC₅₀ is approximately 1.2 ± 0.48 uM. Panel D: ELISA detection and statistical analysis of Aβ produced under co-incubation of purified β-CTF and 2.5 micromolar purified GFP, ApoE2 or ApoE2 NT, respectively. N=3, student's t test.

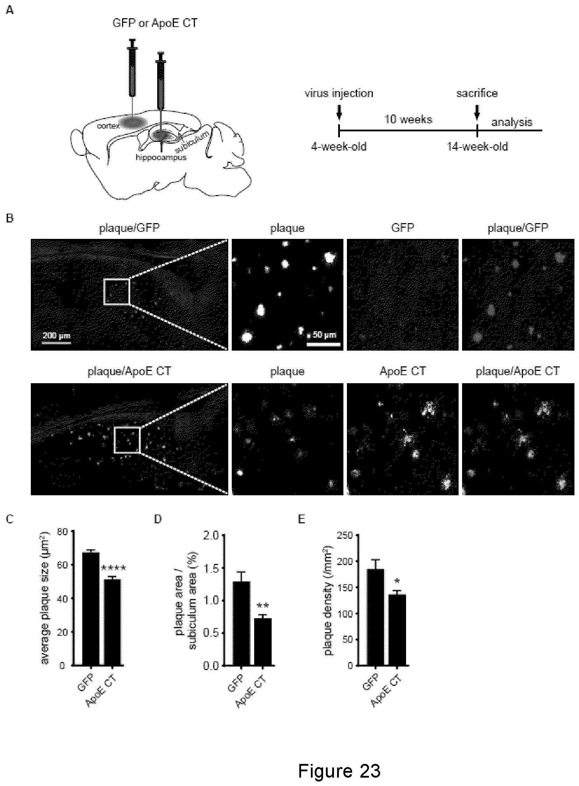

FIG. 23: neuronally expressed ApoE CT migrates to amyloid plaques and slows the formation of amyloid plaques in the brain of 5xFAD mice. Panel A: experimental schema diagram. Panel B: representative pictures of immunofluorescent staining of AAV GFP or ApoE CT expressed in 5×FAD mouse brain neurons for 10 weeks and amyloid plaques. Panel C: statistical analysis of mean size of amyloid plaques. Panel D: statistical analysis of the proportion of the total amyloid plaque area of the subiculum area. Panel E: statistical analysis of density of amyloid plaques. N=21.

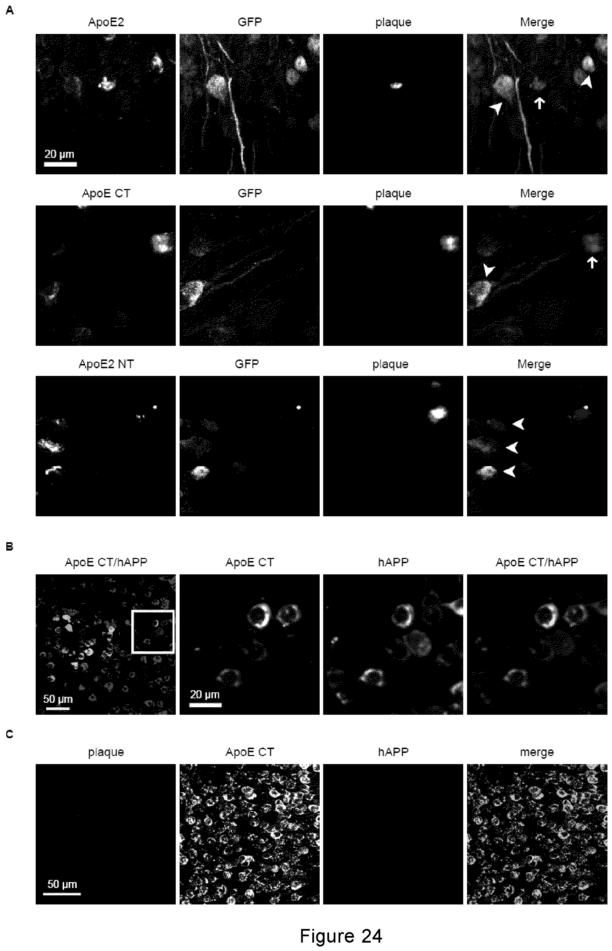

FIG. 24: ApoE expressed in 5×FAD mice neurons accumulates around amyloid plaques by CT. Panel A: representative images of immunofluorescence staining of lentiviral ApoE2-IRES-GFP, ApoE2 NT-IRES-GFP and ApoE CT-IRES-GFP expressed in 5 MAD mouse cortical neurons for three months and amyloid plaques (GFP). Panel B: representative images of immunofluorescent staining of lentiviral ApoE CT and human APP (hAPP) expressed in 5×FAD mouse cortical neurons for one month. Panel C: representative images of immunofluorescent staining of lentiviral ApoE CT and hAPP expressed in wild-type mouse brain neurons for three months. Plaque, amyloid plaque.

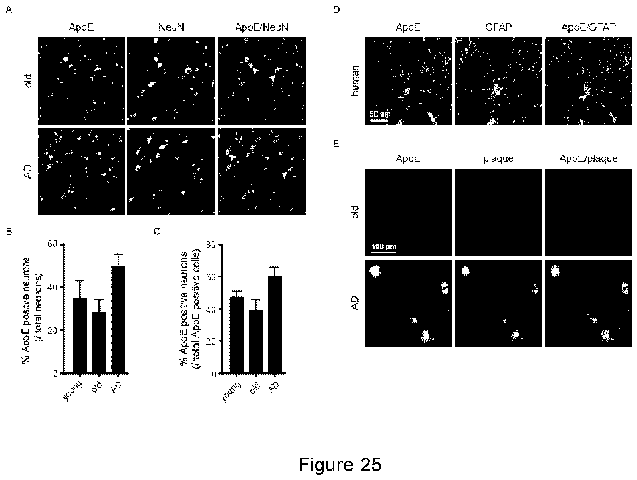

FIG. 25: endogenous ApoE exists in human neurons, and amyloid plaques co-localize with ApoE in the brains of AD patients. Panel A: representative images of co-immunofluorescence co-staining for ApoE and NeuN (neuronal markers) in the cortex of non-AD patients (62 years, top) or AD patients (86 years, bottom). Arrows indicate ApoE-positive neurons. Panel B: percentage of ApoE positive neurons relative to total neurons in human cerebral cortex. Panel C: percentage of ApoE positive neurons relative to all ApoE positive cells. Young: 16-23 years old, n=3; old: >60 years old, n=3; AD: >60 years old, n=6. Panel D: representative images of immunofluorescent co-staining of ApoE and GFAP (astrocyte marker) in human cerebral cortex. Panel E: representative images of co-immunofluorescence co-staining for ApoE and amyloid plaques (6E10) in the cortex of non-AD patients (62 years, top) or AD patients (86 years, bottom).

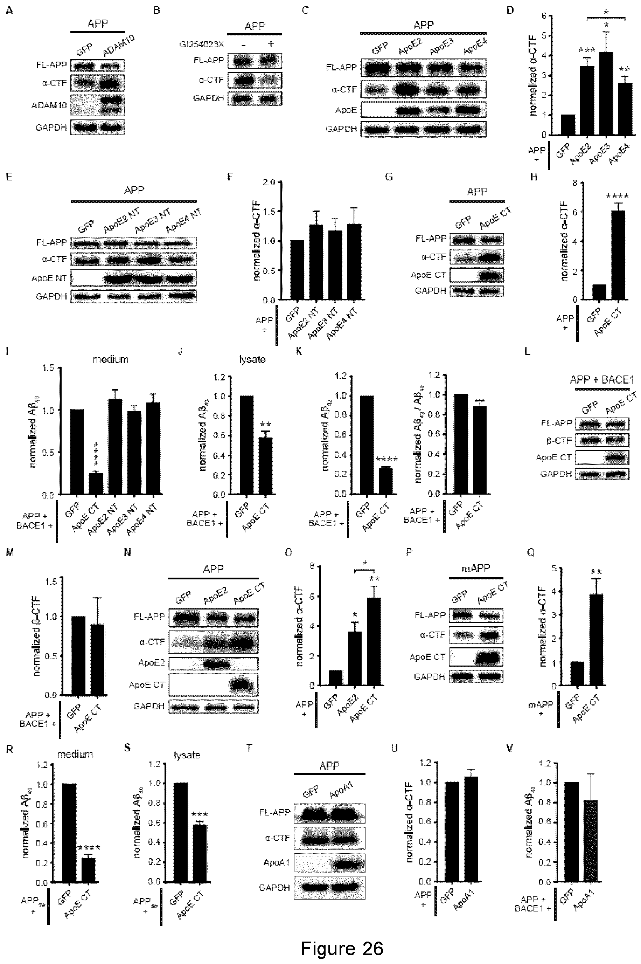

FIG. 26: ApoE2 increases α-CTF and decreases Aβ through ApoE CT in HEK 293T cells. Panel A: Western blot detection of α-CTF produced in HEK 293T cells co-expressing APP with either GFP or ADAM10, respectively. Panel B: Western blot detection of α-CTF produced by HEK 293T cells treated with 1 uM ADAM10 inhibitor GI254023X, and control cells not treated with GI254023X. Panels C and D: Western blot detection and statistical analysis of α-CTF produced in HEK 293T cells co-expressing APP with GFP, ApoE2, ApoE3 or ApoE4, respectively. N=12. Panels E and F: Western blot detection and statistical analysis of α-CTF produced in HEK 293T cells co-expressing APP with GFP, ApoE2 NT, ApoE3 NT or ApoE4 NT, respectively. N=12. Panels G and H: Western blot detection and statistical analysis of α-CTF produced in HEK 293T cells co-expressing APP with GFP or ApoE CT, respectively. N=18. Panel I: ELISA detection and statistical analysis of Aβ40 secreted into the medium that is produced by HEK 293T cells co-expressing APP, BACE1 and GFP, ApoE CT, ApoE2 NT, ApoE3 NT or ApoE4 NT in, respectively. N=4. Panel J: ELISA detection of Aβ40 in cell lysates from HEK 293T cells co-expressing APP, BACE1 and GFP or ApoE CT in HEK 293T cells, respectively. N=5. Panel K: ELISA detection of Aβ42 and Aβ42/Aβ40 produced by co-expression of APP, BACE1 and GFP or ApoE CT, respectively. N=6. Panels L and M: Western blot detection and statistical analysis of β-CTF generated in HEK 293T cells co-expressing APP, BACE1 with GFP or ApoE CT, respectively. N=13. Panels N and O: Western blot detection and statistical analysis of α-CTF produced in HEK 293T cells co-expressing APP and GFP, ApoE2 or ApoE CT, respectively. N=6. Panels P and Q: Western blot detection and statistical analysis of α-CTF produced in HEK 293T cells co-expressing mouse APP (mAPP) and GFP or ApoE CT, respectively. N=6. Panels R and S: ELISA detection and statistics of Aβ40 produced in HEK 293T cells co-expressing APPSW and GFP or ApoE CT, respectively; panel R, Aβ40 in culture medium; panel S, Aβ40 in cell lysate. N=6. Panels T and U: Western blot detection and statistical analysis of α-CTF produced by co-expressing APP and GFP or ApoA1 in HEK 293T cells, respectively. N=6. Panel V: ELISA detection and statistical analysis of Aβ40 produced by co-expressing APP, BACE1 and GFP or ApoA1 in HEK 293T cells, respectively. N=3. Panels D, F, H, I, J, K, M, O, Q, R, S, U and V, one sample t-test.

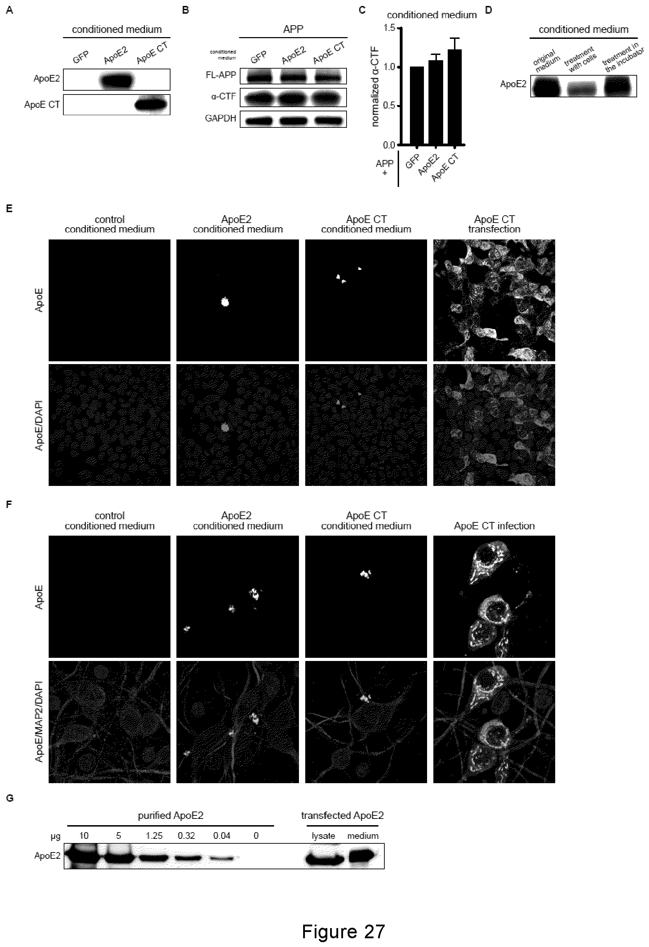

FIG. 27: ApoE2 and ApoE CT in culture medium do not alter APP metabolism. Panel A: Western blot detection of ApoE2 and ApoE CT in cell culture medium. Panels B and C: Western blot detection and statistical analysis of the effects of GFP, ApoE2 or ApoE CT in culture medium on the production of α-CTF from APP co-expressed in HEK 293T cells. N=9. Panel D: Western blot detection of ApoE2 in culture medium under different conditions. The original ApoE2-containing culture medium (lane 1), the ApoE2-containing culture medium after incubation with untransfected cells for 48 hours(lane 2), and the ApoE2-containing culture medium separately stored in a 37°C incubator for 48 hours (lane 3). Panel E: immunofluorescence detection of ApoE2 and ApoE CT in culture medium absorbed by HEK 293T cells, and ApoE CT expressed by HEK 293T cells. DAPI, nuclear marker. Panel F: immunofluorescence detection of ApoE2 and ApoE CT in culture medium absorbed by neurons, and ApoE CT expressed by neurons. MAP2, marker of mature neurons; DAPI, nuclear marker. Panel G: Western blot analysis of purified ApoE2 and ApoE2 from cell lysates or cell culture medium.

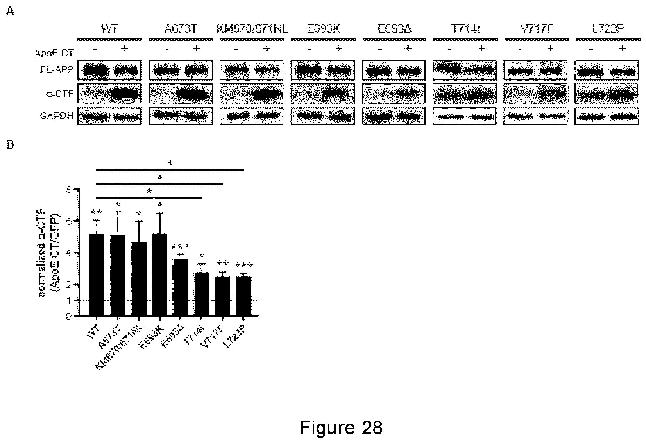

FIG. 28: AD-associated mutations near the γ-cleavage position of APP affect the activity of ApoE CT. Panels A and B: Western blot detection and statistical analysis of α-CTF generated by co-expressing different APPs having AD mutations and GFP or ApoE CT in HEK 293T cells , respectively. N=7, one sample and student's t-test.

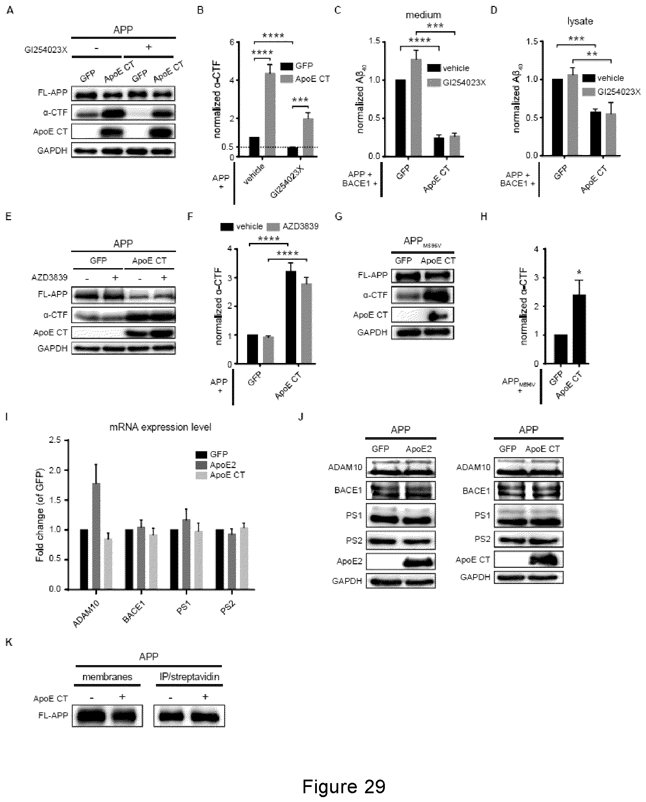

FIG. 29: ApoE CT is still able to regulate APP metabolism when α- or β-secretase is inhibited. Panels A: Western blot detection and analysis of the effects of ApoE CT on the production of α-CTF from co-expressed APP in HEK 293T cells treated with 1 uM ADAM10 inhibitor GI254023X; the control group was not treated with the inhibitor. N=9. Panels C and D: ELISA assay and statistical analysis of Western blot assay for the effects of ApoE CT on Aβ production from co-expressed APP and BACE1 in HEK 293T cells treated with 1 µM ADAM10 inhibitor GI254023X; control group received no inhibitor treatment. Panel C, Aβ40 in cell culture medium; Panel D, Aβ40 in cell lysates. N=6. Panels E and F: Western blot detection and statistical analysis of the effects of ApoE CT on the production of α-CTF from co-expressed APP in HEK 293T cells treated with 5 µM BACE1 inhibitor AZD3839; the control group received no inhibitor treatment. N=10. Panels G and H: Western blot detection and statistical analysis of α-CTF produced by co-expressing APP_{M596V} and GFP or ApoE CT in HEK 293T cells, respectively. N=7. Panel I: qRT-PCR detection and statistical analysis of the effects of expressed ApoE2 or ApoE CT on the mRNA expression of endogenous ADAM10, BACE1, PS1 and PS2 in HEK 293T cells. Panel J: Western blot detection of the effects of ApoE2 or ApoE CT on the protein expression of endogenous ADAM10, BACE1, PS1 and PS2 in HEK 293T cells. Panel K: Western blot detection of the effects of ApoE CT on cell surface expression of APP co-expressed in HEK 293T cells. Cell surface proteins of HEK 293T cells expressing GFP(-) and ApoE CT(+) were labeled with sulfo-N-hydroxysuccinimide-biotin and isolated with streptavidin-coupled agarose beads.

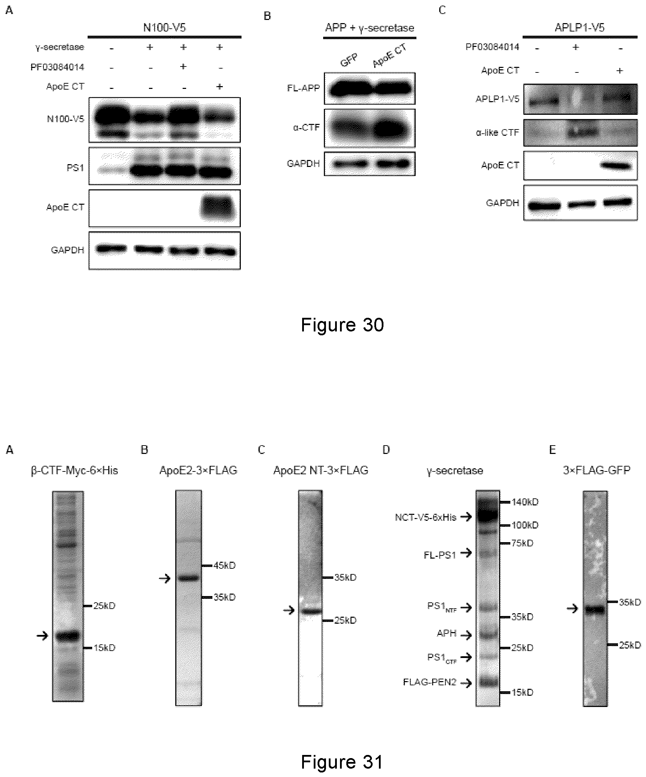

FIG. 30: ApoE CT cannot inhibit the γ-cleavage of NOCTH and APLP1. Panel A: in HEK 293T cells expressing only N100-V5 (lane 1); in HEK 293T cells co-expressing N100-V5 and γ-secretase (lane 2); in HEK 293T cells co-expressing N100-V5 and γ-secretase treated with 1 uM inhibitor of γ-secretase PF03084014 (lane 3); in HEK 293T cells co-expressing N100-V5, γ-secretase and ApoE CT (lane 4). Cleavage of N100-V5 when expressed in the above conditions was detected by Western blot. Panel B: Western blot detection of α-CTF content when APP, γ-secretase and ApoE CT were co-expressed in HEK 293T cells. Panel C: in HEK 293T cells only expression APLP-V5 (lane 1); in HEK 293T cells expressing APLP-V5 treated with 1 uM γ-secretase inhibitor PF03084014 (lane 2); in HEK 293T cells co-expressing APLP1-V5 and ApoE CT (lane 3). The metabolism of APLP1-V5 when expressed in the above conditions was detected by Western blot.
FIG. 31: *in vitro* purified β-CTF, ApoE2, ApoE2 NT and γ-secretase. Coomassie brilliant blue staining shows purified β-CTF-Myc-6×His (panel A), ApoE2-3 ×FLAG (panel B), ApoE2NT-3×FLAG (panel C), γ-secretase (panel D) (NCT-V5-6×His; PLAG-PEN2; APH; full-length PS1, FL-PS1; N-terminal fragment of PS1, PS1 NTF; C-terminal fragment of P1, PS1 CTF) and 3×FLAG-GFP (panel E).

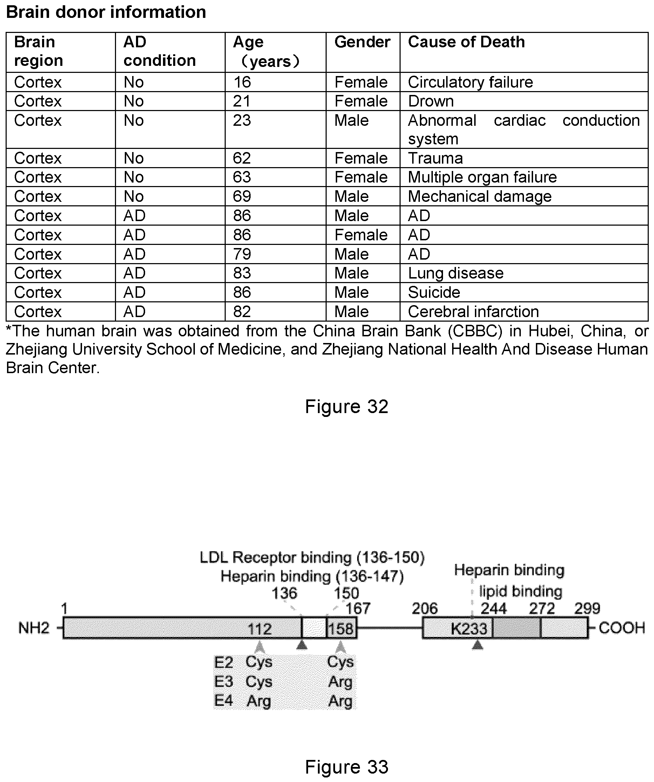

FIG. 32: the human brain information table used in this example.
FIG. 33: human ApoE protein structure. The N-terminal domain (residues at positions 1-167) and the C-terminal domain (residues at positions 206-299) were linked by a flexible hinge region (residues at positions 168-205). The three isoforms are ApoE2 (Cys112; Cys158), ApoE3 (Cys112; Arg158) and ApoE4 (Arg112; Arg158).

### DETAILED DESCRIPTION

It should be understood that within the scope of the present description, the above-mentioned technical features of the present description and the technical features specifically described in the following (such as the examples) can be combined with each other to form a preferred technical solution.

Human apolipoprotein E (ApoE) mainly includes three subtypes, ApoE2, ApoE3 and ApoE4. ApoE comprises three regions: an N-terminal region (ApoE NT), a hinge region and a conserved C-terminal region (ApoE CT). The only sequence difference between the three ApoE isoforms is in their N-terminal domains (FIG. 33). The amino acid sequence of exemplary ApoE2 (human source ApoE2) is as follows:
KVEQAVETEPEPELRQQTEWQSGQRWELALGRFWDYLRWVQTLSEQVQEE LLSSQVTQELRALMDETMKELKAYKSELEEQLTPVAEETRARLSKELQAAQARLG ADMEDVCGRLVQYRGEVQAMLGQSTEELRVRLASHLRKLRKRLLRDADDLQKC LAVYQAGAR*EGAERGLSAIRERLGPLVEQGRVRAATVGSLAGQPLQE*RAQAWGERL RARMEE**MGSRTRDRLDEVKEQVAEVRAKLEEQAQQIRLQAEAFQARLKSWF EPLVEDMQR**QWAGLVEKVQAAVGTSAAPVPSDNH (SEQ ID NO: 12, full-length 299 amino acid residues; the underlined sequence is the 216-299 site of ApoE CT as shown by SEQ ID NO: 33; the boxed sequence is N-terminal domain of 1-167 site ; italic sequence is the hinge region of 168-205 site; residues at the positions 206-299 are the C-terminal domain).

KVEQAVETEPEPELRQQTEWQSGQRWELALGRFWDYLRWVQTLSEQVQEEL
LSSQVTQELRALMDETMKELKAYKSELEEQLTPVAEETRARLSKELQAAQARLGA
DMEDVCGRLVQYRGEVQAMLGQSTEELRVRLASHLRKLRKRLLRDADDLQKCL
AVYQAGAR (SEQ ID NO: 79; N-terminal domain of ApoE2)

EGAERGLSAIRERLGPLVEQGRVRAATVGSLAGQPLQE (SEQ ID NO: 80; hinge region of ApoE2 or ApoE3)

KVEQAVETEPEPELRQQTEWQSGQRWELALGRFWDYLRWVQTLSEQVQEEL
LSSQVTQELRALMDETMKELKAYKSELEEQLTPVAEETRARLSKELQAAQARLGA
DMEDVCGRLVQYRGEVQAMLGQSTEELRVRLASHLRKLRKRLLRDADDLQKCL
AVYQAGAREGAERGLSAIRERLGPLVEQGRVRAATVGSLAGQPLQE (SEQ ID NO:
81; N-terminal domain and hinge region of ApoE2)

The amino acid sequence of exemplary ApoE3 (human source ApoE3) is as follows: KVEQAVETEPEPELRQQTEWQSGQRWELALGRFWDYLRWVQTLSEQVQEE LLSSQVTQELRALMDETMKELKAYKSELEEQLTPVAEETRARLSKELQAAQARLG ADMEDVCGRLVQYRGEVQAMLGQSTEELRVRLASHLRKLRKRLLRDADDLQKR LAVYQAGAR*EGAERGLSAIRERLGPLVEQGRVRAATVGSLAGQPLQE*RAQAWGERL R**ARMEEMGSRTRDRLDEVKEQVAEVRAKLEEQAQQIRLQAEAFQARLKSWF EPLVEDMQR**QWAGLVEKVQAAVGTSAAPVPSDNH (SEQ ID NO: 89, full-length 299 amino acid residues; the underlined sequence is the 216-299 site of ApoE CT as shown by SEQ ID NO: 33; the boxed sequence is the N-terminal domain of 1-167 site; italic sequence is the hinge region of 168-205 site; residues at positions 206-299 are the C-terminal domain).

KVEQAVETEPEPELRQQTEWQSGQRWELALGRFWDYLRWVQTLSEQVQEEL
LSSQVTQELRALMDETMKELKAYKSELEEQLTPVAEETRARLSKELQAAQARLGA

DMEDVCGRLVQYRGEVQAMLGQSTEELRVRLASHLRKLRKRLLRDADDLQKRL
AVYQAGAR (SEQ ID NO: 90; N-terminus domain of ApoE3)

The description provides an isolated ApoE construct comprising an ApoE polypeptide. The ApoE polypeptide comprises a part of the sequence of the C-terminal domain of the ApoE protein (for example, amino acids at positions 221-274 of SEQ ID NO: 12), or a modified sequence based on the sequence. The present description is based on the unexpected discovery by the inventors that the ApoE constructs (such as the ApoE polypeptides or ApoE2 of the present description): (i) can regulate cleavage of the amyloid precursor protein (APP) in neurons (either *in vivo* or *in vitro*) in a cell-autonomous manner; (ii) can inhibit (for example inhibit at least about 10%, 20%, 30%, 40%, 50%, 60%, 70%, 80%, 90%, 95%, or 100% of) the γ-secretase enzymatic activity, such as inhibition of γ-secretase-mediated γ-cleavage of APP, and the inhibitory effect of γ-cleavage of APP is similar to γ-secretase inhibitors (such as PF03084014); (iii) can reduce (e.g. reduce by at least about 10%, 20%, 30%, 40%, 50%, 60%, 70%, 80%, 90%, 95%, or 100%) the amyloidogenic pathway of APP, e.g., reduce (e.g., reduce by at least about 10%, 20%, 30%, 40%, 50%, 60%, 70%, 80%, 90%, 95%, or 100%) production of β-amyloid (Aβ) (e.g., Aβ40 and/or Aβ42), or reduce (e.g., reduce by at least about 10%, 20%, 30%, 40%, 50%, 60%, 70%, 80%, 90%, 95%, or 100%) γ-cleavage of the C-terminal fragment-beta (β-CTF); (iv) can increase (for example, increase by at least about 10%, 20%, 30%, 40%, 50%, 60%, 70%, 80%, 90%, 95%, 100%, 2-fold, 5-fold, 10-fold, 20-fold or higher) non-amyloidogenic pathway of APP, e.g., increase (e.g., increase by at least about 10%, 20%, 30%, 40%, 50%, 60%, 70%, 80%, 90%, 95%, 100%, 2-fold, 5-fold, 10-fold, 20-fold or higher) production of C-terminal fragment-α (α-CTF); (v) does not affect (or affects no more than about 30%, 20%, 10%, 5% or less) mRNA and/or protein expression of α-secretase, β-secretase, and/or γ-secretase (or its subunits) (such as ADAM10, BACE1, PS1 and/or PS2); (vi) does not affect (or affects no more than about 30%, 20%, 10%, 5% or more less) β-secretase-mediated β-cleavage of APP, and/or α-secretase-mediated α-cleavage of APP; (vii) does not inhibit (or inhibits no more than about 30%, 20%, 10%, 5% or less) γ-cleavage of non-APP proteins (such as APLP1 (APP like protein 1) or Notch) or fragments thereof, not increase (or increases no more than about 30%, 20%, 10%, 5% or less) α-CTF produced by γ-cleavage of non-APP protein, indicating that the ApoE construct inhibits γ-cleavage with substrate specificity, while γ-secretase inhibitors do not have this substrate specificity; (viii) the ApoE construct can reduce (e.g. reduce at least about 10%, 20%, 30%, 40%, 50%, 60%, 70%, 80%, 90%, 95%, or 100%) amyloid plaques in mouse brain of AD model, e.g., reduce (e.g., at least about 10%, 20%, 30%, 40%, 50%, 60%, 70%, 80%, 90%, 95%, or 100%) average plaque size, total area, and/or density of amyloid plaques, reduce (e.g., reduce by at least about 10%, 20%, 30%, 40%, 50%, 60%, 70%, 80%, 90% %, 95%, or 100%) production of intraneuronal Aβ (e.g., Aβ40 and/or Aβ42), reduce (e.g., reduce by at least about 10%, 20%, 30%, 40%, 50%, 60%, 70%, 80%, 90%, 95%, or 100%) AD pathological progression in mice, increase (e.g., increase at least about 10%, 20%, 30%, 40%, 50%, 60%, 70%, 80%, 90%, 95%, 100%, 2 times, 5 times, 10 times, 20 times or higher) AD mouse survival rate and/or lifespan; (ix) adding tag proteins (such as purification tags or detection tags) to the end of N-terminus and/or C-terminus of the ApoE construct, and adding or deleting amino acid residues (such as ApoE2-M3F or ApoE2 MD10) inside some of the ApoE constructs (such as ApoE2 or ApoE3) will not affect (or affect no more than about 30%, 20%, 10%, 5% or less) inhibition of the ApoE construct on γ-secretase enzymatic activity, such as inhibiting γ-secretase-mediated γ-cleavage of APP; (x) adding cell-penetrating peptide (CPP) to the N-terminus or C-terminus of the ApoE construct can increase (e.g. increase by at least about 10%, 20%, 30%, 40%, 50%, 60%, 70%, 80%, 90%, 95%, 100%, 2 times, 5 times, 10 times, 20 times or higher) the effect of the inhibition of the ApoE construct on γ-secretase enzyme activity (such as one or more of the above-mentioned multiple effects); (xi) in cells, the N-terminus of the ApoE construct requires a signal peptide to regulate the cleavage of APP by γ-secretase, but there is no strict requirement on the source of the signal peptide; (xii) some of the ApoE constructs described in the present description (such as ApoE CT (SEQ ID NO: 33)) have a higher (for example, at least about 10%, 20%, 30%, 40%, 50%, 60%, 70%, 80%, 90%, 95%, 100%, 2 times, 5 times, 10 times, 20 times or more higher) activity of inhibiting γ-secretase-mediated γ-cleavage of APP than full protein of ApoE2 (such as SEQ ID NO: 12) and/or ApoE3 (such as SEQ ID NO:89); (xiii) the ApoE construct also has an inhibitory effect of γ-cleavage of APP on cells expressing multiple APP variants, such as pathogenic Swedish APP mutant (APP_{sw}), protective Icelandic APP mutation (A673T), APP_{M596V}, or mutations of APP near the β- or α-cleavage site (KM670/671NL, E693K, E693Δ), such as reducing (such as reducing by at least about 10 %, 20%, 30%, 40%, 50%, 60%, 70%, 80%, 90%, 95%, or 100%) generation of Aβ; (xiv) the ApoE construct can exhibit overlapping subcellular distribution patterns around the nucleus with intracellular APP, interacting with endogenous APP, α-secretase and γ-secretase or their components; (xv) in AD model mice, the ApoE construct expressed in neurons locally (such as expression in the cortex, dentate gyrus (DG), and hippocampus CA3), can migrate from a distance to the subiculum and accumulate around amyloid plaques, thereby locally inhibit the γ-secretase enzymatic activity in these amyloidogenic hotspot; and/or (xvi) non-human derived ApoE constructs (such as the C-terminus sequence of monkey ApoE) with high homology (such as at least about 90%, 95%, 96%, 97%, 98%, 99% or higher) to the ApoE constructs described in the present description also exhibit one or more of the aforementioned characteristics.

This application is the first to discover that apolipoprotein E inhibits the cleavage of γ-secretase in a cell-autonomous manner through its C-terminal fragment (that is, it functions in the same cell as γ-secretase), inhibits the amyloidogenic pathway of APP, and reduces generation of Aβ; and increases the non-amyloid metabolite of APP, α-CTF; reduces the size and density of amyloid plaques in AD model mouse brain. Therefore, the C-terminal fragment of ApoE can be used as a medicament targeting γ-secretase to slow down (for example slow down by at least about 10%, 20%, 30%, 40%, 50%, 60%, 70%, 80%, 90%, 95%, or 100% of the AD development process), treat, or prevent the occurrence and development of AD and amyloid cerebrovascular disease. In addition, the C-terminal fragment of ApoE as an inhibitor of γ-secretase may play a role in various biological processes. The application of the C-terminal fragment of ApoE is not limited to the field of neurodegenerative diseases, and can also be used for cancer, inflammatory diseases, kidney disease and other diseases, such as any disease related to the γ-secretase enzymatic digestion activity (inhibition of the γ-secretase enzymatic digestion activity can treat or prevent the disease). Conventional γ-secretase inhibitors cause severe side effects in treatment, all associated with defective Notch signaling, such as immunosuppression, gastrointestinal bleeding and diarrhea, and cancerous skin lesions. The ApoE construct provided by the present description does not affect the γ-cleavage of Notch, so the ApoE construct and related methods provided by the present description may provide a novel therapeutic strategy that is efficient and safe.

The present application further found that the amino acid sequence at positions 221-274 of SEQ ID NO: 12 in the C-terminal fragment of apolipoprotein E (MGSRTRDRLDEVKEQVAEVRAKLEEQAQQIRLQAEAFQARLKSWFEPLVEDMQ R; SEQ ID NO: 78; "ApoE CT ND5CD25") is the amino acid sequence necessary for the C-terminal fragment to perform biological functions (e.g., inhibiting γ-secretase-mediated γ-cleavage of APP). In some embodiments, the shortest functional fragment of the C-terminal fragment of apolipoprotein E is the amino acid sequence at positions 220-274 of SEQ ID NO: 12 (EMGSRTRDRLDEVKEQVAEVRAKLEEQAQQIRLQAEAFQARLKSWFEPLVEDM QR; SEQ ID NO: 87). In some embodiments, the shortest functional fragment of the C-terminal fragment of ApoE is the amino acid sequence at positions 220-279 of SEQ ID NO: 12:

EMGSRTRDRLDEVKEQVAEVRAKLEEQAQQIRLQAEAFQARLKSWFEPLVEDMQ
RQWAGL (SEQ ID NO: 34).

### 1. Definitions

The practice of the present description will employ, unless specifically indicated otherwise, conventional methods within the skill of the art in chemistry, biochemistry, organic chemistry, molecular biology, microbiology, recombinant DNA techniques, genetics, immunology, cell biology, stem cells protocol, cell culture, and transgenic biology, many of which are described below for illustrative purposes. Such techniques are explained fully in the literature. All publications, patents, and patent applications cited herein are hereby incorporated by reference in their entireties.

Unless defined otherwise, all technical and scientific terms used herein have the meaning commonly understood by one of ordinary skill in the art to which this description belongs. For the purposes of the present description, the following terms are defined to be consistent with the meanings commonly understood in the technical field.

As used herein, the terms "treating" and "treatment" mean delaying the onset of one or more symptoms of the disease or condition to which the term applies or such disease or condition, delaying or reversing their progression, reducing their severity, or alleviating or preventing them.

The term "effective amount" or "pharmaceutically effective amount" refers to the amount and/or dosage and/or dosage regimen of one or more agents necessary to produce the desired result, e.g., an amount sufficient to reduce γ-cleavage of APP, or an amount of treatment for one or more symptoms associated with neurodegenerative disease (such as AD), or an amount sufficient to reduce the severity of diseases which characterized by amyloid deposits in the brain of a mammal or delay the progression (e.g., therapeutically effective amount), an amount sufficient to reduce the risk of diseases characterized by amyloid deposits in the brain of a mammal or delay the onset and/or reduce the eventual severity (e.g., a prophylactically effective amount).

The term "alleviate" refers to reducing or eliminating one or more symptoms of the pathology or disease, and/or reducing the rate of one or more symptoms of the pathology or disease or delaying onset or severity of one or more symptoms of the pathology or disease and/or prevention of said pathology or disease. In certain embodiments, reducing or eliminating one or more symptoms of a pathology or disease includes, but is not limited to, reducing or eliminating one or more markers as pathological or disease features (e.g., total-Tau (tTau), phosphorylated-Tau (pTau), APPneo, soluble Aβ40, Aβ42, ratio of pTau/Aβ42 and ratio of tTau/Aβ42 and/or the increase in the level of one or more components in CSF selected from the group consisting of: Aβ42/Aβ40 ratio, Aβ42/Aβ38 ratio, sAPPα, sAPPα/sAPPβ ratio, sAPPα/Aβ40 ratio, sAPPα/Aβ42 ratio, etc.), and/or increase non-amyloid metabolites such as α-CTF, and/or decrease, stabilize, or reverse one or more diagnostic criteria (e.g., Clinical Dementia Rating (CDR)).

"Individual" or "subject" includes, but is not limited to, mammals, birds, frogs, fish, fruit flies, nematodes, and the like. Mammals include, but are not limited to, primates (such as humans, or non-human primates such as monkeys), rodents (such as mice, rats, hamsters, gerbils, squirrels, guinea pigs, and rabbits), livestock (such as pigs, cattle, sheep, horses, donkeys), pets (such as cats, dogs, rabbits and hamsters), etc. In some embodiments, the individual is a human.

The term "formulation" or "pharmaceutical formulation" or "dosage form" or "pharmaceutical formulation" as used herein refers to a composition comprising at least one therapeutic agent or medicine for delivery to a subject.

"Sequence identity" between two polypeptide or nucleic acid sequences indicates the number of residues that are identical between the sequences as a percentage of the total number of residues, and the calculation of the total number of residues is determined based on the type of mutation. Mutation types include insertions (extensions) at either or both ends of the sequence, deletions (truncations) at either or both ends of the sequence, substitutions/replacements of one or more amino acids/nucleotides, insertions within the sequence, deletions within the sequence. Taking polypeptides as an example (the same is true for nucleotides), if the mutation type is one or more of the following: one or more amino acid/nucleotide substitutions/replacements, insertions within the sequence, and deletions within the sequence, the total number of residues is calculated as the longest one of the compared molecules. If the mutation type also includes insertions (extensions) or deletions (truncations) at either or both ends of the sequence, the number of amino acids inserted or deleted at either or both ends (e.g., insertions at both ends or the number of deletions is less than 20) are not counted towards the total number of residues. In calculating percent identity, the sequences being compared are aligned in such a manner as to produce the greatest match between the sequences, with gaps, if any, in the alignment resolved by specific algorithms.

The term "fragment" as used herein refers to a polypeptide and is defined as any isolated portion that is unique or characteristic of a given polypeptide. The term as used herein also refers to any isolated portion of a given polypeptide that retains at least a portion of the activity of the full-length polypeptide. Preferably, the remaining active fraction is at least 10% of the activity of the full-length polypeptide. More preferably, the remaining active portion is at least 20%, 30%, 40%, 50%, 60%, 70%, 80%, or 90% of the activity of the full-length polypeptide. More preferably, the remaining active portion is at least 95%, 96%, 97%, 98%, or 99% of the activity of the full-length polypeptide. Most preferably, the remaining active portion is 100% of the activity of the full-length polypeptide. As used herein, the term also refers to any portion of a given polypeptide that includes at least one defined sequence element of the full-length polypeptide. Preferably, the sequence elements span at least 4-5, more preferably at least about 10, 15, 20, 25, 30, 35, 40, 45, 50 or more amino acids of the full-length polypeptide.

The term "polypeptide" as used herein refers to a chain of sequential amino acids linked by peptide bonds. The term is used to refer to amino acid chains of any length, however those of ordinary skill in the art know that the term does not only refer to long chains, but can also be used to refer to the shortest chains comprising 2 amino acids linked by a peptide bond.

The term "protein" as used herein refers to one or more polypeptides that function as discrete units. The terms "polypeptide" and "protein" are used interchangeably herein if a single polypeptide is a discrete functional unit and no permanent physical association with other polypeptides is required to form that separate functional unit. If the discrete functional unit consists of more than one polypeptide physically associated with each other, the term "protein" as used herein refers to a plurality of polypeptides that are physically coupled and function together as the discrete unit.

"Recombinantly expressed polypeptide", "recombinant polypeptide" and "modified polypeptide" (or protein) refers to a polypeptide expressed from a host cell that has been genetically engineered to express the polypeptide. The recombinantly expressed polypeptide may be the same or similar to the polypeptide normally expressed in the host cell (such as a mammalian cell). Recombinantly expressed polypeptides may also be foreign to the host cell, such as those heterologous to a polypeptide normally expressed by a mammalian host cell. Alternatively, the recombinantly expressed polypeptide can be a chimeric polypeptide, for example, part of the polypeptide comprises the same or similar amino acid sequence as the polypeptide normally expressed in the mammalian host cell, while the other part is foreign to the host cell.

The term "amino acid" means the twenty common naturally occurring amino acids. Naturally occurring amino acids include alanine (Ala; A), arginine (Arg; R), asparagine (Asn; N), aspartic acid (Asp; D), cysteine (Cys; C); glutamic acid (Glu; E), glutamine (Gln; Q), glycine (Gly; G), histidine (His; H), isoleucine (Ile; I), leucine (Leu; L), lysine (Lys; K), methionine (Met; M), phenylalanine (Phe; F), proline (Pro; P), serine (Ser; S), threonine (Thr; T), tryptophan (Trp; W), tyrosine (Tyr; Y) and valine (Val; V).

Non-critical amino acids can be substituted conservatively without affecting the normal function of the protein. Conservative substitutions refer to the replacement of amino acids with chemically or functionally similar amino acids. Conservative substitution tables providing similar amino acids are well known in the art. For example, in some embodiments, the groups of amino acids provided below are considered conservative substitutions for each other.

In certain embodiments, selected groups of amino acids considered to be mutually conservative substitutions include:

| | |
|---|---|
| Acidic residues | D and E |
| Alkaline residues | K, R and H |
| Hydrophilic uncharged residues | S, T, N and Q |
| Aliphatic uncharged residues | G, A, V, L and I |
| Non polar uncharged residues | C, M and P |
| Aromatic residues | F, Y and W |

In certain embodiments, other selected groups of amino acids considered to be mutually conservative substitutions include:

| | |
|---|---|
| Group 1 | A, S and T |
| Group 2 | D and E |
| Group 3 | N and Q |
| Group 4 | R and K |
| Group 5 | I, L and M |
| Group 6 | F, Y and W |

In certain embodiments, other selected groups of amino acids considered to be mutually conservative substitutions include:

| | |
|---|---|
| Group A | A and G |
| Group B | D and E |
| Group C | N and Q |
| Group D | R, K and H |
| Group E | I, L, M, V |
| Group F | F, Y and W |
| Group G | S and T |
| Group H | C and M |

As used herein, the term "about" or "approximately" refers to a change of up to 15%, 10%, 9%, 8%, 7%, 6%, 5%, 4%, 3%, 2% or 1% in quantity, level, value, quantity, frequency, percentage, dimension, size, volume, weight or length. In one embodiment, the term "about" or "approximately" refers to around a reference amount, level, value, quantity, frequency, percentage, dimension, size, amount, weight or length range that fluctuates within ±15%, ±10%, ±9%, ±8%, ±7%, ±6%, ±5%, ±4%, ±3%, ±2%, or ±1%.

As used herein, the term "substantially/essentially", compared to a degree, reference amount, level, value, amount, frequency, percentage, dimension, size, amount, weight or length, means about 70%, 71%, 72%, 73%, 74%, 75%, 76%, 77%, 78%, 79%, 80%, 81%, 82%, 83%, 84%, 85%, 86%, 87% , 88%, 89%, 90%, 91%, 92%, 93%, 94%, 95%, 96%, 97%, 98% or 99% or greater degree, amount, level, value, amount, frequency, percentage, measure, size, volume, weight or length.

A numerical range, inclusive of the endpoints of the range, and each specific value of the range, such as "between 16 and 100 amino acids" includes 16 and 100, and every specific value between 16 and 100.

Throughout this specification, unless the context requires otherwise, the terms "comprising", "including", "comprising" and "having" are to be understood as implying the inclusion of stated step or element or groups of steps or groups of elements, but not the exclusion of any other step or element or groups of steps or groups of elements. In certain embodiments, the terms "comprising", "including", "comprising" and "having" are used synonymously.

"Consisting of" means including, but limited to, anything following the phrase "consisting of'. Thus, the phrase "consisting of" indicates that the listed elements are required or mandatory, and that no other elements may be present.

"Consisting essentially of" is meant to include any of the elements listed after the phrase "consisting essentially of" and is limited to other elements that do not interfere with or contribute to the activities or actions specified in the disclosure of the listed elements. Thus, the phrase "consisting essentially of" indicates that the listed elements are required or mandatory, but that no other elements are optional, and they may or may not exist depending on whether they affect the activity or actions of the listed elements.

The articles "a/an" and "the/said" are used herein to refer to one or more than one (i.e., at least one) grammatical object of the said articles. For example, "an element" means one/an element or more than one/an element.

The use of an alternative (e.g., "or") should be understood to mean either, both, or any combination of the alternatives.

The term "and/or" should be understood to mean either or both of the alternatives.

Throughout this specification, references to "one embodiment", "an embodiment", "a particular embodiment", "a related embodiment", "some embodiments", "another embodiment" or "further embodiments" or a combination thereof means that a particular feature, structure or characteristic described in connection with the embodiment is included in at least one embodiment of the present description. Thus, the appearances of the foregoing phrases in various places throughout this specification are not necessarily all referring to the same embodiment. Furthermore, the particular features, structures or characteristics may be combined in any suitable manner in one or more embodiments.

### 2. ApoE constructs and ApoE polypeptides

In one aspect, the description provides an isolated ApoE construct, which comprises an ApoE polypeptide, wherein the ApoE polypeptide: (1) comprises a fragment from an amino acid sequence as shown by SEQ ID NO: 12, the first amino acid residue at the N-terminus of the fragment (or the ApoE polypeptide) being an amino acid residue at any position between positions 215-221 of SEQ ID NO: 12, and a last amino acid residue at a C-terminus of the fragment (or the ApoE polypeptide) being an amino acid residue at any position between positions 274-299 (e.g., 279-299) of SEQ ID NO: 12; or (2) its amino acid sequence has at least about 70% (e.g., at least about 75%, 80%, 85%, 90%, 95%, 96%, 97%, 98%, 99% or higher) sequence identity with the fragment of (1), and retains at least about 50% (e.g., at least about 60%, 70%, 80%, 85%, 90%, 95%, 96%, 97%, 98%, 99%, 100%, 1.5 times, 2 times, 5 times, 10 times or more) of inhibition of the fragment of (1) against γ-secretase enzymatic digestion activity. It should be understood that the "fragment" described herein refers to a continuous stretch of amino acid sequence from the corresponding protein.

In some embodiments, the ApoE polypeptide comprises a fragment from the amino acid sequence as shown by SEQ ID NO: 12, the first amino acid residue at the N-terminus of the ApoE polypeptide being an amino acid residue at any position between positions 215-221 of SEQ ID NO: 12, the last amino acid residue at the C-terminus of the ApoE polypeptide being an amino acid residue at any position between positions 274-299 (e.g., 279-299) of SEQ ID NO: 12. In some embodiments, the ApoE construct comprises an ApoE polypeptide, the ApoE polypeptide consists of a fragment from the amino acid sequence as shown by SEQ ID NO: 12, the first amino acid residue at the N-terminus of the ApoE polypeptide being an amino acid residue at any position between positions 215-221 of SEQ ID NO: 12, the last amino acid residue at the C-terminus of the ApoE polypeptide being an amino acid residue at any position between positions 274-299 (e.g., 279-299) of SEQ ID NO: 12.

In some embodiments, the ApoE polypeptide has at least about 70% identity with a fragment from the amino acid sequence as shown by SEQ ID NO: 12, and retains at least about 50% of inhibition of γ-secretase enzymatic digestion activity by the fragment, wherein the first amino acid residue at the N-terminus of the fragment is an amino acid residue at any one of positions 215-221 of SEQ ID NO: 12, and the last amino acid residue at the C-terminus of the fragment is an amino acid residue at any one of positions 274-299 (e.g., 279-299) of SEQ ID NO: 12.

In some embodiments, the isolated ApoE construct consists of or essentially consists of the ApoE polypeptide (e.g., isolated ApoE polypeptide). Thus, in some embodiments, the present application also provides an ApoE polypeptide (e.g., isolated ApoE polypeptide).

In some embodiments, the ApoE polypeptide (e.g., isolated ApoE polypeptide) consists of a fragment from the amino acid sequence as shown by SEQ ID NO: 12 of (1), or essentially consists of a fragment from the amino acid sequence as shown by SEQ ID NO: 12 of (1).

In some embodiments, the ApoE construct does not occur naturally. For example, in some embodiments, the ApoE construct or the ApoE polypeptide is not full-length proteins of ApoE2, ApoE3, and/or ApoE4.

In some embodiments, the ApoE construct or the ApoE polypeptide is a full-length protein of ApoE2 or ApoE3 (e.g., a human full-length protein of ApoE2 or ApoE3), such as when used as a ApoE construct with neural-specific expression.

In some embodiments, the starting point of the amino acid sequence (or the first amino acid residue at the N-terminus) of the ApoE polypeptide (such as an isolated ApoE polypeptide) or a fragment of the amino acid sequence as shown by SEQ ID NO: 12 of (1) is any position between positions 215-221 (such as the 215th, 216th, 217th, 218th, 219th, 220th or 221st) of the sequence of ApoE as shown by SEQ ID NO: 12, such as any amino acid residue at position 215, position 216, position 219, position 220 or position 221. In some embodiments, the end point of the amino acid sequence (or the last amino acid residue at the C-terminus) of the ApoE polypeptide or the fragment from the amino acid sequence as shown by SEQ ID NO: 12 of (1) is any position between positions 274-299 (for example, the 274th, 275th, 276th, 277th, 278th, 279th, 280th, 281st, 282nd, 283rd, 284th, 285th, 286th, 287th, 288th, 289th, 290th, 291st, 292nd, 293rd, 294th, 295th, 296th position, 297th, 298th or 299th) of the sequence of ApoE as shown by SEQ ID NO: 12, such as the amino acid residue at any position between positions 279-299 (including the number itself), such as any amino acid residue at position 274th, position 279th or position 299th.

In some embodiments, the ApoE polypeptide (such as an isolated ApoE polypeptide) or the fragment from the amino acid sequence as shown by SEQ ID NO: 12 of (1) comprises (or consists of, or essentially consists of) any one of the following sequences: amino acid residues at positions 215-299, positions 216-299, positions 217-299, positions 218-299, positions 219-299, positions 220-299, positions 221-299, positions 215-274, positions 216-274, positions 217-274, positions 218-274, positions 219-274, positions 220-274, positions 221-274, positions 215-279, positions 216-279, positions 217-279, positions 218-279, positions 219-279, positions 220-279, positions 221-279, positions 215-294, positions 216-294, positions 217-294, positions 218-294, positions 219-294, positions 220-294, positions 221-294, positions 215-289, positions 216-289, positions 217-289, positions 218-289, positions 219-289, positions 220-289, positions 221-289, positions 215-288, positions 216-288, positions 217-288, positions 218-288, positions 219-288, positions 220-288, positions 221-288, positions 215-287, positions 216-287, positions 217-287, positions 218-287, positions 219-287, positions 220-287, positions 221-287, positions 215-286, positions 216-286, positions 217-286, positions 218-286, positions 219-286, positions 220-286, positions 221-286, positions 215-285, positions 216-285, positions 217-285, positions 218-285, positions 219-285, positions 220-285, positions 221-285, positions 215-284, positions 216-284, positions 217-284, positions 218-284, positions 219-284, positions 220-284, or positions 221-284 of SEQ ID NO: 12.

In some embodiments, the ApoE polypeptide (such as the isolated ApoE polypeptide) or the fragment from the amino acid sequence as shown by SEQ ID NO: 12 of (1) comprises (or consists of, or substantially consists of) any of the following sequences: (i) the amino acid sequence of amino acid residues of positions 215-299 of SEQ ID NO: 12; (ii) the amino acid sequence of amino acid residues of positions 216-299 of SEQ ID NO: 12; (iii) the amino acid sequence of amino acid residues of positions 217-299 of SEQ ID NO: 12; (iv) the amino acid sequence of amino acid residues of positions 218-299 of SEQ ID NO: 12; (v) the amino acid sequence of amino acid residues of positions 219-299 of SEQ ID NO: 12; (vi) the amino acid sequence of amino acid residues of positions 220-299 of SEQ ID NO: 12; (vii) the amino acid sequence of amino acid residues of positions 221-299 of SEQ ID NO: 12; (viii) the amino acid sequence of amino acid residues of positions 216-294 of SEQ ID NO: 12; (ix) the amino acid sequence of amino acid residues of positions 216-289 of SEQ ID NO: 12; (x) the amino acid sequence of amino acid residues of positions 219-289 of SEQ ID NO: 12; (xi) the amino acid sequence of amino acid residues of positions 219-288 of SEQ ID NO: 12; (xii) the amino acid sequence of amino acid residues of positions 219-287 of SEQ ID NO: 12; (xiii) the amino acid sequence of amino acid residues of positions 219-286 of SEQ ID NO: 12; (xiv) the amino acid sequence of amino acid residues of positions 219-285 of SEQ ID NO: 12; (xv) the amino acid sequence of amino acid residues of positions 219-284 of SEQ ID NO: 12; (xvi) the amino acid sequence of amino acid residues of positions 219-279 of SEQ ID NO: 12; (xvii) the amino acid sequence of amino acid residues of positions 219-274 of SEQ ID NO: 12; (xviii) the amino acid sequence of amino acid residues of positions 220-274 of SEQ ID NO: 12; (xix) the amino acid sequence of amino acid residues of positions 220-279 of SEQ ID NO: 12; or (xx) the amino acid sequence of amino acid residues of positions 221-274 of SEQ ID NO: 12.

In some embodiments, the ApoE construct or ApoE polypeptide does not comprise one or more amino acid sequences between positions 211-214 of SEQ ID NO: 12. In some embodiments, the ApoE construct or ApoE polypeptide does not comprise one or more (or not comprise any) of the following amino acid fragments: positions 212-214, positions 213-214, positions 211-214, positions 211-215, positions 211-216, positions 211-217, positions 211-218, positions 211-219, positions 211-220, positions 211-221, positions 211-222, positions 212-214, positions 212-215, positions 212-216, positions 212-217, positions 212-218, positions 212-219, positions 212-220, positions 212-221, positions 212-222, positions 213-214, positions 213-215, positions 213-216, positions 213-217, positions 213-218, positions 213-219, positions 213-220, positions 213-221, positions 213-222, positions 214-215, positions 214-216, positions 214-217, positions 214-218, positions 214-219, positions 214-220, positions 214-221, or positions 214-222 of SEQ ID NO: 12.

In some embodiments, the ApoE construct or ApoE polypeptide comprises the amino acid sequence of positions 211-214 of SEQ ID NO: 12, but the C-terminus of the amino acid sequence of positions 211-214 of SEQ ID NO: 12 is not directly linked to the N-terminus of the ApoE polypeptide; for example, the two might be linked by at least one non-ApoE derived sequence (such as FLAG sequence) that is inserted between them. In some embodiments, the 3XFLAG sequence as shown by SEQ ID NO: 77 is inserted between the C-terminus of the amino acid sequence of positions 211-214 of SEQ ID NO: 12 and the N-terminus of the ApoE polypeptide.

In some embodiments, the ApoE construct or the ApoE polypeptide may additionally comprise one or more non-ApoE derived amino acid residues at the N-terminus and/or C-terminus of a fragment of the amino acid sequence as shown by SEQ ID NO: 12 of (1). In some embodiments, the ApoE construct or the ApoE polypeptide may additionally comprise one or more non-ApoE derived amino acid residues at the N-terminus and/or C-terminus of a fragment of the amino acid sequence as shown by SEQ ID NO: 12 of (1), and the amino acid residue is different from the amino acid residue at the corresponding site of SEQ ID NO: 12 when added to the N-terminus or C-terminus of the fragment of (1). For example, the first amino acid residue at the N-terminus of the fragment of the amino acid sequence as shown by SEQ ID NO: 12 of (1) can be the amino acid residue at position 215 of SEQ ID NO: 12, and the ApoE construct or the ApoE polypeptide may additionally comprise one or more amino acid residues at the N-terminus of amino acid residue at position 215 of SEQ ID NO: 12, but the one or more amino acid residues are different from the amino acid residues at positions 214, 213, 212, 211, etc. of SEQ ID NO: 12.

In some embodiments, the ApoE construct may additionally contain one or more non-ApoE derived amino acid residues at the N-terminus and/or C-terminus of the ApoE peptide (for example, a fragment of the amino acid sequence as shown by SEQ ID NO: 12 of (1), or an ApoE mutant). In some embodiments, the ApoE construct may additionally comprise one or more amino acid residues at the N-terminus and/or C-terminus of the ApoE polypeptide, and the amino acid residues are different from the amino acid residue at the corresponding site of SEQ ID NO: 12 when added to the N-terminus or C-terminus of the ApoE peptide. For example, the first amino acid residue at the N-terminus of the ApoE polypeptide can be the amino acid residue at position 215 of SEQ ID NO: 12, and the ApoE construct can additionally contain one or more amino acid residues at the N-terminus of the 215th amino acid residue of SEQ ID NO: 12, but the one or more amino acid residues are different from the amino acid residues at positions 214, 213, 212, 211, etc. of SEQ ID NO: 12.

In some embodiments, the ApoE polypeptide (such as an isolated ApoE polypeptide) or the fragment of the amino acid sequence as shown by SEQ ID NO: 12 of (1) at least comprises the amino acid sequence of positions 221-274 of SEQ ID NO: 12 (SEQ ID NO: 78). For example, the ApoE polypeptide or the fragment of the amino acid sequence as shown by SEQ ID NO: 12 of (1) may additionally contain 1, 2, 3, 4, 5 or 6 amino acid residues at the N-terminus of the amino acid sequence as shown by SEQ ID NO: 78, and/or may additionally contain 1, 2, 3, 4, 5, 6, 7, 8, 9, 10, 11, 12, 13, 14, 15, 16, 17, 18, 19, 20, 21, 22, 23, 24 or 25 amino acid residues at the C-terminus. In some embodiments, the ApoE polypeptide (such as an isolated ApoE polypeptide) or the fragment of the amino acid sequence as shown by SEQ ID NO: 12 of (1) consists of the amino acid sequence as shown by SEQ ID NO: 78, or substantially consists of the amino acid sequence as shown by SEQ ID NO: 78. In some embodiments, the ApoE polypeptide (such as an isolated ApoE polypeptide) or the fragment of the amino acid sequence as shown by SEQ ID NO: 12 of (1) at least comprises the amino acid sequence between of positions 220-279 of SEQ ID NO: 12 (SEQ ID NO: 34). For example, the ApoE polypeptide or the fragment from the amino acid sequence as shown by SEQ ID NO: 12 of (1) may additionally contain 1, 2, 3, 4, or 5 amino acid residues at the N-terminus of the amino acid sequence as shown by SEQ ID NO: 34, and/or may additionally contain 1, 2, 3, 4, 5, 5, 7, 8, 9, 10, 11, 12, 13, 14, 15, 16, 17, 18, 19, or 20 amino acid residues at the C-terminus. In some embodiments, the ApoE polypeptide (such as an isolated ApoE polypeptide) or the fragment of the amino acid sequence as shown by SEQ ID NO: 12 of (1) consists of the amino acid sequence as shown by SEQ ID NO: 34, or substantially consists of the amino acid sequence as shown by SEQ ID NO: 34. In some embodiments, the ApoE polypeptide (such as an isolated ApoE polypeptide) or the fragment of the amino acid sequence as shown by SEQ ID NO: 12 of (1) at least comprises the amino acid sequence between positions 219-274 of SEQ ID NO: 12 (SEQ ID NO: 32). For example, the ApoE polypeptide or the fragment of the amino acid sequence as shown by SEQ ID NO: 12 of (1) can additionally contain 1, 2, 3 or 4 amino acid residues at the N-terminus of the amino acid sequence as shown by SEQ ID NO: 32, and/or can additionally contain 1, 2, 3, 4, 5, 6, 7, 8, 9, 10, 11, 12, 13, 14, 15, 16, 17, 18, 19, 20, 21, 22, 23, 24 or 25 amino acid residues at the C-terminus. In some embodiments, the ApoE polypeptide (such as an isolated ApoE polypeptide) or the fragment of the amino acid sequence as shown by SEQ ID NO: 12 of (1) consists of the amino acid sequence as shown by SEQ ID NO: 32, or substantially consists of the amino acid sequence as shown by SEQ ID NO: 32. In some embodiments, the ApoE polypeptide (such as an isolated ApoE polypeptide) or the fragment from the amino acid sequence as shown by SEQ ID NO: 12 of (1) at least comprises the amino acid sequence between positions 219-279 of SEQ ID NO: 12 (SEQ ID NO: 31). For example, the ApoE polypeptide or the fragment of the amino acid sequence as shown by SEQ ID NO: 12 of (1) can additionally contain 1, 2, 3 or 4 amino acid residues at the N-terminus of the amino acid sequence as shown by SEQ ID NO: 31, and/or can additionally contain 1, 2, 3, 4, 5, 5, 7, 8, 9, 10, 11, 12, 13, 14, 15, 16, 17, 18, 19, or 20 amino acid residues at the C-terminus. In some embodiments, the ApoE polypeptide (such as an isolated ApoE polypeptide) or the fragment of the amino acid sequence as shown by SEQ ID NO: 12 of (1) consists of the amino acid sequence as shown by SEQ ID NO: 31, or substantially consists of the amino acid sequence as shown by SEQ ID NO: 31.

In some embodiments, the ApoE construct (such as an isolated ApoE construct), the ApoE polypeptide (such as an isolated ApoE polypeptide), or the fragment of the amino acid sequence as shown by SEQ ID NO: 12 of (1) comprises any amino acid sequence of SEQ ID NOs: 13, 18-34, 78 and 87 (e.g., SEQ ID NOs: 31-34, 78 and 87, or SEQ ID NO: 33 or 78). In some embodiments, the ApoE construct (such as an isolated ApoE construct), the ApoE polypeptide (such as an isolated ApoE polypeptide), or the fragment of the amino acid sequence as shown by SEQ ID NO: 12 of (1) consists of, or essentially consists of any amino acid sequence of SEQ ID NOs: 13, 18-34, 78 and 87 (e.g., SEQ ID NOs: 31-34, 78 and 87, or SEQ ID NO: 33 or 78).

The ApoE constructs and the ApoE polypeptides described herein also include mutants thereof (also referred to herein as "ApoE mutants"). For example, compared with the fragment of the amino acid sequence as shown by SEQ ID NO: 12 of (1) (such as any one of SEQ ID NOs: 13, 18-34, 78 and 87), the mutant has one or more amino acid mutations (such as additions, deletions or substitutions), including but not limited to "conservative modification for inhibiting enzyme activity". For another example, the mutant has at least about 70% (e.g., at least about 75%, 80%, 85%, 90%, 95%, 96%, 97%, 98%, 99% or more) sequence identity with the fragment of the amino acid sequence as shown by SEQ ID NO: 12 of (1) (for example, any one of SEQ ID NOs: 13, 18-34, 78 and 87). In some embodiments, the mutant retains at least about 50% (e.g., at least about 60%, 70%, 80%, 85%, 90%, 95%, 96%, 97%, 98%, 99%, 100% %, 1.5 times, 2 times, 5 times, 10 times or more) of inhibition of γ-secretase enzymatic digestion activity by the fragment of the amino acid sequence as shown by SEQ ID NO: 12 of (1) (such as any one of SEQ ID NOs: 13, 18-34, 78 and 87). In some embodiments, the mutant retains at least about 50% (e.g., at least about 60%, 70%, 80%, 85%, 90%, 95%, 96%, 97%, 98%, 99%, 100% %, 1.5 times, 2 times, 5 times, 10 times or more) of inhibition of γ-secretase enzymatic digestion activity by ApoE2 (SEQ ID NO: 12) or ApoE3 (SEQ ID NO: 89) or the ApoE fragment as shown by SEQ ID NO: 33.

"Conservative modification for inhibiting enzyme activity" herein refers to amino acid modification that does not significantly affect (for example, reduced by no more than about 50%, 40%, 30%, 20%, 10%, 5%, 1% or less) or change the protein construct or the polypeptide to inhibit the γ-secretase cleavage (e.g., γ-cleavage of APP). Such conservative modification for inhibiting enzyme activity comprises amino acid substitutions, additions and/or deletions. Modifications can be introduced into protein constructs or polypeptides of the description by standard techniques known in the art, such as site-directed mutagenesis or PCR-mediated mutagenesis. In some embodiments, compared with the fragment (e.g., any one of SEQ ID NOs: 13, 18-34, 78 and 87) of the amino acid sequence as shown by SEQ ID NO: 12 of (1), the ApoE mutants have one or more (e.g., no more than 40, no more than 30, no more than 20, no more than 10, no more than 8, no more than 5, no more than 3, 2 or 1) amino acid insertions, substitutions, and/or deletions (including insertions and/or deletions at the N-terminus and/or C-terminus), while still retaining at least about 50% (e.g., at least about 60%, 70%, 80%, 85%, 90%, 95%, 96%, 97%, 98%, 99%, 100%, 1.5 times, 2 times, 5 times, 10 times or more) of biological activity of the fragment of the amino acid sequence as shown by SEQ ID NO: 12 of (1) in inhibiting γ-secretase enzymatic digestion (e.g., γ-cleavage of APP) . In some embodiments, the ApoE polypeptide (e.g., an isolated ApoE polypeptide) retains at least about 50% (e.g., at least about 60%, 70%, 80%, 85%, 90%, 95%, 96%, 97%, 98%, 99%, 100%, 1.5 times, 2 times, 5 times, 10 times or more) of inhibition of γ-secretase enzymatic digestion activity by ApoE2 (SEQ ID NO: 12) or ApoE3 (SEQ ID NO: 89) or the ApoE fragment as shown by SEQ ID NO: 33. In some embodiments, the ApoE polypeptide (such as an isolated ApoE polypeptide) has a higher (e.g., at least about 1.2-fold, 1.5-fold, 2-fold, 5-fold, 10-fold or more) biological activity of inhibiting γ-secretase enzymatic digestion (e.g., γ-cleavage of APP), compared to a fragment of the amino acid sequence as shown by SEQ ID NO: 12 of (1) (such as SEQ ID NOs: 13, 18-34, 78 and 87, such as SEQ ID NO: 33).

In some embodiments, the ApoE polypeptide comprises a mutation (e.g., addition, deletion, or substitution) at one or more amino acid positions in any of the ApoE polypeptides or ApoE constructs in Example 1. The description provides any of the ApoE polypeptides or ApoE constructs (e.g., isolated) of Example 1 and Example 2.

In some embodiments, the ApoE polypeptide comprises mutations at one or more amino acid positions compared to the amino acid sequence as shown by SEQ ID NO: 12 (e.g., addition, deletion, or substitution): R226, D227, R228, L229, D230, Q235, E238, V239, R240, K242, E244, E245, Q246, A247, Q248, Q249, I250, R251, L252, Q253, A254, E255, Q275, V280, V287, T289, S290, A291, P293, V294, P295, S296, D297, N298, H299, R251+T289, R251+T289+A291, S290+P293+V294+P295+S296+D297+N298+H299, R226+D227+R228+L229+D230, E238+V239+R240+K242, or E244+E245+Q246+A247+Q248+Q249+I250+R251+L252+Q253+A254. In some embodiments, the ApoE polypeptide comprises mutations at one or more amino acid positions compared to the amino acid sequence as shown by SEQ ID NO: 12: Q235, E255, Q275, V280, V287, P295, R251+T289, S290+P293+V294+P295+S296+D297+N298+H299, R226+D227+R228+L229+D230, E23 8+V23 9+R240+K242, E244+E245+Q246+A247+Q248+Q249+I250+R251+L252+Q253+A254, or R251+T289+A291. In some embodiments, the mutation comprises a substitution of A, S or T at the one or more positions, for example a substitution of A. In some embodiments, the mutation comprises deletion of amino acid residues at the one or more positions.

In some embodiments, the conservative modification for inhibiting enzyme activity is a conservative substitution. Conservative substitutions refer to substitutions of amino acid residues with amino acid residues having similar side chains. Families of amino acid residues having similar side chains have been defined in the art. These families include amino acids with the following side chains: basic side chains (e.g. lysine, arginine, histidine), acidic side chains (e.g. aspartic acid, glutamic acid), uncharged polar side chains (e.g. glycine, asparagine, glutamine, serine, threonine, tyrosine, cysteine, tryptophan), nonpolar side chains (e.g. alanine, valine, leucine amino acid, isoleucine, proline, phenylalanine, methionine), β-branched side chains (such as threonine, valine, isoleucine) and aromatic side chains (such as tyrosine amino acid, phenylalanine, tryptophan, histidine).

In some embodiments, the ApoE polypeptide comprises mutations at one or more amino acid positions compared to the amino acid sequence as shown by SEQ ID NO: 12: R226A, D227A, R228A, L229A, D230A, Q235A, E238A, V239A, R240A, K242A, E244del, E245del, Q246del, A247del, Q248del, Q249del, I250del, R251S, L252del, Q253del, A254del, E255A, Q275A, V280A, V287A, T289A, S290A, A291T, P293A, V294A, P295A, S296A, D297A, N298A, H299A, R251A+T289A, R251S+T289A+A291T, S290A+P293A+V294A+P295A+S296A+D297A+N298A+H299A, R226A+D227A+R228A+L229A+D230A, E238A+V239A+R240A+K242A, or E244del+ E245del+Q246del+A247del+Q248del+Q249del+I250del+R251del+L252del+Q253del+A 254del. In some embodiments, the ApoE polypeptide comprises mutations at one or more amino acid positions compared to the amino acid sequence as shown by SEQ ID NO: 12: Q235A, E255A, Q275A, V280A, V287A, P295A, R251A+T289A, S290A +P293A+V294A+P295A+S296A+D297A+N298A+H299A, R226A+D227A+R228A+L229A+D230A, E238A+V239A+R240A+K242A, E244del+E245del+Q246del+A247del+Q248del+Q249del+I250del+R251del+L252del+Q2 53del+A254del, or R251S+T289A+A291T.

In some embodiments, the ApoE polypeptide or the ApoE mutant has at least about 70% sequence identity with the fragment from the amino acid sequence as shown by SEQ ID NO: 12 of (1) (such as any sequence of SEQ ID NOs: 13, 18-34, 78 and 87, for example SEQ ID NO: 33), such as at least about 75%, 80%, 85%, 90%, 95%, 96%, 97%, 98%, 99%, or higher (but not 100%) identity. In some embodiments, the ApoE mutant, compared with the fragment from the amino acid sequence as shown by SEQ ID NO: 12 of (1) (for example, any sequence of SEQ ID NOs: 13, 18-34, 78 and 87, for example, SEQ ID NO: 33), can also inhibit the γ-secretase cleavage, or retain at least about 50% (such as at least about 60%, 70%, 80%, 85%, 90%, 95%, 96%, 97%, 98%, 99%, 100%, 1.5 times, 2 times, 5 times, 10 times or more) of inhibition of γ-secretase enzymatic digestion activity by the fragment of the amino acid sequence as shown by SEQ ID NO: 12 of (1). In some embodiments, the ApoE mutant and the fragment of the amino acid sequence as shown by SEQ ID NO: 12 of (1) are also derived from humans. In some embodiments, the ApoE polypeptide comprises or is derived from a non-human sequence. The sequence identity between the mutant and the fragment of the amino acid sequence as shown by SEQ ID NO: 12 of (1) can be determined by methods known in the art, such as BLASTP.

In some embodiments, the ApoE polypeptide comprises or is derived from a non-human sequence, and its amino acid sequence has at least about 70% sequence identity with the fragment of the amino acid sequence as shown by SEQ ID NO: 12 of (1) (such as any sequence of SEQ ID NOs: 13, 18-34, 78 and 87, such as SEQ ID NO: 33), and retain at least about 50% of inhibition of γ-secretase enzymatic digestion activity by the fragment of the amino acid sequence as shown by SEQ ID NO: 12 of (1). In some embodiments, the ApoE polypeptide comprises or is derived from an ApoE sequence of any of the following species: mammals, birds, frogs, fish, drosophila, nematodes and the like. In some embodiments, mammals include, but are not limited to, primates (such as humans, or non-human primates such as monkeys), rodents (such as mice, rats, hamsters, gerbils, squirrels, guinea pigs, and rabbits), livestock (such as pigs, cattle, sheep, horses, donkeys), pets (such as cats, dogs, rabbits and hamsters), etc. In some embodiments, the ApoE polypeptide comprises non-naturally occurring modifications, such as further comprising amino acid modifications on the monkey ApoE sequence.

In some embodiments, the ApoE polypeptide or the ApoE mutant comprises a non-human primate sequence, such as a sequence highly homologous and conserved with the fragment of the amino acid sequence as shown by SEQ ID NO: 12 of (1) (such as SEQ ID NOs: 13, 18-34, 78 and 87, such as SEQ ID NO: 33). In some embodiments, the ApoE polypeptide comprises the monkey-derived ApoE C-terminal amino acid sequence as shown by SEQ ID NO: 62. In some embodiments, the ApoE polypeptide consists of, or essentially consists of, the amino acid sequence as shown by SEQ ID NO: 62.

In some embodiments, the ApoE polypeptide comprises one or more mutations (such as additions, deletions or substitutions) based on the amino acid sequence as shown by SEQ ID NO: 33, such as mutations at any one or more of the above positions, or has at least about 70% (e.g., at least about 75%, 80%, 85%, 90%, 95%, 96%, 97%, 98%, 99% or higher) sequence identity with the amino acid sequence as shown by SEQ ID NO: 33. In some embodiments, the ApoE polypeptide comprises one or more mutations (such as additions, deletions or substitutions) based on the amino acid sequence as shown by SEQ ID NO: 33, such as mutations at any one or more of the above positions, or has at least about 70% (e.g., at least about 75%, 80%, 85%, 90%, 95%, 96%, 97%, 98%, 99% or higher) sequence identity with the amino acid sequence as shown by SEQ ID NO: 33, and retain at least about 50% (e.g., at least about 60%, 70%, 80%, 85%, 90%, 95%, 96%, 97%, 98%, 99%, 100%, 1.5-fold, 2 times, 5 times, 10 times or more) of inhibition of γ-secretase enzymatic digestion activity by the amino acid sequence as shown by SEQ ID NO:33.

In some embodiments, the ApoE construct or the ApoE polypeptide comprises any of the amino acid sequence of SEQ ID NOs: 46-52, 57-59, 62, and 76. In some embodiments, the ApoE construct or the ApoE polypeptide consists of any of the amino acid sequences of SEQ ID NOs: 46-52, 57-59, 62, and 76, or essentially consists of any of the amino acid sequences of SEQ ID NOs: 46-52, 57-59, 62 and 76.

In some embodiments, the inhibitory effect of the ApoE construct (such as the isolated ApoE construct), the ApoE polypeptide (such as the isolated ApoE polypeptide, or an ApoE mutant), or the fragment of the amino acid sequence as shown by SEQ ID NO: 12 of (1) (for example, any one of SEQ ID NOs: 13, 18-34, 78 and 87) on γ-secretase-mediated cleavage (such as γ-cleavage of APP) includes one or more of the following characteristics: (i) inhibits (e.g. inhibits at least about 10%, 20%, 30%, 40%, 50%, 60%, 70%, 80%, 90%, 95%, or 100% of) γ-secretase-mediated γ-cleavage of APP; (ii) decreases (e.g., decreases by at least about 10%, 20%, 30%, 40%, 50%, 60%, 70%, 80%, 90%, 95%, or 100%) amyloidogenic pathway of APP; (iii) decreases (e.g., decreases by at least about 10%, 20%, 30%, 40%, 50%, 60%, 70%, 80%, 90%, 95%, or 100%) production of Aβ (e.g. Aβ40 and/or Aβ42); (iv) decreases (e.g. decreases by at least about 10%, 20%, 30%, 40%, 50%, 60%, 70%, 80%, 90%, 95%, or 100%) γ-cleavage of β-CTF; (v) increases (for example, increases by at least about 10%, 20%, 30%, 40%, 50%, 60%, 70%, 80%, 90%, 95%, 100%, 2-fold, 5-fold, 10-fold, 20-fold or higher) non-amyloidogenic pathway of APP; (vi) increases (for example, increases by at least about 10%, 20%, 30%, 40%, 50%, 60%, 70%, 80%, 90%, 95%, 100%, 2 times, 5 times, 10 times, 20 times or higher) generation of α-CTF; (vii) does not inhibit (or inhibits no more than about 30%, 20%, 10%, 5% or less) γ-cleavage of non-APP proteins (such as APLP1 or Notch) or fragments thereof; (viii) does not increase (or increases no more than about 30%, 20%, 10%, 5% or less) α-CTF generated by γ-cleavage of non-APP proteins (such as APLP 1, Notch, ErbB4, E-cadherin, N-cadherin, ephrin-B2 or CD44); (ix) reduces (e.g., reduces by at least about 10%, 20%, 30%, 40%, 50%, 60%, 70%, 80%, 90%, 95%, or 100%) amyloid plaques in the brain of an individual with AD, e.g., reduces (e.g., reduces by at least about 10%, 20%, 30%, 40%, 50%, 60%, 70%, 80%, 90%, 95%, or 100%) the average plaque size, total area, and/or density of plaque; (x) reduces (e.g., reduces by at least about 10%, 20%, 30%, 40%, 50%, 60%, 70%, 80%, 90%, 95%, or 100%) generation of Aβ (such as Aβ40 and/or Aβ42) in AD individual neurons; (xi) reduces (such as reduces at least about 10%, 20%, 30%, 40%, 50%, 60% %, 70%, 80%, 90%, 95%, or 100%) AD individual pathological process; (xii) increases (such as increases at least about 10%, 20%, 30%, 40%, 50%, 60%, 70%, 80%, 90%, 95%, 100%, 2 times, 5 times, 10 times, 20 times or higher) AD individual survival rate and/or life span; and/or (xiii) does not affect (or affects no more than about 30%, 20%, 10%, 5% or less) mRNA and/or protein expression of α-secretase, β-secretase, and/or γ-secretase (or subunits thereof) (e.g., ADAM10, BACE1, PS1 and/or PS2).

In some embodiments, the ApoE construct (such as an isolated ApoE construct), the ApoE polypeptide (such as an isolated ApoE polypeptide, or an ApoE mutant), or the fragment of the amino acid sequence as shown by SEQ ID NO: 12 of (1) has a higher (e.g., at least about 10%, 20%, 30%, 40%, 50%, 60%, 70%, 80%, 90%, 95%, 100%, 2 times, 5 times, 10 times, 20 times or more higher) inhibitory ability on γ-secretase cleavage (e.g., γ-cleavage of APP) than the protein as shown by SEQ ID NO: 12,33 or 89. In some embodiments, the ApoE construct, the ApoE polypeptide (such as an isolated ApoE polypeptide, or an ApoE mutant), or a fragment of the amino acid sequence as shown by SEQ ID NO: 12 of (1) has the same or similar inhibitory ability on γ-secretase cleavage (such as γ-secretase cleavage of APP) compared to the protein as shown by SEQ ID NO: 12, 33 or 89 (for example, the difference is about within 10%). In some embodiments, the inhibitory ability of the ApoE construct, the ApoE polypeptide (such as an isolated ApoE polypeptide, or an ApoE mutant), or a fragment of the amino acid sequence as shown by SEQ ID NO: 12 of (1) on γ-secretase cleavage (such as γ-secretase cleavage of APP) is at most about 50% (for example, 40%, 30%, 20%, 10%, 5%, 1% or less lower) lower than the protein as shown by SEQ ID NO: 12, 33 or 89.

In some embodiments, the ApoE construct (such as an isolated ApoE construct), the ApoE polypeptide (such as an isolated ApoE polypeptide, or an ApoE mutant), or a fragment of the amino acid sequence as shown by SEQ ID NO: 12 of (1) has a inhibitory activity on γ-secretase-mediated cleavage (such as γ-secretase cleavage of APP) same or similar to a γ-secretase inhibitor (e.g., PF03084014) (for example, the difference is about within 10%, 5% or 1%). In some embodiments, the ApoE construct (such as an isolated ApoE construct), the ApoE polypeptide (such as an isolated ApoE polypeptide, or an ApoE mutant), or the fragment of the amino acid sequences as shown by SEQ ID NO: 12 of (1) has a higher (e.g., at least about 10%, 20%, 50%, 100%, 1.5 times, 2 times, 5 times or more higher) inhibitory ability on γ-secretase cleavage (e.g., γ-cleavage of APP) than a γ-secretase inhibitor (e.g., PF03084014). In some embodiments, the inhibitory activity of the ApoE construct (such as an isolated ApoE construct), the ApoE polypeptide (such as an isolated ApoE polypeptide, or an ApoE mutant), or the fragment of the amino acid sequence as shown by SEQ ID NO: 12 of (1) on γ-secretase-mediated cleavage is lower (e.g., at least about 10%, 20%, 50%, 30%, 1.5 times, 2 times, 5 times or more lower) than a γ-secretase inhibitor (e.g., PF03084014).

In some embodiments, the ApoE construct (such as an isolated ApoE construct), the ApoE polypeptide (such as an isolated ApoE polypeptide, or an ApoE mutant), or the fragment of the amino acid sequence as shown by SEQ ID NO: 12 of (1) has a higher (e.g., at least about 30%, 40%, 50%, 60%, 70%, 80%, 90%, 95%, 100%, 2 times, 5 times, 10 times, 20 times or more higher) inhibitory ability on γ-secretase cleavage of APP than that of non-APP protein (such as APLP1, Notch). In some embodiments, the ApoE construct (such as an isolated ApoE construct), the ApoE polypeptide (such as an isolated ApoE polypeptide, or an ApoE mutant), or the fragment of the amino acid sequences as shown by SEQ ID NO: 12 of (1) only has inhibition on the γ-cleavage of APP, and has no or almost no inhibition on the γ-cleavage of non-APP proteins (such as APLP1, Notch).

In some embodiments, α-amylase inhibitors (e.g., GI254023X) and/or β-amylase inhibitors (e.g., AZD3839) do not affect, or affect no more than about 50% (e.g., no more than about 40%, 30%, 20%, 10%, 5%, 1% or less) of the inhibition of the γ-secretase enzymatic digestion activity (e.g., γ-cleavage of APP) by the ApoE construct (such as an isolated ApoE construct), the ApoE polypeptide (such as an isolated ApoE polypeptide, or an ApoE mutant), or the fragment of the amino acid sequence as shown by SEQ ID NO: 12 of (1). For example, the ApoE construct or ApoE polypeptide can still increase the level of α-CTF produced by APP and reduce the production of Aβ in the presence of an α-amylase inhibitor or a β-amylase inhibitor.

The impact on the γ-secretase enzymatic digestion (such as the γ-secretase cleavage of APP) activity can be studied in any suitable manner in the art, such as studying the enzymatic activity of γ-secretase itself, or studying the characteristics or quantity of cleavage product of γ-secretase (for example, Aβ, such as Aβ40 or Aβ42), or studying the characteristics or quantity (for example, whether it is reduced by γ-cleavage) of γ-secretase substrates (such as APP, α-CTF, β-CTF, APLP1, Notch, ErbB4, E-cadherin, N-cadherin, ephrin-B2 or CD44). These research methods include but are not limited to qRT-PCR, BCA, flow cytometry, proteomics, metabolomics, ELISA, Western blot, co-immunoprecipitation, protein fluorescent labeling, immunostaining, etc., which can be *in vivo* methods or can be an *in vitro* method (cellular or non-cellular assay). Any γ-secretase activity assay kit can be used here, e.g., #MBS704513 kit from MyBioSource, or #FP003 kit from R&D Systems. See also the methods described in Examples 1 and 2.

In some embodiments, the ApoE construct further comprises the N-terminal domain sequence or partial sequence of the ApoE2 protein (for example, the amino acid sequence or partial sequence of positions 1-167 of SEQ ID NO: 12; SEQ ID NO: 79) in the N-terminus of any one of the ApoE polypeptides (e.g., any sequence as shown by SEQ ID NOs: 13, 18-34, 46-52, 57-59, 62, 76, 78, and 87), and/or the hinge region sequence or partial sequence (such as the amino acid sequence or partial sequence of positions 168-205 of SEQ ID NO: 12; SEQ ID NO: 80), or has a sequence having at least about 70% (e.g., at least about 75%, 80%, 85%, 90%, 95%, 96%, 97%, 98%, 99% or higher) identity with the N-terminal domain sequence (or partial sequence) and/or hinge region sequence (or partial sequence) of ApoE2 protein (e.g., ApoE homologous sequences from non-human primates (such as monkeys)). In some embodiments, the ApoE construct further comprises the N-terminal domain sequence or partial sequence of the ApoE3 protein (for example, the amino acid sequence or partial sequence of positions 1-167; SEQ ID NO: 90) in the N-terminus of any one of the ApoE polypeptides (e.g., any sequence as shown by SEQ ID NOs: 13, 18-34, 46-52, 57-59, 62, 76, 78, and 87), and/or the hinge region sequence or partial sequence (such as the amino acid sequence or partial sequence of positions 168-205; SEQ ID NO: 80), or has a sequence having at least about 70% (e.g., at least about 75%, 80%, 85%, 90%, 95%, 96%, 97%, 98%, 99% or higher) identity with the N-terminus domain sequence (or partial sequence) and/or hinge region sequence (or partial sequence) of ApoE3 protein (e.g., ApoE homologous sequences from non-human primates (such as monkeys)). In some embodiments, the ApoE construct further comprises a part of the sequence from the ApoE C-terminal domain (positions 206-299 of SEQ ID NO: 12) at the N-terminus of the ApoE polypeptide (for example, its N-terminal partial sequence), such as the sequence or partial sequence of amino acids of positions 206-214 of SEQ ID NO: 12, or comprises a sequence having at least about 70% (e.g., at least about 75%, 80%, 85%, 90%, 95%, 96%, 97%, 98%, 99% or higher) identity with the partial sequence of the ApoE C-terminal domain. In some embodiments, the C-terminus of the N-terminal domain sequence or partial sequence of the ApoE2 or ApoE3 protein (or non-human homologous protein), or the C-terminus of the (N-terminal domain+hinge region) sequence or partial sequence, or the C-terminus of the (N-terminal domain+hinge region+partial N-terminal sequence of the C-terminal domain) sequence, linked to the N-terminus of the ApoE polypeptide by a peptide bond or an adapter sequence (such as a protein adapter sequence). In some embodiments, the ApoE protein sequence, which is homologous to the N-terminal domain (or partial sequence) and/or hinge region (or partial sequence) and/or C-terminal domain (or its N-terminus partial sequence) of human ApoE2 or ApoE3 protein, was derived from the sequence of any of the following species: mammals (e.g., non-human primates such as monkeys, rodents, livestock, pets), birds, frogs, fish, drosophila, nematodes, and the like.

In some embodiments, the ApoE construct further comprises amino acid sequence of positions 1-167 (SEQ ID NO: 79) or 1-205 (SEQ ID NO: 81) or 1-214 of SEQ ID NO: 12 at the N-terminus of any one of the ApoE polypeptides (e.g., any sequence as shown by SEQ ID NOs: 13, 18-34, 46-52, 57-59, 62, 76, 78, and 87), or comprises an amino acid sequence which has at least about 70% identity with amino acids of positions 1-167 or 1-205 or 1-214 of SEQ ID NO: 12. In some embodiments, the ApoE construct comprises the amino acid sequence as shown by SEQ ID NO: 60 or 61. In some embodiments, the ApoE construct consists of, or essentially consists of, the amino acid sequence as shown by SEQ ID NO: 60 or 61.

In some embodiments, the ApoE construct further comprises amino acid sequence of positions 1-167 (SEQ ID NO: 90) or 1-205 or 1-214 of ApoE3 (SEQ ID NO: 89) at the N-terminus of any one of the ApoE polypeptides (e.g., any sequence as shown by SEQ ID NOs: 13, 18-34, 46-52, 57-59, 62, 76, 78, and 87), or comprises an amino acid sequence which has at least about 70% identity with amino acids of positions 1-167 or 1-205 or 1-214 of ApoE3.

In some embodiments, the ApoE construct comprises (or consists of, or essentially consists of) the full sequence of ApoE2 (e.g., human ApoE2, SEQ ID NO: 12). In some embodiments, the ApoE construct comprises (or consists of, or essentially consists of) the full sequence of ApoE3 (e.g., human ApoE3, SEQ ID NO: 89).

In some embodiments, the ApoE construct further comprises a cell-penetrating peptide at the N-terminus or C-terminus of any one of the ApoE polypeptides (e.g., any sequence as shown by SEQ ID NOs: 13, 18-34, 46-52, 57-59, 62, 76, 78, and 87, or ApoE2 or ApoE3 full-length sequence). In some embodiments, the cell-penetrating peptide comprises (or consists of, or essentially consists of) any amino acid sequence as shown by SEQ ID NOs: 35-45, 63-65 and 84-86, such as the amino acid sequence as shown by SEQ ID NO: 63 or 65. In some embodiments, the cell-penetrating peptide (such as the C-terminus) is linked to the ApoE polypeptide (such as the N-terminus), or the amino acid sequence comprising amino acid residues of positions 1-167 (or fragments thereof) or 1-205 (or fragments thereof), or 1-214 (or fragments thereof) of SEQ ID NO: 12 or 89 (e.g., N-terminal), or the sequence which has at least about 70% identity with amino acids of positions 1-167 (or fragments thereof) or 1-205 (or fragments thereof) or 1-214 (or fragments thereof) of SEQ ID NO: 12 or 89 (e.g., N-terminal) by an adapter sequence (such as protein adapter sequence). In some embodiments, the N-terminus of the cell-penetrating peptide is further linked to the C-terminus of a signal peptide through a peptide bond or an adapter sequence. In some embodiments, the cell-penetrating peptide is located at the most N-terminus of the ApoE construct. In some embodiments, the cell-penetrating peptide is located at the most C-terminus of the ApoE construct.

In some embodiments, the present description provides an ApoE construct (or fusion protein) comprising the following elements (1) and (2): (1) a cell-penetrating peptide, and (2) the full-length sequence of ApoE2 (e.g., SEQ ID NO: 12) or a full-length sequence of ApoE3 (e.g., SEQ ID NO: 89) or a fragment of ApoE2 at least including amino acid residues of positions 221-274 of SEQ ID NO: 12. The element (1) and element (2) can be linked directly through a peptide bond, or through an adapter sequence.

Membrane-penetrating peptides or cell-penetrating peptides refer to a class of short peptides that can carry macromolecular substances into cells. Cell-penetrating peptides known in the art can be used to construct the fusion protein of the present description. Exemplary cell-penetrating peptides can be found in Siegmund Reissmann, Cell penetration: scope and limitations by the application of cell-penetrating peptides, J. Pept. Sci. 2014; 20: 760-784. This description incorporates its entire content by reference. Cell-penetrating peptides suitable for use in the present description include, but are not limited to, RQIKIWFQNRRMKWKK (SEQ ID NO: 35); YGRKKRRQRRR (SEQ ID NO: 36); KQAIPVAK (SEQ ID NO: 37); RRRRNRTRRNRRVR (SEQ ID NO: 38); oligoarginine (that is, a fragment of 9-12 arginine residues; R₉ (SEQ ID NO: 63), R₁₀ (SEQ ID NO: 84), R₁₁ (SEQ ID NO: 85), R₁₂ (SEQ ID NO: 86)); KLTRAQRRAAARKNKRNTRGC (SEQ ID NO: 39); ALWKTLLKKVLKAPKKKRKVC (SEQ ID NO: 40); RKKRRQRRR (SEQ ID NO: 41); DAATATRGRSAASRPTERPRAPARSASRPRRPVE (SEQ ID NO: 42); GWTLNSAGYLLGKINLKALAALAKKIL (SEQ ID NO: 43); AGYLLGKINLKALAALAKKIL (SEQ ID NO: 44); YTAIAWVKAFIRKLRK (SEQ ID NO: 45); and KSVRTWNEIIPSKGCLRVGGRCHPHVNGGGRRRRRRRRR (SEQ ID NO: 65).

In some embodiments, the fragment of ApoE2 described in element (2) in the fusion protein at least including amino acid residues of positions 221-274 of SEQ ID NO: 12 can be the isolated ApoE polypeptide described in any embodiment herein, and can be a fragment comprising the N-terminal, hinge regions (or fragments thereof) and at least the amino acid residues of positions 221-274 at the C-terminus of ApoE2 or ApoE3. In some embodiments, the ApoE2 polypeptide may comprise any of the following sequences: amino acid residues of positions 1-274, 1-279, and 1-280, 1-281, 1-282, 1-283, 1-284, 1-285, 1-286, 1-287, 1-288, 1-289, 1-290, 1-291, 1-292, 1-293, 1-294, 1-295, 1-296, 1-297, 1-298 or 1-299 of SEQ ID NO: 12.

In some embodiments, the element (2) in the fusion protein is selected from any of the amino acid sequences as shown by SEQ ID NOs: 12, 13, 18-34, 60, 78, 87 and 89.

In some embodiments, the linker is a non-protein linker. In some embodiments, the linker is a protein linker. In some embodiments, the linker is a peptide comprising 3-25 residues, e.g., a peptide comprising 3-15, 5-15, 10-20, 20-25, or 3-10 residues. Suitable examples of peptide linkers are well known in the art. Typically, linkers contain one or more tandemly repeated motifs, usually comprising Gly and/or Ser. For example, the motif can be SGGS (SEQ ID NO: 69), GSSGS (SEQ ID NO: 70), GGGS (SEQ ID NO: 71), GGGGS (SEQ ID NO: 72), SSSSG (SEQ ID NO: 73), GSGSA (SEQ ID NO: 74), GGSGG (SEQ ID NO: 75) or GGGSGGGS (SEQ ID NO: 88). In some embodiments, the above sequences are contiguous in the adapter sequence with no intervening amino acid residues between the repeats. Adapter sequences may consist of 1, 2, 3, 4 or 5 repeat motifs. In certain embodiments, the linker sequence is a polyglycine adapter sequence. The number of glycines in the adapter sequence is not particularly limited, usually 2-20, such as 2-15, 2-10, 2-8. In addition to glycine and serine, the linker may contain other known amino acid residues, such as alanine, leucine, threonine, glutamic acid, phenylalanine, arginine, glutamine, and the like. In some embodiments, the adapter sequence is shown in SEQ ID NO: 88.

The ApoE construct or fusion protein of the present description may also include amino acid residues or sequences introduced into fusion proteins due to gene cloning procedures, and/or in order to construct fusion proteins, promote the expression of recombinant proteins, obtain recombinant proteins that are automatically secreted outside the host cell, or facilitate the detection and processing of recombinant proteins, etc. In some embodiments, the ApoE construct or fusion protein of the present description also includes signal peptides, leader peptides, terminal extensions, tag proteins and the like.

In some embodiments, the ApoE construct further comprises a signal peptide at the N-terminus of the ApoE polypeptide, especially when it needs to be expressed in cells. In some embodiments, the C-terminus of the signal peptide is linked to the N-terminus of the ApoE peptide (for example, any sequence as shown by SEQ ID NOs: 13, 18-34, 46-52, 57-59, 62, 76, 78, and 87, or the full-length sequence of ApoE2 or ApoE3), or the N-terminus of the amino acid sequence comprising amino acid residues of positions 1-167 (or fragments thereof) or 1-205 (or fragments thereof), or 1-214 (or fragments thereof) of SEQ ID NO: 12 or 89, or the N-terminus of the sequence which has at least about 70% identity with amino acids of positions 1-167 (or fragments thereof) or 1-205 (or fragments thereof) or 1-214 (or fragments thereof) of SEQ ID NO: 12 or 89, or the N-terminus of the cell-penetrating peptide through peptide bonds or adapter sequences (such as adapter sequences of protein). In some embodiments, the signal peptide is located at the most N-terminus of the ApoE construct. In some embodiments, the signal peptide is further added to the N-terminus of the amino acid sequence as shown by SEQ ID NO: 60 or 61. In some embodiments, the signal peptide is further added to the N-terminus of the full-length of ApoE2 (such as SEQ ID NO: 12) or ApoE3 (such as SEQ ID NO: 89). In some embodiments, the C-terminus of the signal peptide is further linked to the N-terminus of a cell-penetrating peptide through peptide bonds or adapter sequences (such as protein adapter sequences). Any known signal peptide can be used here. In some embodiments, the signal peptide is homologous to the ApoE construct or the ApoE polypeptide, e.g., the signal peptide from the ApoE2 (SEQ ID NO: 12) or ApoE3 (SEQ ID NO: 89) protein itself. In some embodiments, the signal peptide is heterologous to the ApoE construct or the ApoE polypeptide, e.g., the signal peptide from ApoA1 or ApoJ. In some embodiments, the signal peptide comprises (or consists of, or essentially consists of) any one of the amino acid sequences selected from SEQ ID NOs: 66-68.

In some embodiments, the ApoE construct comprises (or consists of, or essentially consists of) the following sequence from N-terminus to C-terminus:
signal peptide - optional linker 1 - cell-penetrating peptide (such as the amino acid sequence as shown by SEQ ID NO: 63 or 65) - optional linker 2 - ApoE2 protein (such as SEQ ID NO: 12) or ApoE3 protein (such as SEQ ID NO: 89);
signal peptide - optional linker - ApoE2 protein (e.g., SEQ ID NO: 12) or ApoE3 protein (e.g., SEQ ID NO: 89);
ApoE2 protein (e.g., SEQ ID NO: 12) or ApoE3 protein (e.g., SEQ ID NO: 89);
cell-penetrating peptide (such as the amino acid sequence as shown by SEQ ID NO: 63 or 65) - optional linker - ApoE2 protein (such as SEQ ID NO: 12) or ApoE3 protein (such as SEQ ID NO: ID NO: 89);
signal peptide - optional linker 1 - cell-penetrating peptide (such as the amino acid sequence as shown by SEQ ID NO: 63 or 65) - optional linker 2 - any one of the ApoE polypeptide (such as any one of SEQ ID NOs: 13, 18-34, 46-52, 57-59, 62, 76, 78 and 87, such as SEQ ID NO: 33);
cell-penetrating peptide (such as the amino acid sequence as shown by SEQ ID NO: 63 or 65) - optional linker - any one of the ApoE polypeptide (such as any one of SEQ ID NOs: 13, 18-34, 46-52, 57-59, 62, 76, 78 and 87, such as SEQ ID NO: 33);
signal peptide - optional linker - any one of the ApoE polypeptides (such as any sequence as shown by SEQ ID NOs: 13, 18-34, 46-52, 57-59, 62, 76, 78 and 87, such as SEQ ID NO :33);
any one of the ApoE polypeptides (such as any sequence as shown by SEQ ID NOs: 13, 18-34, 46-52, 57-59, 62, 76, 78 and 87, such as SEQ ID NO :33);
signal peptide - optional linker 1 - cell-penetrating peptide (such as the amino acid sequence as shown by SEQ ID NO: 63 or 65) - optional linker 2 - N-terminal domain and/or hinge region and/or (the N-terminal of the C-terminal domain) sequence or fragment of ApoE2 or ApoE3 (such as any one of the amino acid sequence or fragment thereof as shown by SEQ ID NOs:79-81 and 90) - optional linker 3 - any one of the ApoE polypeptide (such as any one of SEQ ID NOs: 13, 18-34, 46-52, 57-59, 62, 76, 78 and 87, such as SEQ ID NO: 33);
cell-penetrating peptide (such as the amino acid sequence as shown by SEQ ID NO: 63 or 65) - optional linker 1 - N-terminal domain and/or hinge region and/or (the N-terminal of the C-terminal domain) sequence or fragment of ApoE2 or ApoE3 (such as any one of the amino acid sequence or fragment thereof as shown by SEQ ID NOs:79-81 and 90) - optional linker 2 - any one of the ApoE polypeptide (such as any one of SEQ ID NOs: 13, 18-34, 46-52, 57-59, 62, 76, 78 and 87, such as SEQ ID NO: 33);
signal peptide - optional linker 1 - N-terminal domain and/or hinge region and/or (the N-terminal of the C-terminal domain) sequence or fragment of ApoE2 or ApoE3 (such as any one of the amino acid sequence or fragment thereof as shown by SEQ ID NOs:79-81 and 90) - optional linker 2 - any one of the ApoE polypeptide (such as any one of SEQ ID NOs: 13, 18-34, 46-52, 57-59, 62, 76, 78 and 87, such as SEQ ID NO: 33);
N-terminus domain and/or hinge region and/or (the N-terminus of the C-terminus domain) sequence or fragment of ApoE2 or ApoE3 (such as any one of the amino acid sequence or fragment thereof as shown by SEQ ID NOs:79-81 and 90) - optional linker - any one of the ApoE polypeptide (such as any one of SEQ ID NOs: 13, 18-34, 46-52, 57-59, 62, 76, 78 and 87, such as SEQ ID NO: 33);
signal peptide - optional linker 1 - cell-penetrating peptide (such as the amino acid sequence as shown by SEQ ID NO: 63 or 65) - optional linker 2 - (the amino acid sequence as shown by SEQ ID NO: 60 or SEQ ID NO: 61);
cell-penetrating peptide (such as the amino acid sequence as shown by SEQ ID NO: 63 or 65) - optional linker - (the amino acid sequence as shown by SEQ ID NO: 60 or SEQ ID NO: 61);
the amino acid sequence as shown by SEQ ID NO: 60 or SEQ ID NO: 61; or
signal peptide - optional linker - (the amino acid sequence as shown by SEQ ID NO: 60 or SEQ ID NO: 61).

In some embodiments, the ApoE construct further comprises a tag protein at the N-terminus and/or C-terminus of the ApoE polypeptide. In some embodiments, the tag protein is located at the most N-terminus of the ApoE construct. In some embodiments, the tag protein is located at the most C-terminus of the ApoE construct. In some embodiments, the tag protein (such as the 3XFLAG sequence as shown by SEQ ID NO: 77) is located inside the ApoE construct but at the N-terminus of the ApoE polypeptide, such as directly linked with the N-terminus of the ApoE polypeptide. The tag protein can be an expression purification tag (such as His-tag, FLAG-tag, GST-tag, c-Myc-tag, V5-tag, HA-tag), or a detection tag (such as GFP, RFP, V5, HA). In some embodiments, purification tags can also be used for detection, such as immunoblotting or antibody detection. In some embodiments, the tag protein is located at the most N-terminus of the ApoE construct (e.g., ApoE2 or ApoE3, or an ApoE polypeptide comprising the sequence of SEQ ID NO: 33). In some embodiments, the tag protein is located at the most C-terminus of the ApoE construct. In some embodiments, the tag protein is V5 tag: GKPIPNPLLGLDST (SEQ ID NO: 82) or IPNPLLGLD (SEQ ID NO: 83). In some embodiments, the tag protein is a FLAG tag (e.g., SEQ ID NO: 77).

### Amyloid-beta precursor protein (APP) and hydrolysis

APP is a cell membrane embedded protein, mainly concentrated in neuronal synapses. It comprises cleavage sites of α, β, γ secretases. APP is expressed in many different species and is highly homologous. The APP described herein can be from any species, such as mammals, birds, frogs, fish, fruit flies, nematodes, and the like. Mammals include, but are not limited to, primates (such as humans, or non-human primates such as monkeys), rodents (such as mice, rats, hamsters, gerbils, squirrels, guinea pigs, and rabbits), livestock (such as pigs, cattle, sheep, horses, donkeys), pets (such as cats, dogs, rabbits and hamsters), etc. In some embodiments, the APP is a human APP.

Amyloidogenic pathway of APP can generate short amyloid β-peptides (Aβ). Aβ polypeptides are produced by cleavage of APP by β-secretase activity near the N-terminal position of Aβ and by γ-secretase activity near the C-terminal position of Aβ. APP is also cleaved by α-secretase activity, yielding a secreted, non-amyloid fragment of soluble APPα. β-site APP-cleaving enzyme 1 ("BACE-1") is believed to be the major aspartyl protease responsible for the production of Aβ by β-secretase activity. BACE-1 inhibition can suppress Aβ production. BACE1 is a type I transmembrane protein with a luminal active site that cleaves APP to release the extracellular domain (sAPPβ) into the extracellular space. The remaining C-terminal fragment (CTF) undergoes further cleavage by γ-secretase, resulting in the release of the intracellular C-terminal domain (AICD) of Aβ and APP. Presenilin is the major enzymatic component of γ-secretase, and imprecise cleavage of APP by γ-secretase produces contiguous Aβ polypeptides at the C-terminus varying in length by several amino acids. The majority of Aβ normally ends at amino acid 40 (Aβ40), but the 42-amino acid variant (Aβ42) has been shown to be more susceptible to aggregation and has been postulated to nucleate senile plaque formation. Modulation of γ-secretase can also lead to an increase in the 38-amino acid variant (Aβ38). The competing α-secretase pathway results from sequential cleavage by α-secretase and γ-secretase. ADAM-10 is an α-secretase for APP cleavage that releases an ectodomain (sAPPα) that exhibits neuroprotective functions (Lammich et al. (1999) Proc. Natl. Acad. Sci. USA, 96: 3922-3927). Subsequent cleavage of the 83-amino acid CTF (C83) releases p3 (which is non-amyloid) and AICD (Furukawa et al. (1996) J. Neurochem., 67:1882-1896).

Aβ is a major component of β-amyloid fibrils and amyloid plaques, which are thought to play a role in a growing number of pathologies. Examples of such pathologies include, but are not limited to, Alzheimer's disease, Down's syndrome, Parkinson's disease, memory loss (including memory loss associated with Alzheimer's disease and Parkinson's disease), Attention Deficit Disorder (including attention deficit disorders associated with Alzheimer's disease, Parkinson's disease, and Down syndrome), Dementia (including pre senile dementia, senile dementia, dementia associated with Alzheimer's disease (AD), Parkinson's disease and Down syndrome), progressive supranuclear palsy, corticobasal degeneration, neurodegeneration, olfactory disturbances (including those associated with Alzheimer's disease, Parkinson's disease, and Down syndrome), β-amyloid cerebrovascular disease (including cerebral amyloid cerebrovascular disease), hereditary cerebral hemorrhage, mild cognitive impairment ("MCI"), glaucoma, amyloidosis, type II diabetes, hemodialysis (β2 microsphere protein and the complications therefrom), neurodegenerative diseases such as scrapie, bovine spongiform encephalitis, Creutzfeld Jakobdisease, traumatic brain injury, etc.

In some embodiments, the APP is wild-type APP. In some embodiments, the APP is a naturally occurring APP subtype. In some embodiments, the APP is a mutant, including but not limited to any known or unknown APP mutation. APP mutations have been identified in 121 families. The most common missense mutations replace the Val⁷¹⁷ residue with an Ile (London mutation), Phe, or Gly residue, replace the Glu⁶⁹³ with a Gln residue (Dutch mutation) or Gly residue (Arctic mutation), or replace the Lys⁶⁷⁰ and Met⁶⁷¹ with Asn and Leu residues (the double mutation is called the Swedish mutation APPsw and is numbered in the APP₇₇₀ isoform). In some embodiments, the APP is human APP comprising one or more of the following mutations: K670N, M671L, Swedish (K670N+M671L), Florida (1716V), London (V717I), APPM_{596V} (incapable of β cleavage), fAD mutations near γ cleavage sites (e.g., T714I, V717F, L723P), fAD mutations near β- or α-cleavage sites (e.g., KM670/671NL, E693K, E693Δ), or protective Icelandic mutations (A673T). In some embodiments, the APP does not comprise mutations affecting γ-cleavage, such as mutations in the attachment of the γ site.

In some embodiments, the ApoE construct or ApoE polypeptide can inhibit γ-cleavage of any APP (e.g., inhibit at least about 10%, 20%, 30%, 40%, 50%, 60%, 70%, 80%, 85%, 90%, 95%, 96%, 97%, 98%, 99% or 100%). In some embodiments, the ApoE construct or ApoE polypeptide has an ability to inhibit γ-cleavage of any of the APP mutants that is at least about 30% (e.g., at least about 40%, 50%, 60%, 70%, 80%, 85%, 90%, 95%, 96%, 97%, 98%, 99%, 100%, 1.5 times, 2 times, 5 times, 10 times or more) of its ability to inhibit γ-cleavage of wild-type APP. In some embodiments, the γ-cleavage inhibition ability of the ApoE construct or ApoE polypeptide to APP comprising a mutation affecting γ-cleavage (e.g., T714I, V717F, L723P) is less than (e.g., at least about 20%, 30%, %, 40%, 50%, 60%, 70%, 80%, 85%, 90%, 95%, 99% or 100%) its ability to inhibit γ-cleavage of wild-type APP.

### γ-secretase

The γ-secretase complex consists of four separate proteins: PSEN1 (presenilin-1, PS1), nicastrin, APH-1 (anterior pharynx-defective 1), and PEN-2 (presenilin enhancer 2). Recent evidence suggests that CD147, the fifth protein of the complex, is a non-essential regulator whose loss increases γ-secretase activity.

The γ-secretase described herein can be from any species, such as mammals, birds, frogs, fish, fruit flies, nematodes, and the like. In some embodiments, the γ-secretase is from a human, or a non-human primate such as a monkey.

In some embodiments, the γ-secretase described herein is wild-type γ-secretase, or a naturally occurring isoform. In some embodiments, the γ-secretase comprises one or more mutations, such as mutations that enhance or decrease its enzymatic digestion activity. In some embodiments, the γ-secretase has one or two mutations in PS 1: M146L and/or L286V. In some embodiments, the PS1 and/or PS2 activity of the γ-secretase is reduced or absent.

In some embodiments, the ApoE construct or ApoE polypeptide can inhibit the enzymatic activity of any γ-secretase (e.g., inhibit at least about 10%, 20%, 30%, 40%, 50%, 60%, 70%, 80%, 85%, 90%, 95%, 96%, 97%, 98%, 99% or 100%). In some embodiments, the ApoE construct or ApoE polypeptide has an ability to inhibit enzymatic activities of any one of the γ-secretase mutants that is at least about 30% (e.g., at least about 40%, 50%, 60%, 70%, 80%, 85%, 90%, 95%, 96%, 97%, 98%, 99%, 100%, 1.5 times, 2 times, 5 times, 10 times or more) of its ability to inhibit enzymatic activities of wild-type γ-secretase.

### 3. Manufacturing method of ApoE construct

The present description also provides isolated nucleic acids and vectors (such as cloning vectors or expression vectors) encoding the protein portion of any of the above isolated ApoE constructs (such as any amino acid sequence as shown by SEQ ID NOs: 12, 60, 61 and 89), fusion proteins, or ApoE polypeptides (such as any one of the amino acid sequences as shown by SEQ ID NOs: 13, 18-34, 46-52, 57-59, 62, 76, 78 and 87, such as of SEQ ID NO: 33), and a host cell expressing any of the above isolated ApoE constructs, or a host cell comprising the nucleic acid or vector. In some embodiments, the vector is a recombinant AAV or lentivirus expression vector. In some embodiments, the vector comprises a neuron-specific promoter at the 5' end of the isolated nucleic acid. In some embodiments, the neuron-specific promoter is selected from the synapsin promoter (e.g., hSYN), the thy-1 promoter, and the calmodulin-dependent protein kinase II-alpha promoter. In some embodiments, the host cell is a neural cell, such as a neuron (e.g., a human neuron, a non-human primate neuron, or a rodent neuron). See Examples 1 and 2 for vectors, cells and methods of manufacture.

In some embodiments, the present description provides a nucleic acid or vector capable of mediating the specific expression of any one of the ApoE constructs, fusion proteins, or ApoE polypeptides in neural cells. In some embodiments, the description provides a vector or isolated nucleic acid comprising the following 5' to 3' sequence: neuron-specific promoter (e.g., hSYN) - nucleotide sequence encoding signal peptide - nucleotide sequence encoding cell-penetrating peptide (e.g., SEQ ID NO: 63 or 65) - nucleotide sequence encoding any of the ApoE constructs (e.g., SEQ ID NO: 12 or 89)/fusion proteins/ApoE polypeptides (e.g., SEQ ID NO: 33). In some embodiments, the description provides a vector or isolated nucleic acid comprising the following 5' to 3' sequence: neuron-specific promoter (e.g., hSYN) - nucleotide sequence encoding cell-penetrating peptide (e.g., SEQ ID NO: 63 or 65) - nucleotide sequence encoding any of the ApoE constructs (e.g., SEQ ID NO: 12 or 89)/fusion proteins/ApoE polypeptides (e.g., SEQ ID NO: 33). In some embodiments, the description provides a vector or isolated nucleic acid comprising the following 5' to 3' sequence: neuron-specific promoter (e.g., hSYN) - nucleotide sequence encoding signal peptide - nucleotide sequence encoding any of the ApoE constructs (e.g., SEQ ID NO: 12 or 89)/fusion proteins/ApoE polypeptides (e.g., SEQ ID NO: 33). In some embodiments, the ApoE construct comprises (or consists of, or essentially consists of) the full sequence of ApoE2 or ApoE3. In some embodiments, the ApoE construct comprises (or consists of, or essentially consists of) the amino acid sequence as shown by SEQ ID NO: 60 or 61. In some embodiments, the ApoE polypeptide comprises (or consists of, or essentially consists of) any one of the amino acid sequence as shown by SEQ ID NOs: 13, 18-34, 46-52, 57-59, 62, 76, 78 and 87, such as SEQ ID NO: 33.

The present application also comprises nucleic acid molecules encoding any of the above isolated ApoE constructs, fusion proteins, or ApoE polypeptides, and their complements. The description includes all forms of the nucleic acid molecules of the description, including RNA and DNA. It should be understood that the complementary sequence described herein refers to a complementary sequence that is substantially identical in length to the nucleic acid molecule (e.g., at least about 85%, 90%, 95%, 96%, 97%, 98%, 99% or 100% identical in length), for example, at least about 70%, 75%, 80%, 85%, 90%, 95%, 96%, 97%, 98%, 99% or 100% complementary.

Nucleic acid constructs comprising the nucleic acid molecule or its complement are also included within the scope of the present description. The nucleic acid construct may be an expression cassette comprising a promoter, the nucleic acid molecule and a transcription termination sequence. Depending on the need for expression, the expression cassette may optionally contain an enhancer. Promoters, enhancers, transcription termination sequences, selectable markers and signal sequences are well known in the art, and can be appropriately selected by those skilled in the art according to the selected host cell for expressing the polypeptide. These elements of the expression cassette are operably linked to each other such that they perform their intended functions. For example, operably linking a promoter sequence to a nucleotide sequence of interest refers to linking the promoter sequence and the nucleotide sequence of interest such that the promoter sequence is capable of directing the transcription of interested nucleotide sequences in target cells and/or the synthesis of peptides encoded by interested nucleotide sequences. A selectable marker can aid in the selection of host cells into which the vector has been inserted from those into which the vector has not been inserted. For example, a selectable marker can be a gene that confers antibiotic resistance. The signal sequence can be chosen to allow trafficking of the expressed polypeptide to the outside of the host cell.

Genetic control elements can be used to regulate gene expression of polypeptides or proteins. Such genetic control elements are selected to be active in the relevant host cell. Control elements may be constitutively active or inducible under specified conditions. Inducible control elements are particularly useful when expressing proteins that are toxic or have other adverse effects on cell growth and/or viability. In such cases, regulation of polypeptide or protein expression by inducible control elements can improve cell viability, cell density and/or overall yield of the expressed polypeptide or protein. A large number of control elements that can be used in the practice of the present description are known and available in the art.

Representative constitutive mammalian promoters that can be used in the present description include, but are not limited to, hypoxanthine phosphoribosyltransferase (HPTR) promoter, adenosine deaminase promoter, pyruvate kinase promoter, β actin promoter and other constitutive promoters known to those of ordinary skill in the art. In addition, viral promoters capable of driving constitutive expression of coding sequences in eukaryotic cells include, for example, simian virus promoters, herpes simplex virus promoters, papillomavirus promoters, adenovirus promoters, human immunodeficiency virus (HIV) promoters, the Rous sarcoma virus promoters, the cytomegalovirus (CMV) promoters, the long terminal repeat (LTR) of Moloney murine leukemia virus and other retroviruses, the herpes simplex virus thymidine kinase promoters, and other viral promoters known to those of ordinary skill in the art.

Inducible promoters, which drive the expression of an operably linked coding sequence in the presence of an inducing agent, can also be used in accordance with the present description. For example, in mammalian cells, the metallothionein promoter can induce transcription of downstream coding sequences in the presence of certain metal ions. Other inducible promoters are recognized and/or known to those of ordinary skill in the art.

In some embodiments, gene expression control elements suitable for use in the present description are neuron-specific control elements, including promoters and enhancers. Herein, the term "neuron-specific" control element refers to control elements for specifically expressing a nucleic acid molecule of interest in neurons, such as promoters and enhancers. In some embodiments, a neuron-specific promoter is a promoter used to express a transgene in a certain subtype of neurons (e.g., dopaminergic neurons, excitatory neurons, lateral prefrontal cortex neurons, etc.).

Neuron-specific promoters and other control elements (e.g., enhancers) are known in the art. Suitable neuron-specific promoters include, but are not limited to, the neuron-specific enolase (NSE) promoter, aromatic amino acid decarboxylase promoter, neurofilament promoter, synapsin promoter, thy-1 promoter, Serotonin receptor promoter, tyrosine hydroxylase promoter, GnRH promoter, L7 promoter, DNMT promoter, enkephalin promoter, myelin basic protein promoter, calmodulin-dependent protein kinase II-α promoter, and CMV enhancer/platelet-derived growth factor-β promoter. In some embodiments, the neuron-specific promoter is selected from the synapsin promoter (e.g., hSYN), the thy-1 promoter, and the calmodulin-dependent protein kinase II-α promoter.

In general, gene expression sequences also include 5' non-transcribed and 5' non-translated sequences involved in the initiation of transcription and translation, respectively, such as TATA box, cap sequence, CAAT sequence, etc. Enhancer elements can optionally be used to enhance the expression level of a polypeptide or protein to be expressed. Examples of enhancer elements that can function in mammalian cells include the SV40 early gene enhancer (as described by Dijkema et al., EMBOJ. (1985) 4:761), and include enhancers/promoters from the long terminal repeat sequences of Rous sarcoma virus (RSV) (as described by Gorman et al. Human, Proc.Natl.Acad.Scl.USA (1982b) 79:6777) and human cytomegalovirus (as described by Boshart et al., Cell (1985) 41:521).

Systems for linking control elements to coding sequences are well known in the art (General Molecular Biology and Recombinant DNA Techniques, e.g., Sambrook, Fritsch and Maniatis, Molecular Cloning: A Laboratory Manual, Second Edition, Cold Spring Harbor Laboratory Press, Cold Spring Harbor, NY, 1989, incorporated herein by reference). Commercial vectors suitable for insertion of preferred coding sequences for expression in a variety of mammalian cells under a variety of growth and induction conditions are also known in the art.

In some embodiments, the nucleic acid constructs are vectors, including expression vectors and cloning vectors. The expression vector is a vector suitable for expressing a foreign gene such as a nucleic acid molecule encoding a polypeptide of the present description (such as ApoE2 or ApoE3 full-length sequence or any one of the ApoE constructs, ApoE polypeptides or fusion proteins described herein) in a host cell, including prokaryotic expression vectors and eukaryotic expression vectors. Eukaryotic expression systems include yeast expression systems, mammalian cell expression systems and insect cell expression systems. A cloning vector is used to amplify the target gene (such as the nucleic acid molecule encoding the polypeptide of the present description) in the host cell. Cloning vectors include plasmid vectors, phage vectors, viral vectors, and combinations thereof or combinations with other genomic DNA. The most commonly used host cell in molecular cloning is *Escherichia coli.*

In some embodiments, the vector is a viral vector, including but not limited to lentiviral vectors, herpes simplex viral vectors, adenoviral vectors, and adeno-associated viral (AAV) vectors. Such vectors can be prepared using standard methods in the art. In some embodiments, the vector is a lentivirus or lentiviral vector.

In some embodiments, the vector is a recombinant AAV vector. AAV vectors can be prepared using standard methods in the art. Adeno-associated virus vectors of any serotype may be used, including but not limited to AAV1, AAV2, AAV3, AAV4, AAV5, AAV6, AAV7, AAV8, AAV9, AAV10, AAV1 1, AAV12, AAV13, AAV14, AAV15, and AAV16.

In some embodiments, the description provides a vector (e.g., an AAV or lentiviral vector) comprising an expression cassette which comprises a neuron-specific promoter (e.g., hSyn) operably linked to a nucleic acid molecule of interest, the nucleic acid molecule of interest being selected from: a nucleic acid molecule encoding any of the ApoE C-terminal fragments of the present description, a nucleic acid molecule encoding ApoE2 or ApoE3 full-length protein, a nucleic acid molecule encoding any of the ApoE2 constructs or fusion proteins of the present description, or a nucleic acid molecule encoding any of the ApoE2 polypeptides of the present description. Preferably, the vector is a recombinant AAV vector. Preferably, the neuron-specific promoters suitable for use herein include the synapsin promoter, the thy-1 promoter and the calmodulin-dependent protein kinase II-α promoter. In some embodiments, the nucleic acid molecule of interest encodes any one of amino acid sequence as shown by SEQ ID NOs: 12, 13, 18-34, 46-52, 57-59, 60-62, 76, 78, 87 and 89. In some embodiments, the nucleic acid molecule of interest further encodes an N-terminal signal peptide (e.g., any one of the amino acid sequence as shown by SEQ ID NOs: 66-68). In some embodiments, the nucleic acid molecule of interest further encodes an N-terminal and/or C-terminal cell-penetrating peptide (e.g., any one of the amino acid sequence as shown by SEQ ID NOs: 35-45, 63-65, and 84-86). In some embodiments, the nucleic acid molecule of interest also encodes an N-terminal and/or C-terminal tag protein (e.g., V5, EGFP).

The present description also provides an expression system comprising the nucleic acid molecule, nucleic acid construct or vector disclosed herein. The expression system can be, for example, a Gram-positive or Gram-negative prokaryotic host cell system, such as *E. coli* modified to express a polypeptide of the description. In some embodiments, the expression system is a eukaryotic host cell system, such as yeast, such as *Pichia pastoris* or *Saccharomyces cerevisiae.* In some embodiments, the host cell is a mammalian host cell, such as HEK293T, N2A, CHO cells, and the like. In some embodiments, the host cell is a neural cell, including but not limited to glial cells and neurons. Higher eukaryotic cells, particularly those derived from multicellular organisms, can be used for the expression of glycosylated polypeptides. Suitable higher eukaryotic cells include, but are not limited to, invertebrate cells and insect cells, and vertebrate cells.

Methods suitable for introducing sufficient amounts of a nucleic acid required to express a polypeptide or protein of interest into host cells (e.g., mammalian host cells) are well known in the art. See e.g., Gething et al., Nature, 293:620-625 (1981); Mantei et al., Nature, 281:40-46 (1979); Levinson et al.; EP 117,060; and EP 117,058, all herein cited here for reference. The vector can be introduced into the host cell using any suitable method known in the art, including but not limited to DEAE-dextran-mediated delivery, calcium phosphate precipitation method, cationic lipid-mediated delivery, liposome-mediated transfection, electroporation, particle bombardment, receptor-mediated gene delivery, delivery mediated by polylysine, histones, chitosan and peptides. For mammalian cells, preferred transformation methods include the calcium phosphate precipitation method of Graham and van der Erb, Virology, 52: 456-457 (1978), or the lipofectamine^{™} of Hawley-Nelson, Focus 15: 73 (1193). (Gibco BRL) method. General aspects of mammalian cell host system transformation are described by Axel in U.S. Patent No. 4,399,216, filed August 16, 1983. Various techniques for transforming mammalian cells can be found in Keown et al., Methods in Enzymology (1989), Keown et al., Methods in Enzymology, 185:527-537 (1990), and Mansour et al., Nature, 336:348-352 (1988). Non-limiting representative examples of suitable vectors for expression of polypeptides or proteins in mammalian cells include P(3)NA1; p(3), see Okayama et al. (1985) Mol. Cell Biol. 5:1136-1142; pMClneoPoly-A, see Thomas et al. (1987) Cell 51:503-512; and baculovirus vectors such as PAC373 or pAC610.

In some embodiments, the polypeptide or protein of the description is stably transfected into a host cell. However, one of ordinary skill in the art will recognize that the present description can be used to transiently or stably transfect host cells.

There are many ways to allow the target protein or polypeptide to enter the target cell, such as using liposomes to deliver DNA (such as a plasmid) or RNA encoding the target protein, or using lentivirus or adeno-associated virus encoding the target protein to infect cells.

Any mammalian cell or cell type that is amenable to cell culture and expression of a polypeptide may be used in the present description. Non-limiting examples of mammalian cells that can be used in the present description include BALB/c mouse myeloma cell line (NSO/1, ECACC No: 85110503); human retinoblasts (PER.C6 (CruCel, Leiden, The Netherlands )); SV40-transformed monkey kidney CV1 line (COS-7, ATCC CRL 1651); human fetal kidney cell line (subcloned 293 or 293 cells for growth in suspension medium, Graham et al., J. Gen Virol ., 36:59 (1977)); baby hamster kidney cells (BHK, ATCC CCL 10); Chinese hamster ovary cells +/-DHFR (CH0, Urlaub and Chasin, Proc.Natl.Acad.Scl.USA, 77:4216 (1980)); Mouse Sertoli cells (TM4, Mather, Biol. Reprod., 23:243-251 (1980)); Monkey kidney cells (CVIATCC CCL70); African green monkey kidney cells (VER0-76, ATCC CRL-1587); human cervical cancer cells (HeLa, ATCC CCL 2); dog kidney cells (MDCK, ATCC CCL 34); Buffalo rat hepatocytes (BRL3A, ATCC CRL 1442); human lung cells (Wl38, ATCC CCL 75); human hepatocytes (Hep G2, HB 8065); mouse mammary tumor (MMT 060562, ATCC CCL51); TRI cells (Mather et al., Annals N.Y. Acad. Sci., 383:44-68 (1982)); MRC 5 cells; FS4 cells and human hepatoma cell line (Hep G2); mouse neuronal cells N2A.

The isolated host cell is cultured under conditions that permit expression of the isolated nucleic acid inserted into the vector. Conditions suitable for the expression of polynucleotides may include, but are not limited to, suitable medium, suitable density of host cells in the medium, presence of necessary nutrients, presence of complementary factors, suitable temperature and humidity, and absence of microbial contaminants. One of ordinary skill in the art may appropriately modify the steps and compositions of the present description by selecting appropriate conditions for expression purposes to optimize cell growth and/or production of any given expressed polypeptide or protein.

Various methods of preparing host cells (e.g., mammalian cells) are known in the art. For example, a nucleic acid sufficient for expression (typically a vector comprising a gene encoding a polypeptide or protein of interest and any operably linked genetic control elements) can be introduced into a host cell line by any number of well-known techniques. In general, cells are screened to determine which host cells actually acquire the vector and express the polypeptide or protein of interest. Routine methods for detecting a specific polypeptide or protein of interest expressed by host cells, include but not limited to immunohistochemistry, immunoprecipitation, flow cytometry, immunofluorescence microscopy, SDS-PAGE, Western blot, enzyme-linked immunosorbent assay (ELISA), high performance liquid chromatography (HPLC) techniques, bioactivity assays and affinity chromatography. Those of ordinary skill in the art will be aware of other suitable techniques for detecting expressed polypeptides or proteins. If multiple host cells express a polypeptide or protein of interest, some or all of the techniques described above can be used to determine which cells express the polypeptide or protein at the highest level.

The one or more expressed polypeptides and/or polypeptide complexes can be collected using any suitable method. One or more polypeptides and/or polypeptide complexes can be expressed inside the cell, in the periplasmic space, or secreted outside the cell into the culture medium. If the polypeptide and/or polypeptide complex is expressed intracellularly, the host cell comprising the polypeptide and/or polypeptide complex can be lysed, and the polypeptide and/or polypeptide complex can be isolated from the lysate by centrifugation or ultrafiltration to remove unwanted debris. If polypeptides and/or polypeptide complexes are secreted into the periplasmic space of *E. coli,* the cell paste can be thawed for about 30 minutes in the presence of agents such as sodium acetate (pH 3.5), EDTA, and phenylmethylsulfonyl fluoride (PMSF), and cell debris can be removed by centrifugation (Carter et al., BioTechnology 10:163-167 (1992)). If the polypeptide and/or polypeptide complex is secreted into the culture medium, cell culture supernatants can be collected and concentrated using commercially available protein concentration filters such as Amincon or Millipore Pellicon ultrafiltration devices. Protease inhibitors and/or antibiotics may be included in the collection and concentration steps to inhibit protein degradation and/or growth of contaminating microorganisms.

Polypeptides or proteins can be isolated and purified by standard methods including, but not limited to, chromatography (e.g., ion exchange, affinity, size exclusion, and hydroxyapatite chromatography), gel filtration, centrifugation or differential solubility, ethanol precipitation or any other available protein purification techniques (see e.g. Scopes, Protein Purification Principles and Practice 2nd Edition, Springer-Verlag, New York, 1987; Higgins, S.J. and Hames' B.D. (eds.), Protein Expression: APractical Approach, Oxford Univ Press, 1999; and Deutscher, M.P., Simon, M.1., Abelson, J.N. (eds.), Guide to Protein Purification: Methods in Enzymology (Methods in Enzymology Series, Vol 182), Academic Press, 1997, both incorporated herein by reference). For immunoaffinity chromatography in particular, proteins can be separated by binding them to an affinity column comprising antibodies raised against the protein and immobilized on a solid support. Alternatively standard recombinant techniques can be used to attach an affinity tag such as influenza coat sequence, polyhistidine, glutathione-S-transferase to the protein to allow simple purification through a suitable affinity column. Protease inhibitors such as phenylmethylsulfonyl fluoride (PMSF), leupeptin, pepstatin or aprotinin can be added at any or all stages to reduce or eliminate degradation of the polypeptide or protein during purification. Protease inhibitors are especially needed when cells must be lysed to isolate and purify expressed polypeptides or proteins. Those of ordinary skill in the art should recognize that specific purification techniques vary depending on the properties of the polypeptide or protein to be purified, the properties of the cell expressing the polypeptide or protein, and the composition of the medium for cell growth.

### 4. Pharmaceutical composition and kit

The present description also provides a pharmaceutical composition, which comprises (i) any of the isolated ApoE constructs (such as any amino acid sequence as shown by SEQ ID NOs: 12, 60, 61 and 89), fusion proteins, isolated ApoE polypeptides (for example, any amino acid sequence as shown by SEQ ID NOs: 13, 18-34, 46-52, 57-59, 62, 76, 78 and 87, such as SEQ ID NO: 33), isolated nucleic acids, vectors (for example, a vector for neural-specific expression of an ApoE construct (for example, full-length ApoE2 or ApoE3) or an ApoE polypeptide), or host cells described herein, and (ii) a pharmaceutically acceptable carrier.

The actual dosage level of the active ingredient (i.e. the ApoE construct of the present description, ApoE polypeptide (such as ApoE C-terminus polypeptide), fusion protein or expression vector) in the pharmaceutical composition can be changed to obtain the amount of the active ingredient effective to achieve the following effects: achieving a desired therapeutic response for a particular patient, composition and mode of administration without toxicity to the patient. The selected dosage level will depend on a variety of pharmacokinetic factors, including the activity of the polypeptide or expression vector used in the description, the route of administration, time of administration, duration of treatment, other medicaments used in combination with the particular composition used, compound and/or substance, age, sex, weight, conditions, general health, and prior medical history of the patient being treated, and similar factors well known in the medical arts.

Herein, pharmaceutically acceptable carriers include diluents, adjuvants, excipients or vehicles, including but not limited to water, animal oil, vegetable oil, starch, glucose, lactose, sucrose, gelatin, sodium stearate, glycerol monostearate esters, sodium chloride, skimmed milk powder, glycerin, propylene, propylene glycol, water, ethanol, etc. The composition, if desired, can also contain minor amounts of wetting or emulsifying agents, or pH buffering agents. Typically, the physiologically acceptable carrier is a pH buffered aqueous solution. Acceptable carriers, excipients, or stabilizers are nontoxic to the cells of the recipient to which they are exposed at the dosages and concentrations employed, and comprise buffers, such as phosphates, citrates, and other organic acids; antioxidants, including ascorbic acid and methionine; preservatives (such as octadecyldimethylbenzyl ammonium chloride; hexamethonium chloride; benzalkonium chloride, benzethonium chloride; phenol, butanol or benzyl alcohol; para alkyl hydroxybenzoates such as methylparaben or propylparaben; catechol; resorcinol; cyclohexanol; 3-pentanol; and m-cresol); low molecular weight (less than about 10 residues) polypeptides; proteins such as serum albumin, gelatin or immunoglobulins; hydrophilic polymers such as polyvinylpyrrolidone; amino acids such as glycine, glutamine, asparagine, histidine, arginine acids or lysine; monosaccharides, disaccharides, and other carbohydrates, including glucose, mannose, or dextrin; chelating agents, such as EDTA; sugars, such as sucrose, mannitol, trehalose or sorbitol; ions, such as sodium; metal complexes (e.g., Zn-protein complexes); and/or nonionic surfactants, such as TWEENT^{™}, PLURONICS^{™}, or polyethylene glycol (PEG).

In some embodiments, the pharmaceutical composition is formulated to have a pH in the range of about 4.5 to about 9.0, including for example any of about 5.0 to about 8.0, about 6.5 to about 7.5, or about 6.5 to about 7.0 pH range. In some embodiments, the pharmaceutical composition can also be made isotonic with blood by the addition of a suitable tonicity adjusting agent, such as glycerol.

Pharmaceutical compositions to be used for *in vivo* administration are generally formulated as sterile substantially isotonic pharmaceutical compositions and in full compliance with all Good Manufacturing Practice (GMP) regulations of the United States Food and Drug Administration. Sterility is readily achieved by filtration through sterile filtration membranes. In some embodiments, the composition is pathogen-free. For injection, the pharmaceutical compositions may be in liquid solutions, e.g., in physiologically compatible buffers such as Hank's solution or Ringer's solution. Additionally, the pharmaceutical composition may be in solid form and reconstituted or suspended immediately prior to use. Also provided herein are lyophilized compositions.

Herein, the pharmaceutical composition may be in the form of solutions, suspensions, emulsions, tablets, pills, capsules, powders, sustained release formulations and the like. The polypeptide of the present description can be prepared into a suitable dosage form according to the specific administration mode and administration route.

The pharmaceutical composition can be adapted for various modes of administration as described herein, including, for example, systemic or localized administration. In some embodiments, the pharmaceutical composition is formulated for intravenous administration or subcutaneous injection. In some embodiments, the pharmaceutical composition is formulated for local administration, such as local administration to a tumor site (e.g., intratumoral injection), or local administration (e.g., injection) to the brain (e.g., cortex or hippocampus).

In some embodiments, the pharmaceutical composition is formulated according to conventional procedures as a pharmaceutical composition suitable for intravenous, intraperitoneal or intracerebral injection. Typically, injectable compositions are solutions in sterile isotonic aqueous buffer. If necessary, the composition may also include a solubilizer and a local anesthetic such as lignocaine to relieve pain at the injection site. Generally, the ingredients are presented separately or mixed together in unit dosage form, for example, as a dry lyophilized powder or water-free concentrate in an airtight container such as an ampoule or sachet indicating the quantity of active agent. When the composition is to be administered by infusion, it can be dispensed with an infusion bottle comprising sterile pharmaceutical grade water or saline. When the composition is administered by injection, an ampoule comprising sterile water for injection or saline can be provided so that the ingredients can be mixed prior to administration.

In some embodiments, the pharmaceutical composition is suitable for administration to humans. In some embodiments, the pharmaceutical composition is suitable for administration to rodents (e.g., mice, rats, rabbits) or non-human primates (e.g., monkeys). In some embodiments, the pharmaceutical composition is contained in a single use vial, such as a single use sealed vial. In some embodiments, the pharmaceutical composition is contained in a multiple-use vial. In some embodiments, the pharmaceutical composition is contained in bulk in a container. In some embodiments, the pharmaceutical composition is cryopreserved.

The description also provides unit dosage forms of the isolated ApoE construct, fusion protein, isolated ApoE polypeptide, isolated nucleic acid, vector, host cell, or composition thereof (such as a pharmaceutical composition) described herein. The term "unit dosage form" refers to physically discrete units suitable as unitary dosages for individuals, each unit comprising a predetermined quantity of active material calculated to produce the desired therapeutic effect, in association with a suitable pharmaceutical carrier, diluent or excipient. These unit dosage forms may be stored in suitable packaging in single or multiple unit doses and may also be further sterilized and hermetically sealed.

The application also provides a product that includes the composition described herein (such as a pharmaceutical composition) in a suitable packaging. Packaging suitable for the compositions described herein, such as pharmaceutical compositions, are known in the art and include, for example, vials (such as sealed vials), containers, ampoules, bottles, jars, flexible packaging (such as sealed Mylar or plastic bags), etc. These articles can be further sterilized and/or sealed.

The description also provides kits comprising the isolated ApoE construct, fusion protein, isolated ApoE polypeptide, isolated nucleic acid, vector, host cell, or pharmaceutical composition of any of the herein described. In some embodiments, the kit also includes one or more instructions for methods of using the protein, expression vector, or composition, such as the uses described herein. The kits described herein may also include other materials that may be desirable from a commercial and user standpoint, including other buffers, diluents, filters, needles, syringes, and packaging inserts with instructions on any method described in this description.

### 5. Disease treatment methods

The present description also provides the use of the isolated ApoE constructs (such as any amino acid sequence as shown by SEQ ID NOs: 12, 60, 61 and 89), fusion protein, isolated ApoE polypeptide (for example, any amino acid sequence as shown by SEQ ID NOs: 13, 18-34, 46-52, 57-59, 62, 76, 78 and 87, such as SEQ ID NO: 33), isolated nucleic acid, vector (for example, a vector for neural-specific expression of an ApoE construct (for example, full-length ApoE2 or ApoE3) or an ApoE polypeptide), the host cell or pharmaceutical composition in the preparation of medicaments for the treatment or prevention of diseases related to the γ-secretase enzymatic digestion activity (such as γ-cleavage of APP) in an individual. In some embodiments, the medicament inhibits the γ-secretase enzymatic digestion activity (such as the γ-cleavage activity of APP), so as to treat or prevent the diseases related to the γ-secretase enzymatic digestion activity. In some embodiments, the present description provides a method for treating or preventing a disease associated with γ-secretase digestion activity (e.g., γ-cleavage activity of APP) in an individual (e.g., human), comprising administering to the individual an effective dose of any isolated ApoE construct (such as any amino acid sequence as shown by SEQ ID NOs: 12, 60, 61 and 89), fusion protein, isolated ApoE polypeptide (such as any amino acid sequence as shown by SEQ ID NOs: 13, 18-34, 46-52, 57-59, 62, 76, 78 and 87, such as SEQ ID NO: 33), isolated nucleic acid, vector (such as a vector for neural-specific expression of an ApoE construct (for example, full-length ApoE2 or ApoE3) or an ApoE polypeptide), host cell or pharmaceutical composition of the present description. In some embodiments, the method inhibits the γ-secretase enzymatic digestion activity (such as the γ-cleavage activity of APP), so as to treat or prevent the diseases related to the γ-secretase enzymatic digestion activity. In some embodiments, the disease associated with the γ-secretase enzymatic digestion activity is selected from the group consisting of: neurodegenerative diseases, tumors or cancers, inflammatory diseases and renal diseases. In some embodiments, the diseases are selected from the group consisting of: Alzheimer's disease and related diseases, amyloid cerebrovascular disease, Down's syndrome, other neurodegenerative diseases associated with aging (such as frontotemporal dementia), head and neck cancers (such as head and neck squamous cell carcinoma and oral squamous cell carcinoma), breast cancer, liver cancer, pancreatic cancer (including metastatic pancreatic cancer), ovarian cancer, lung cancer (such as non-small cell lung cancer, lung adenocarcinoma and small cell lung cancer), glioma (e.g., malignant glioma), fibroma, lymphoma (e.g., B-cell lymphoma), osteosarcoma, gastric cancer, bladder cancer, asthma (e.g., allergic asthma), pneumonia, airway inflammation, acute kidney injury, clear cell renal cell carcinoma, renal fibrosis, and obstructive nephropathy. In some embodiments, the disease is AD and related diseases, such as sporadic Alzheimer's disease (sAD).

The medicine, treatment or prevention method provided by the present description is applicable to any individual, including but not limited to mammals, birds, frogs, fish, fruit flies, nematodes and the like. Mammals include, but are not limited to, primates (such as humans, or non-human primates such as monkeys), rodents (such as mice, rats, hamsters, gerbils, squirrels, guinea pigs, and rabbits), livestock (such as pigs, cattle, sheep, horses, donkeys), pets (such as cats, dogs, rabbits and hamsters), etc. In some embodiments, the individual is a mouse or a monkey. In some embodiments, the individual is a human.

Herein, suitable administration methods and administration routes are well known in the art, and suitable administration routes include intravenous, intramuscular, intradermal, intraperitoneal, subcutaneous, spinal or other parenteral administration routes, such as via injection or perfusion. "Parenteral administration" is a mode of administration other than enteral and topical administration, usually by injection, and includes, but is not limited to, intravenous, intramuscular, intraarterial, intrathecal, intrathecal, intraorbital, intracardiac, intradermal, intraperitoneal, nasal, transtracheal, subcutaneous, subepidermal, intra-articular, subcapsular, subarachnoid, intraspinal, epidural, and intrasternal injections and infusions. The terms "systemic administration" and "systemically administered" refer to a method which the administration of an agent or composition described herein to an individual (such as a mammal) such that the agent or composition is delivered to a site (including the target site of drug action) in the body via the circulatory system. Systemic administration includes, but is not limited to, administration of oral, intranasal, rectal, and parenteral (e.g., other than through the alimentary canal, such as intramuscular, intravenous, intraarterial, transdermal, and subcutaneous).

In some embodiments, any of the isolated ApoE constructs (such as any amino acid sequence as shown by SEQ ID NOs: 12, 60, 61 and 89), fusion proteins, isolated ApoE polypeptides (such as any amino acid sequence as shown by SEQ ID NOs: 13, 18-34, 46-52, 57-59, 62, 76, 78 and 87, such as SEQ ID NO: 33), isolated nucleic acid, vector, or pharmaceutical composition of the present description, via any suitable routes into the central nervous system, including intraventricular and intrathecal injections. Intraventricular injection can be assisted, for example, via an intracerebroventricular catheter connected to a depot, such as the Ommaya depot.

In some embodiments, nucleic acid molecules encoding polypeptides of the description, such as coding sequences for polypeptides of the description, including RNA and DNA sequences, can be delivered using techniques well known in the art. In some embodiments, a coding sequence for a polypeptide of the description is delivered to a subject in need (e.g., a human) in an expression vector (e.g., using a viral vector, e.g., AAV or lentiviral vector). In some embodiments, liposomes are used to deliver DNA or RNA molecules described herein that encode polypeptides of the description. Thus, in these embodiments, the pharmaceutical compositions of the description comprise liposomes together with the DNA or RNA molecules encoding the polypeptides of the description and their expression vectors described herein. The liposomes suitable for the present description may be liposomes known in the art and suitable for human administration.

The ApoE construct (such as full-length ApoE2 or ApoE3), fusion protein, or ApoE polypeptide provided by the present description is an inhibitor of γ-secretase (such as specific inhibition of γ-cleavage of APP), so the polypeptide or its pharmaceutical compositions of the present description are useful for the treatment or prevention of diseases benefiting from the inhibition of γ-secretase, targeting the inhibition of γ-secretase for therapeutic or prophylactic benefit (also referred to herein as γ-secretase mediated diseases or diseases related to the γ-secretase enzymatic digestion activity). A variety of diseases are known to be mediated by γ-secretase, including but not limited to: neurodegenerative diseases such as Alzheimer's disease and related diseases and amyloid cerebrovascular diseases (Erik D. Roberson and Lennart Mucke, 100 years and Counting: Prospects for Defeating Alzheimer's Disease, 314: 781-784 (2006); Bart De Strooper et al., Presenilins and γ-Secretase: Structure, Function, and Role in Alzheimer's Disease, 2: a006304 (2012); Weiming Xia, γ-Secretase and its modulators: Twenty years and beyond, Neuroscience Letters 701:162-169(2019)); tumors or cancers, including head and neck cancers such as head and neck squamous cell carcinoma and oral squamous cell carcinoma (Liang Mao et al., γ-Secretase inhibitor reduces immunosuppressive cells and enhances tumor immunity in head and neck squamous cell carcinoma, Int.J.Cancer: 142, 999-1009 (2018)), breast cancer and hepatocellular carcinoma (Hui Jia et al., γ-Secretase inhibitors for breast cancer and hepatocellular carcinoma: From mechanism to treatment, Life Sciences 268(2021)119007), pancreatic cancer (including metastatic pancreatic cancer) (Ana De Jesus-Acosta et al., A Phase II Study of the Gamma Secretase Inhibitor RO4929097 in Patients with Previously Treated Metastatic Pancreatic Adenocarcinoma, Invest New Drugs.2014August; 32(4):739-745), Ovarian Cancer (Zhaoyi Feng, Inhibition of gamma-secretase in Notch1 signaling pathway as a novel treatment for ovarian cancer, Oncotarget, 2017, Vol.8 , (No.5), pp:8215-8225), lung cancer (such as non-small cell lung cancer or lung adenocarcinoma and small cell lung cancer) (Antonio Maraver, Therapeutic effect of γ-secretase inhibition in KrasG12V-driven non-small cell lung carcinoma through derepression of DUSP1 phosphatase and inhibition of ERK, Cancer Cell. 2012 August 14; 22(2); Katherine M. Morgan et al., Gamma secretase inhibition by BMS-906024 enhances efficacy of paclitaxel in lung adenocarcinoma, Mol Cancer The r.2017 December; 16(12):2759-2769), gliomas such as malignant glioma (Edward Pan et al., Phase I Study of RO4929097 with Bevacizumab in Patients with Recurrent Malignant Glioma, Neurooncol.2016 December; 130(3):571-579), fibromatosis (Shivaani Kummar et al., Clinical Activity of the g-Secretase Inhibitor PF-03084014 in Adults With Desmoid Tumors (Aggressive Fibromatosis), JOURNAL OF CLINICAL ONCOLOGY, VOLUME 35, NUMBER 14, MAY 10, 2017), lymph tumors such as B-cell lymphoma (Shuji Tohda, Establishment of a novel B-cell lymphoma cell line with suppressed growth by gamma-secretase inhibitors, Leukemia Research 30 (2006) 1385-1390), gastric cancer (Hyun-Woo Lee et al., Targeting Notch signaling by c-secretase inhibitor I enhances the cytotoxic effect of 5-FU in gastric cancer, Clin Exp Metastasis (2015) 32: 593-603), bladder cancer (YIBING WANG et al., γ-secretase inhibitor inhibits bladder cancer cell drug resistance and invasion by reducing epithelial-mesenchymal transition, MOLECULAR MEDICINE REPORTS 12:2821-2827, 2015), osteosarcoma (M Tanaka et al., Inhibition of Notch pathway prevents osteosarcoma growth by cell cycle regulation, British Journal of Cancer (2009) 100,1957-1965); inflammatory diseases, including asthma, such as allergic asthma (Weixi Zhang et al., γ-Secretase Inhibitor Alleviates Acute Airway Inflammation of Allergic Asthma in Mice by Downregulating Th 17 Cell Differentiation, Mediators of Inflammation, Volume 2015, Article ID 258168, 7 pages), airway inflammation such as allergic airway inflammation (Weixi Zhang et al., γ-Secretase Inhibitor Alleviates Acute Airway Inflammation of Allergic Asthma in Mice by Downregulating Th17 Cell Differentiation, Mediators of Inflammation, Volume 2015, Article ID 258168), pneumonia (Jin Hyun Kang et al., g-Secretase Inhibitor Reduces Allergic Pulmonary Inflammation by Modulating Th1 and Th2 Responses, AMERICAN JOURNAL OF RESPIRATORY AND CRITICAL CARE MEDICINE VOL 179, 2009) etc.; kidney disease, such as acute kidney injury (Jean-Christophe Wyss et al., Targeted γ-secretase inhibition of Notch signaling activation in acute renal injury, Am J Physiol Renal Physiol 314: F736-F746, 2018).

In some embodiments, the disease associated with the γ-secretase digestion activity (such as the γ-cleavage activity of APP) is a neurodegenerative disease, such as Alzheimer's disease (AD), dementia with Lewy bodies (DLB), mild cognitive impairment (MCI), frontotemporal dementia (FTD), cerebral amyloid angiopathy (CAA), CAA-related cerebral hemorrhage, vascular cognitive impairment, Parkinson's disease (PD), multiple sclerosis (MS), traumatic brain injury (TBI), or Fragile X-associated tremor/ataxia syndrome. CAA is a condition characterized by the deposition of proteins, primarily β-amyloid, in the walls of blood vessels in the brain. CAA is common in AD patients but can also occur in the absence of evidence of AD.

In some embodiments, the disease associated with γ-secretase enzymatic digestion activity (e.g., γ-cleavage activity of APP) is "amyloid β-associated disease", which term refers to any disorder characterized by the abnormally high levels of production, accumulation and/or aggregation of β-amyloid peptides, e.g., in the form of amyloid plaques, especially in the brain. Amyloid β-associated diseases include AD, Down syndrome, Fragile X syndrome, CAA, CAA-associated intracerebral hemorrhage, and sporadic inclusion body myositis.

AD is the most common neurodegenerative disease in developed countries. Histopathologically, AD is characterized by the accumulation of amyloid plaques comprising Aβ peptides and NFTs made of tau protein. Clinically, AD is associated with progressive cognitive impairment characterized by loss of memory, function, language ability, judgment, and executive functions. AD often causes severe behavioral symptoms in its later stages.

Roughly half of early-onset Alzheimer's cases (ie, diagnosed before age 65) are familial Alzheimer's disease (FAD), and are inherited in an autosomal dominant manner. FAD is caused by mutations in at least one of three genes: APP mutation, PSEN1 mutation, and PSEN2 mutation. Amyloid precursor protein (APP) mutations have been identified in 121 families. The most common missense mutations replace the Val⁷¹⁷ residue with an Ile (London mutation), Phe, or Gly residue, replace the Glu⁶⁹³ with a Gln residue (Dutch mutation) or Gly residue (Arctic mutation), or replace the Lys⁶⁷⁰ and Met⁶⁷¹ with Asn and Leu residues (the double mutation is called the Swedish mutation and is numbered in the APP₇₇₀ isoform). Two hundred nineteen presenilin 1 (PSEN1) have been identified in 480 families with attenuated γ-secretase cleavage, increased oxidative stress in some mutations and increased neuronal susceptibility to DNA damage-induced death. Thirteen presenilin 2 (PSEN2) have been identified in 34 families with attenuated γ-secretase cleavage and increased oxidative stress in some mutations.

In some embodiments, any isolated ApoE construct (such as any amino acid sequence as shown by SEQ ID NOs: 12, 60, 61 and 89), fusion protein, isolated ApoE polypeptide (such as any amino acid sequence as shown by SEQ ID NOs: 13, 18-34, 46-52, 57-59, 62, 76, 78 and 87, such as SEQ ID NO: 33), isolated nucleic acid, vector (such as a vector for neural-specific expression of an ApoE construct (for example, full-length ApoE2 or ApoE3) or an ApoE polypeptide), host cells or pharmaceutical compositions provided by the present description can be used to treat any disease (such as AD) related to γ-cleavage of APP by inhibiting the γ-secretase enzymatic activity, for example, with any APP mutational diseases (such as AD). In some embodiments, the APP is human APP comprising one or more of the following mutations: K670N, M671L, Swedish (K670N+M671L), Florida (I716V), London (V717I), APPM_{596V} (incapable of β cleavage), fAD mutations near γ cleavage sites (e.g., T714I, V717F, L723P), fAD mutations near β- or α-cleavage sites (e.g., KM670/671NL, E693K, E693Δ), or protective Icelandic mutations (A673T).

Several behavioral and histopathological tests for assessing the Alzheimer's disease phenotype, for characterizing therapeutic agents, and evaluating treatment are known in the art. Histological analysis is usually performed postmortem. Histological analysis of Aβ levels can be performed using Thioflavin-S. Visualization of Aβ deposition on sliced brain tissue by Congo red, or anti-Aβ staining (e.g., 4G8, 10D5, or 6E10 antibodies) (see, e.g., Holcomb et al., 1998, Nat. Med. 4:97-100; Borchelt et al., 1997, Neuron 19:939-945; Dickson et al., 1988, Am. J. Path. 132:86-101). In vivo methods to visualize Aβ deposits in transgenic mice have also been described. BSB ((trans,trans)-1-bromo-2,5-bis-(3-hydroxycarbonyl-4-hydroxy)styrylbenzene) and PET tracer ¹¹C-labeled Pittsburgh compound-B (PIB) binds to Aβ plaques (see, e.g., Skovronsky et al., 2000, Proc. Natl. Acad. Sci. USA 97:7609-7614; Klunk et al., 2004, Ann. Neurol. 55:306-319). The ¹⁹F-comprising amyloidophilic Congo red-type compound FSB ((E, E)-1-fluoro-2,5-bis-(3-hydroxycarbonyl-4-hydroxy)styrylbenzene) allows Aβ plaques to be visualized by MRI. Block visualization (see, e.g., Higuchi et al., 2005, Nature Neurosci. 8:527-533). Radiolabeled, putrescine-modified β-amyloid peptides label amyloid deposits in vivo in a mouse model of Alzheimer's disease (see, e.g., Wengenack et al., 2000, Nat. Biotechnol. 18:868-872).

A subject suffering from Alzheimer's disease can be identified using standard diagnostic methods known in the art for Alzheimer's disease. Typically, the diagnosis of Alzheimer's disease is based on the patient's symptoms (e.g., progressive decline in memory function, progressive abandonment and frustration of normal activities, apathy, restlessness or irritability, aggressiveness, anxiety, sleep disturbance, upset, abnormal motor behavior, disinhibition, social withdrawal, decreased appetite, hallucinations, dementia), medical history, neuropsychological testing, neurological and/or physical examination. Cerebrospinal fluid can also be tested for a variety of proteins associated with Alzheimer's pathology, including tau, amyloid-β peptide, and AD7C-NTP. Genetic testing is also available for early-onset familial Alzheimer's disease (eFAD), an autosomal dominant genetic disorder. Clinical genetic testing is available for individuals with symptoms of AD or at-risk family members of patients with early-onset disease. In the United States, testing for PS2 mutations and APP is available in clinical or federally approved clinical laboratories under the Clinical Laboratory Improvement Amendments. Commercial tests for PS1 mutations are also available (Elan Pharmaceuticals).

The therapeutic effect of the present description can be evaluated by any of the methods or indicators mentioned above. In some embodiments, the ApoE constructs, ApoE polypeptides, vectors, compositions, or treatment/prevention methods of the present description can achieve one or more of the following effects: (i) regulate APP splicing in neurons (in vivo or in vitro) through cellular autonomy; (ii) inhibit (for example, inhibit at least about 10%, 20%, 30%, 40%, 50%, 60%, 70%, 80%, 90%, 95%, or 100% of) γ-secretase enzymatic activity, such as inhibition of γ-secretase-mediated γ-cleavage of APP, similar to the inhibitory effect of γ-secretase inhibitors on γ-cleavage of APP (such as PF03084014); (iii) decrease (e.g., reduce by at least about 10%, 20%, 30%, 40%, 50%, 60%, 70%, 80%, 90%, 95%, or 100%) amyloidogenic pathway of APP, such as reduce (such as reduce by at least about 10%, 20%, 30%, 40%, 50%, 60%, 70%, 80%, 90%, 95%, or 100%) the generation of β-amyloid (Aβ) (e.g., Aβ40 and/or Aβ42), or reduce (for example reduce by at least about 10%, 20%, 30%, 40%, 50%, 60%, 70%, 80%, 90%, 95%, or 100%) the γ-cleavage of C-terminal fragment-β (β-CTF); (iv) increase (e.g. increase by at least about 10%, 20%, 30%, 40%, 50%, 60%, 70%, 80%, 90%, 95%, 100%, 2-fold, 5-fold, 10-fold, 20-fold or higher) non-amyloidogenic pathway of APP, e.g., increase (e.g., increase by at least about 10%, 20%, 30%, 40%, 50%, 60%, 70%, 80%, 90%, 95%, 100%, 2-fold, 5-fold, 10-fold, 20-fold or higher) generation of C-terminal fragment-α (α-CTF); (v) do not affect (or affect no more than about 30%, 20%, 10%, 5% or less) mRNA and/or protein expression of α-secretase, β-secretase, and/or γ-secretase (or subunits thereof) (e.g., ADAM10, BACE1, PS1 and/or PS2); (vi) do not affect (or affect no more than about 30%, 20%, 10%, 5% or less) β-secretase-mediated β-cleavage of APP, and/or α-secretase-mediated α-cleavage of APP; (vii) do not inhibit (or inhibit no more than about 30%, 20%, 10%, 5% or less) γ-cleavage of non-APP proteins (such as APLP1 (APP like protein 1) or Notch) or fragments thereof, and do not increase (or increase by no more than about 30%, 20%, 10%, 5% or less) the α-CTF generated by γ-cleavage of non-APP proteins; (viii) reduce (e.g., reduce by at least about 10%, 20%, 30%, 40%, 50%, 60%, 70%, 80%, 90%, 95%, or 100%) or prevent amyloid plaques in the brain, e.g., reduction (e.g., reduction of at least about 10%, 20%, 30%, 40%, 50%, 60%, 70%, 80%, 90%, 95%, or 100%) of average plaque size, total area, and/or density of amyloid plaques, reduce (e.g., reduce by at least about 10%, 20%, 30%, 40%, 50%, 60%, 70%, 80%, 90%, 95%, or 100%) or prevent generation of intraneuronal Aβ (such as Aβ40 and/or Aβ42), slow down (such as slow down at least about 10%, 20%, 30%, 40%, 50%, 60%, 70%, 80%, 90%, 95%, 100%, 2-fold, 5-fold, 10-fold or more) AD pathological progression, increase (e.g., increase by at least about 10%, 20%, 30%, 40%, 50%, 60%, 70%, 80%, 90%, 95%, 100%, 2-fold, 5-fold, 10-fold, 20-fold or higher) individual survival and/or lifespan; (ix) neuronal local expression (e.g. in cortex, tooth Gyrus (DG) and hippocampal CA3 expression) of the ApoE constructs of the present description can migrate from a distance to the subiculum and accumulate around amyloid plaques, thereby locally inhibit the enzymatic activity of γ-secretase in these amyloidogenic hotspots or (x) reduce (e.g. reduce by at least about 10%, 20%, 30%, 40%, 50%, 60%, 70%, 80%, 90%, 95%, or 100%), eliminate, or prevent (including slowing the onset of) one or more AD symptoms (e.g., progressive decline in memory function, progressive abandonment and frustration of normal activities, apathy, restlessness or irritability, aggressiveness, anxiety, sleep disturbance, upset, abnormal motor behavior, disinhibition, social withdrawal, decreased appetite, hallucinations, dementia).

In some embodiments, the cancers associated with the γ-secretase enzymatic digestion activity include one or more of the following: head and neck cancer (such as head and neck squamous cell carcinoma and oral squamous cell carcinoma), breast cancer, liver cancer, pancreatic cancer (including metastatic pancreatic cancer), ovarian cancer, lung cancer (such as non-small cell lung cancer, lung adenocarcinoma, and small cell lung cancer), glioma (such as malignant glioma), fibroma, lymphoma (such as B-cell lymphoma tumor), osteosarcoma, gastric cancer, bladder cancer.

In some embodiments, the medicaments or methods described herein for treating cancers associated with the γ-secretase enzymatic digestion activity have one or more of the following biological activities: (1) kill cancer cells, for example at least about any one of 30% , 40%, 50%, 60%, 70%, 80%, 90%, 95% or higher tumor cell death rate; (2) inhibit the proliferation of cancer cells, such as inhibit at least about 10% (including, for example, at least about any one of 20%, 30%, 40%, 60%, 70%, 80%, 90%, or 100%) of proliferation; (3) reduce tumor size, for example, by at least about 10%, 20%, 30%, 40%, 60%, 70%, 80%, 90%, or 100% of tumor size; (4) alleviate one or more symptoms in individuals with cancer; (5) inhibit tumor metastasis (such as metastasis to lymph nodes), such as inhibit tumor metastasis by at least about 10% (including, for example, at least about any one of 20%, 30%, 40%, 60%, 70%, 80%, 90%, or 100%) of tumor metastasis; (6) prolong survival, e.g. prolong any one of at least 1, 2, 3, 4, 5, 6, 7, 8, 9, 10, 11, 12, 18 or 24 months or longer; (7) prevent cancer or prolong the time to cancer progression, such as prolong the time to cancer progression by at least any one of 1, 2, 3, 4, 5, 6, 7, 8, 9, 10, 11, or 12 months or more any of the times; and (8) prevent, inhibit or reduce the likelihood of cancer recurrence.

In some embodiments, the disease associated with the γ-secretase enzymatic digestion activity is an inflammatory disease, including but not limited to asthma (such as allergic asthma), pneumonia, and airway inflammation. In some embodiments, the medicaments or methods of treatment/prevention described herein can reduce (e.g., reduce by at least about any one of 10%, 20%, 30%, 40%, 60%, 70%, 80%, 90%, or 100%) inflammation triggers secretion and/or activity of cytokines, and/or alleviate (for example, alleviate by at least about any one of 10%, 20%, 30%, 40%, 60%, 70%, 80%, 90%, or 100%), eliminate or prevent (including slow the time of onset) one or more disease symptoms.

In some embodiments, the disease associated with the γ-secretase enzymatic digestion activity is renal disease, which refers to any disease, disorder or condition affecting the kidney or renal system. Examples of kidney-related diseases or conditions include, but are not limited to, acute kidney disease (or failure), clear cell renal cell carcinoma, renal fibrosis, or obstructive kidney disease. Conventional markers for evaluating and diagnosing renal diseases include GFR, creatinine and albumin, and any one of the markers or related methods can be used to evaluate the therapeutic effect of the present description. In some embodiments, the medicaments or methods of treatment/prevention described herein can (i) reduce (e.g., reduce by at least about any one of 10%, 20%, 30%, 40%, 60%, 70%, 80%, 90%, or 100%), eliminate or prevent (including slow the time to onset) one or more indicators of renal disease; and/or (ii) protect or improve renal function.

The present description provides a method for treating or preventing a disease mediated by γ-secretase in an object (such as a human), the method comprising administering to the object a therapeutically effective amount or a preventively effective amount of any one of the ApoE constructs, fusion proteins, ApoE polypeptides, nucleic acid molecules, vectors or pharmaceutical composition thereof. Effective amounts can be determined by those skilled in the art based on factors such as the subject's age, sex, weight, conditions, general health, and previous medical history. The diseases preferably include the aforementioned degenerative diseases of the nervous system, tumors or cancers, inflammatory diseases, kidney diseases and the like.

Some embodiments of the present description provide a method of inhibiting the γ-secretase enzymatic digestion, thereby inhibiting the amyloidogenic pathway of APP and reducing the production of Aβ, the method comprising producing within cells (such as neurons) expressing γ-secretase with ApoE2 (for example, ApoE2 with a cell-penetrating peptide at the N-terminal) or the ApoE construct (such as a C-terminal fragment of ApoE2, or full-length ApoE2 or ApoE3), the fusion protein or the ApoE polypeptide (such as any amino acid sequences as shown by SEQ ID NOs: 13, 18-34, 46-52, 57-59, 62, 76, 78 and 87, such as SEQ ID NO: 33) described in any embodiment of this description. In some embodiments, the cells are neurons. In some embodiments, the method is used to slow down (e.g., slow down by at least about any one of 10%, 20%, 30%, 40%, 60%, 70%, 80%, 90%, or 100%), prevent or treat the occurrence and development of AD and amyloid cerebrovascular disease. Production as described herein includes expression production of the polypeptide and delivery of the polypeptide into the cell from outside the cell. In some embodiments, liposomes are used to deliver the DNA or RNA encoding the ApoE construct (such as ApoE2 and/or ApoE3 and/or its C-terminal fragment) or ApoE polypeptide into the cell, so that the ApoE construct or ApoE polypeptide is produced by expression in the cell; or the ApoE construct or ApoE polypeptide is produced in the cell by infecting cells with lentivirus and adeno-associated virus; or the ApoE construct or ApoE polypeptide into the cell by using cell-penetrating peptide or enhanced cellular endocytosis manner.

In some embodiments, the polypeptides mentioned in the ApoE constructs or fusion proteins, uses and methods described in any of the embodiments herein particularly include any one of the polypeptide as shown by SEQ ID NOs: 12, 13, 18-33 and 89.

### EXAMPLES

The disclosure is further illustrated by the specific examples described below. It should be understood that these examples are merely illustrative, and do not limit the scope of the present disclosure. In addition, it should be understood that based on the teaching content of the disclosure, those skilled in the art can practice various changes or modifications to the disclosure, and these equivalent forms also fall within the scope of the appended claims.

In the examples, data were analyzed by using GraphPad Prism software, and all data were shown as mean±SEM. The following data analysis used unpaired, paired or one sample t-test. Significant differences are shown by asterisks (*). One asterisk (*) represents a P value less than 0.05; two asterisks (**) represents a P value less than 0.01; three asterisks (***) represents a P value less than 0.001; four asterisks (****) represent a P value less than 0.0001.

### Example 1

### 1. Main reagents and materials

The HEK 293T cell line was cultured in DMEM medium (HyClone) containing 10% fetal bovine serum (FBS) (Life Company) and 1% penicillin/streptomycin, and cultured in a sterile incubator (ThermoFisher Company) containing 5% CO₂ at 37°C .

Primary cultured neurons were derived from the cortex or hippocampus of 16-day embryonic mice. The mouse cortex or hippocampus was obtained under a stereoscope, and was digested at 37°C for 10 minutes with 20 U/ml papain (Worthington Company). After digestion, the neurons that exist in single-cell form were seeded with 100,000 neurons per well (24-well plate); the neurons were cultured in the neuron culture medium (Neurobasal medium; ThermoFisher Company) containing 2% B27 (ThermoFisher Company) and 1% penicillin/streptomycin; the fresh culture medium was changed once a week.

0-7 months old C57 wild-type mice and AD model mice (5×FAD mice [Tg6799], mice expressing APP and PS1 comprising pathogenic mutations. The expressed APP comprises three mutations: K670N/M671L, I716V, V717I; and PS1 comprises two mutations: M146L, L286V). The mice were given sufficient food and water, and were reared at room temperature with a 12h/12h light-dark cycle, and the feeding environment reached the SPF (Specific Pathogen Free) standard.

Enzyme-linked immunosorbent assay kits (ELISA) were purchased from Invitrogen, including human Aβ40 (KHB3481), human Aβ42 (KHB3441), and mouse Aβ40 (KMB3481).

Transfection reagents were purchased from ThermoFisher's lipofectamine2000 and SignaGen's Poly jet.

The Quantitect SYBR-Green qRT-PCR kit for qRT-PCR was purchased from Qiagen.

**Table 1: Antibody Information**

| Reagent | Source | Catalog number |
|---|---|---|
| Rabbit anti- APP [Y188] (monoclonal) | Abcam | ab32136 |
| Rabbit anti-ApoE [EP1374Y] (monoclonal) | Abcam | ab52607 |
| Rabbit anti-ApoE [EP1373Y] (monoclonal) | Abcam | ab51015 |
| Rabbit anti-PS1 [EP2000Y] (monoclonal) | Abcam | ab76083 |
| Rabbit anti-PS2 [EP1515Y] (monoclonal) | Abcam | ab51249 |
| Mouse anti-GAPDH (monoclonal) | Proteintech | 60004-1-lg |
| Rabbit anti-alpha/beta-Tubulin (polyclonal) | Cell Signaling Technology | 2148 |
| Chicken anti-MAP2 (polyclonal) | Synaptic Systems | 188006 |

### 2. Experimental Methods

### 2.1 Protein sample preparation and Western blot

a) A plasmid expressing the target protein in mammalian cells was constructed, and the protein expression promoter is CAG.
b) The target protein was expressed in HEK 293T cell line. Cells were subcultured one day before transfection, and transfection was performed when the degree of cell adhesion reached 70%-80%. The transfection reagent was lipofectamine2000 (ThermoFisher).
c) 4-6 hours post transfection, the medium were replaced with fresh cell culture medium.
d) 36-48 hours post transfection, the cells were lysed (10% 1M tris-HCl (pH=6.8), 4% SDS, 20% glycerol, 0.01% bromophenol blue), and the protein samples were collected and used for Western blot or stored in a -80°C freezer.
e) Polyacrylamide gel electrophoresis. Before protein loading, the protein was boiled at 98°C for 10 min, and then centrifuged at 12000 rpm for 10 min. Electrophoresis: 80V for the upper stacking gel; 120V for the lower separating gel.
f) Protein transfer. The filter paper was soaked in the electrophoresis solution before use, and the PVDF membrane was put in methanol for 10s to activate, and the gel after electrophoresis was taken out from the electrophoresis tank, put in the order of filter paper - PVD membrane - gel-filter paper from bottom to top, and they were put into the transfer instrument, with transferring in constant current at 0.1 A for 70 min.
g) Blocking and antibody incubation: the transferred PVDF membrane was placed in 5% BSA/TBST blocking solution, and blocked on a shaker at room temperature for 1 hour. The primary antibody was used to incubate at 4°C overnight on a shaker (12-16h); 1×TBST was used to wash three times, once every 10 minutes; the secondary antibody was used to incubate, shaked at room temperature for 1-2 h; 1×TBST was used to wash three times, once every 10 minutes.
h) Imaging by development: development was performed with ECL chemiluminescence hypersensitive chromogenic reagent.

### 2.2 Brain slice preparation and immunohistochemistry

a) The brain was taken by perfusion. The mice were perfused at the heart, the blood was washed out with 20 ml PBS, and then the mice were perfused with 20 ml 4% PFA.
b) Fixation and dehydration. The mice were fixed with 4% PFA at 4°C for 24 hours, and then dehydrated with 30% sucrose at 4°C.
c) Mouse brain slices. After 24 hours of dehydration, the mice brain was cut into slices at a thickness of 40 µm, stored in frozen buffer solution or used for subsequent immunohistochemistry.
d) THS staining. The mouse brain slices were washed three times with ddH₂O, 5 minutes each; soaked in 1% THS aqueous solution for 10 minutes; washed twice with 80% ethanol, each time for 3 minutes; washed once with 95% ethanol for 3 minutes. Immunohistochemistry was performed subsequently. If THS staining is not performed, the following immunohistochemistry was performed directly.
e) Immunohistochemistry. The mouse brain slices were washed three times with 1×PBS for 10 minutes each time; the cell membrane was permeated using 0.1% TritonX100; the brain slices were blocked by using 5% donkey or goat serum; then the primary antibody was incubated at 4°C, shaken overnight (12-16h); washed three times with 1 ×PBS for 10 minutes each time; the secondary antibody was incubated, shaken at room temperature for 1-2h; washed three times with 1×PBS for 10 minutes each time.
f) Patching and sealing.
g) Imaging, 24 hours post sealing.
h) Images were processed and signal density was analyzed by Image J.

### 2.3 Enzyme-linked immunosorbent assay (ELISA)

The experiment was performed according to the standard procedure in the ELISA analysis kit of Invitrogen Company.
a) The target protein was expressed in HEK 293T cell line. Cells were subcultured one day before transfection, and transfection was performed when the degree of cell adhesion reached 70%-80%. The transfection reagent was lipofectamine2000 (ThermoFisher).
b) 4-6 hours post transfection, medium were replaced with fresh cell culture medium.
c) 36-48 hours post transfection, the cell culture medium was collected, and then the ELISA process was performed or the cell culture medium was frozen in a -80°C refrigerator.
d) 50 µl of standard samples and experimental samples were added to each well of the 96-well plate.
e) 50 µl of human-derived Aβ40 or Aβ42 or mouse-derived Aβ40 detection antibody was added to each well of the 96-well plate.
f) The above samples were shaken gently and placed at room temperature for 3 hours.
g) The liquid in the well were removed, and the plate was washed 4 times with 1×wash buffer.
h) 100 µl of the buffer containing 1 ×anti-Rabbit IgG HRP was added.
i) The plate was shaken gently and placed at room temperature for 30 hours.
j) The liquid in the well were removed, and the well was washed 4 times with 1 ×wash buffer.
k) 100 µl of Stabilized Chromogen was added to each well, and the plate was placed in the dark for 30 minutes at room temperature.
l) 100 µl of Stop Solution was added to each well.
m) The absorbance at 450 nm was measured with a microplate reader.

### 2.4 qRT-PCR

RNA was extracted from the treated cells according to the manual of TRIzol^{™} Reagent (ThermoFisher).
a) The target protein was expressed in HEK 293T cell line. Cells were subcultured one day before transfection, and transfection was performed when the degree of cell adhesion reached 70%-80%. The transfection reagent was lipofectamine2000 (ThermoFisher).
b) 6 hours post transfection, medium was replaced with fresh cell culture medium.
c) 48 hours after transfection, RNA was extracted.
d) 500 µl Trizol (ThermoFisher Company) was added to each well of the 24-well plate, and the plate was shaken, and kept at room temperature for 20 minutes.
e) The cell suspension was collected into a centrifuge tube.
f) 250 µl of chloroform was added, then the centrifuge tube was mixed thoroughly by inverting, and placed at room temperature for 3 minutes.
g) Centrifugation at 12000 g for 15 minutes at 4°C. After centrifugation, the solution appeared to be clearly stratified. The bottom was chloroform containing phenol (pink), the middle was DNA and protein, and the uppermost layer was the aqueous phase containing RNA (transparent and clear). The supernatant was transferred to a new centrifuge tube.
h) an equal volume (supernatant) of isopropanol was added, then the centrifuge tube was inverted to mix, and placed at room temperature for 20 minutes.
i) The tube was centrifuged at 12000 g for 15 minutes at 4°C. The supernatant was removed, and a small amount of white precipitate (RNA) was visible at the bottom.
j) 400 µl of 75% ethanol was added. The tube was centrifuged at 12000 g for 5 minutes at 4°C.
k) Ethanol was removed as much as possible, and dried thoroughly in a fume hood, but without over-drying. An appropriate amount of DEPC water was added.
l) After DNA removal, the samples were stored at -80°C for subsequent experiments.

Quantitect SYBR-Green qRT-PCR kit from Qiagen was used to quantitatively analyze the relative content of mRNA. qRT-PCR primers were listed in Table 2.

**Table 2: Primers for qRT-PCR**

| | | |
|---|---|---|
| ADAM10 | F | 5'-AGAGCAACATCTGGGGACAAA-3' (SEQ ID NO:1) |
| | R | 5'-TCCACAAATAGGTTGGCCAGAT-3' (SEQ ID NO:2) |
| | R | 5'-GCTCTGGGAGAGCCAACATA-3' (SEQ ID NO:3) |
| BACE1 | F | 5'-AACGAATTGGCTTTGCTGTCA-3' (SEQ ID NO:4) |
| | R | 5'-AGGCTATGGTCATGAGGGTTG-3' (SEQ ID NO: 5) |
| PS1 | F | 5'-TGACTCTCTGCATGGTGGTG-3' (SEQ ID NO: 6) |
| | R | 5'-TCAGAATTGAGTGCAGGGCT-3' (SEQ ID NO: 7) |
| PS2 | F | 5'-TTTGAGCCTCCCTTGACTGG-3' (SEQ ID NO:8) |
| | R | 5'-GGTATTCCAGTCCCCGCTG-3' (SEQ ID NO:9) |

### 2.5 Construction of PS1 KO, PS2 KO and PS1/2 dKO cell lines

In the present description, HEK 293T cells were selected, and PS1 KO, PS2 KO and PS1/2 dKO cell lines were constructed using CRISPR/CAS9 technology according to the standard procedure published by Zhang Feng et al. in 2013 (Ran et al., 2013). The sgRNAs for specifically knocking out PS1 and PS2 were designed by using the website: https://zlab.bio/guide-design-resources, as shown by Table 3.
a) Expression of PS2 sgRNA and control empty plasmid in HEK 293T cells. Cells were subcultured one day before transfection, and transfection was performed when the degree of cell adhesion reached 70%-80%. The transfection reagent was lipofectamine2000 (ThermoFisher).
b) 6 hours post transfection, medium was replaced with fresh cell culture medium.
c) 18 hours post transfection, medium was replaced with fresh culture medium comprising 3 µg/ml puromycin (Sellect).
d) When the HEK 293T cell line transfected with the empty plasmid died completely under the action of 3 µg/ml puromycin, the HEK 293T cells transfected with PS2 sgRNA were collected.
e) The HEK 293T cells transfected with PS2 sgRNA were passaged into a 10 cm cell culture dish at a ratio of 1:400 to ensure that the cells propagate in a monoclonal form.
f) After the monoclonally propagated cells grow into a cell mass, the single cell mass was collected and passaged into a 12-well plate.
g) When the cell mass grows to cover the entire 12-well plate, the cells were collected. Half of the cells were used to extract DNA and protein, and the DNA sequence and protein expression of PS2 were detected. Another fraction of cells was used for passaging.
h) Cells in which the DNA sequence of PS2 had been edited and no longer express PS2 protein was PS2 knockout cell lines-PS2 KO cell lines.
i) A PS1/PS2 dKO cell line of the present description was constructed based on the PS2 KO cell line. PS1 sgRNA or control empty plasmid was further expressed in PS2 KO cell line; and steps a-g were performed for detection of DNA sequence and protein expression of PS1.
j) The PS2 KO cell line in which the DNA sequence of PS1 had been edited and no longer expresses PS1 protein was PS1 and PS2 double knockout cell line-PS1/2 KO cell line.

**Table 3: sgRNAs**

| | |
|---|---|
| PS1 sgRNA | 5'-GATTATCTAATGGACGACCCC-3' (SEQ ID NO:10) |
| PS2 sgRNA | 5'-GCGTGCTTCGCTCCGTATTTG-3' (SEQ ID NO:11) |

### 2.6 Virus preparation and injection.

### 2.6.1 Lentivirus preparation

a) Construction of lentivirus expression plasmids, lentivirus-GFP and lentivirus-ApoE CT, which can be specifically expressed in neurons.
b) The lentivirus vectors were cotransfected with coat protein vectors (PSPAX2 and PMD2G) in HEK 293T cell line. Cells were subcultured one day before transfection, and transfection was performed when the degree of cell adhesion reached 70%-80%. The transfection reagent was Polyjet (SignaGen).
c) 36-48 hours after the transfection, the cell culture was collected and filtered through a 0.22 µm filter membrane.
d) The filtered cell culture solution was ultracentrifuged. Centrifuged at 20000 rpm, 4°C for 2 hours and 5 minutes.
e) After centrifugation, the supernatant was removed, and the precipitation of every three precipitates of 10 cm cell culture medium was resuspended with 50 µl of PBS.
f) The resuspended virus was subpackaged and stored in a -80°C refrigerator for subsequent experiments.

### 2.6.2 Preparation of adeno-associated virus

Adeno-associated virus AAV-CT (AAV2/9-hSyn-ApoE CT) and AAV-GFP (AAV2/9-hSyn-EGFP-WPRE-pA) expressed in neurons were purchased from Tertu Biotechnology Company.

### 2.6.3 Virus injection

1 µl of the adeno-associated virus prepared above was injected into the cerebral cortex (x, y, z, +/-1.6,0, -1) and hippocampus (x, y, z, +/-1.5, -2, -1.5) of 1-month-old mice by using a stereotaxic injection device.

### 2.7 Protein purification

### 2.7.1 Purification of γ-secretase

For the expression vector of human γ-secretase, see Lu et al., 2014.
a) Human γ-secretase was expressed in HEK 293T cell line (four proteins of human γ-secretase: NCT, PS1, PLAG-PEN2, APH were expressed simultaneously in the same expression plasmid). Cells were subcultured one day before transfection, and transfection was performed when the degree of cell adhesion reached 70%-80%. The transfection reagent was lipofectamine2000 (ThermoFisher).
b) 4-6 hours post transfection, medium was replaced with fresh cell culture medium.
c) 48 hours post transfection, the cells were washed once with 1×PBS on ice, scraped off with a cell scraper, and collected by centrifugation at 800 g for 5 min.
d) The resuspended cells were lysed with lysis solution [25 mM HEPES, pH 7.4; 150 mM NaCl, 5 mM MgCl₂, 1 mM dithiothreitol, 0.05% (v/v) Tween-20, 1×cocktail].
e) the lysed cells were sonicated, 20 cycles (3 seconds on, 3 seconds off), 45% power output.
f) The above cells were centrifuged at 3000g for 10 minutes at 4°C, and the supernatant was collected into a new centrifuge tube.
g) 15000g, the above supernatant was centrifuged at 4°C for 1 hour, and the supernatant was discarded.
h) The precipitate was resuspended with 1 ml of lysate, 1% CHAPSO was added after resuspension, and the mixture was incubated at 4°C for 2 hours. The above precipitate was centrifuged at 15,000g for 30 minutes, and the supernatant was collected into a new centrifuge tube.
i) 100 µl of FLAG beads (SIGMA) was added to the supernatant and the mixture was incubated at 4° C for 2 hours.
j) The mixture was washed three times with the following buffer. 25 mM HEPES, pH7.4; 150 mM NaCl, 0.1% digitonin.
k) The human γ-secretase was eluted with the following buffer. 25 mM HEPES, pH 7.4; 150 mM NaCl, 0.1% digitonin and 500 µg/ml 3xFLAG peptide (SIGMA).
l) The purified human γ-secretase was detected by Western blot; stored at -80°C.

### 2.7.2 Purification of ApoE

a) The ApoE2 and ApoE2NT were expressed in HEK 293T cell line. ApoE2 and ApoE2NT proteins contain fusion expressed FLAG. Cells were subcultured one day before transfection, and transfection was performed when the degree of cell adhesion reached 70%-80%. The transfection reagent was lipofectamine2000 (ThermoFisher).
b) 4-6 hours post transfection, medium was replaced with fresh cell culture medium.
c) 48 hours post transfection, the cells were washed once with 1 ×PBS on ice, scraped off with a cell scraper, and collected by centrifugation at 800 g for 5 min.
d) The resuspended cells were lysed with lysis solution [25 mM HEPES, pH 7.4; 150 mM NaCl, 1 ×cocktail].
e) the lysed cells were sonicated, 20 cycles (3 seconds on, 3 seconds off), 45% power output.
f) The cells were centrifuged to obtain the supernatant comprising protein, 12000 g, 15 minutes, 4°C, the supernatant was collected into a new centrifuge tube.
g) The protein-containing supernatant was incubated with anti-FLAGM2 affinity resin (SIGMA) for 90 minutes at 4°C. After incubation, ApoE2 and ApoE2NT were transferred to anti-FLAG M2 affinity resin.
h) The anti-FLAG M2 affinity resin comprising ApoE2 and ApoE2NT was washed three times with the following buffer. 25 mM HEPES, pH 7.4; 150 mM NaCl, 5 mM MgCl₂, 1 mM DTT, 0.05% (v/v) Tween-20.
i) ApoE2 and ApoE2 NT were eluted with the following buffer. 25 mM HEPES, pH 7.4; 150 mM NaCl and 500 µg/ml 3xFLAG peptide (SIGMA).
j) The purified ApoE2 and ApoE2NT were detected by Western blot; stored at - 80°C.

### 2.7.3 Purification of β-CTF

a) β-CTF was expressed in *Escherichia coli,* the expression plasmid was pET22β-CTF-Myc-6xHis.
b) The *E. coli* was induced by 0.5 mM IPTG and expressed at 37°C for 4 hours, and lysed with the following lysate. 30 mM Tris-HCl, pH 7.5; 150 mM NaCl, 1 mM EDTA.
c) The lysate was centrifuged at 25000 g for 10 minutes at room temperature. The supernatant was removed.
d) The precipitate was resuspended with the following buffer and mixed slowly at room temperature. 30 mM Tris-HCl, pH 8.0; 300 mM NaCl, 1 mM EDTA, 6 M urea, 1% TritonX-100, 1 mM CaCl₂, 1×cocktail.
e) The precipitate was centrifuged at 40000 g for 30 minutes, and discarded, and the supernatant was filtered through a 0.45 mM filter membrane.
f) The filtered supernatant was incubated with the Ni-NTA-beads at room temperature for 2 hours, and then the incubated Ni-NTA-beads and the supernatant were loaded into an empty chromatography column at the same time to make them flow out under the action of gravity. The flow-through was collected and allowed to bind to the beads once.
g) The Ni-NTA-beads were washed with 10 times the Ni-NTA-bead column volume of the following buffer: 30 mM Tris-HCl, pH 8.0; 300 mM NaCl, 1 mM EDTA, 6 M urea, 1% TritonX-100, 1 mM CaCl₂, 1×cocktail.
h) The Ni-NTA-beads were washed with 20 times Ni-NTA-bead column volumes of the following buffer: 30 mM Tris-HCl, pH 8.0; 300 mM NaCl, 0.1% Trion X-100.
i) The Ni-NTA-beads were washed with 20 times Ni-NTA-bead column volumes of the following buffer : 50 mM Tris-HCl, pH 8.0; 300 mM NaCl, 0.1% CHAPSO.
j) β-CTF was eluted with the following buffers: 50 mM Tris-HCl, pH 8.0; 300 mM NaCl, 0.1% CHAPSO, 300 mM imidazole.
k) The purified β-CTF was detected by Western blot; stored at -80°C.

### 2.7.4 Purification of GFP

The purification method of GFP was the same as the purification method of ApoE in 2.7.2.

### 3. Experimental results

### 3.1 Effects of three ApoE isoforms and different protein fragments of ApoE on APP metabolism

In the present description, human APP was co-expressed with ApoE2, ApoE3 or ApoE4 in HEK 293T cells, and GFP was used as a control group, and Western blot was used to measure the production amount of α-CTF. The results of Western blot (FIG. 1, panels A-B) showed that ApoE2 and ApoE3 co-expressed with APP can significantly increase the non-amyloid metabolite α-CTF of APP; while ApoE4 co-expressed with APP cannot significantly change α-CTF (compared with α-CTF of the GFP control group). The present description further explored the influence of ApoE on APP amyloidogenic pathway. Aβ is the product of amyloidogenic pathway, and the less Aβ is produced, the weaker the amyloidogenic pathway of APP is. In the present description, human wild-type APP and β-secretase were respectively co-expressed with ApoE2, ApoE3 or ApoE4 in HEK 293T cells, and GFP was used as a control group to detect the influence of ApoE on production of Aβ. ELISA results (Fig. 1, panel C) showed that expression of ApoE2 significantly reduces production of Aβ40, but ApoE3 and ApoE4 have no such effect. Expression of ApoE2 also significantly reduces Aβ40 compared to ApoE4. These results indicated that ApoE2 expressed in the same cells as APP can attenuate the amyloidogenic pathway of APP, reduce Aβ40, and increase the non-amyloid metabolite α-CTF of APP. ApoE4 does not have this effect.

The present description further studied which fragments of ApoE function to weaken APP amyloidogenic pathway. ApoE2, ApoE3 and ApoE4 only have two different amino acid residues in the N-terminal domain, and have identical amino acid residue sequences in the C-terminal domain. Therefore, the present description constructed the expression plasmids of the N-terminal fragment and the C-terminal fragment of human ApoE, and named the plasmids respectively ApoE2NT, ApoE3NT, ApoE4NT or ApoECT protein for subsequent experiments. When expressed in cells, the N-terminus of all constructed proteins were further linked with the signal peptide of human ApoE itself (SEQ ID NO: 66). APP was co-expressed with ApoE2 NT, ApoE4 NT and ApoE CT in HEK 293T cells, respectively, and GFP was used as the control group. The results of Western blot (FIG. 1, panels D-E) showed that only co-expression of ApoE CT significantly increases α-CTF to more than 5 times the original level. Co-expression of either ApoE2 NT or ApoE4 NT do not alter α-CTF. Meanwhile, the ELISA results showed that ApoE CT significantly reduces Aβ 40 to 25% of GFP control, all three ApoE NTs do not decrease Aβ40 (FIG. 1, Panel F); similarly, ApoE CT significantly reduces Aβ42 to about 26% of GFP control (FIG. 1, Panel G).

AD is a neurological disease. In the brain, Aβ is mainly produced by cleavage of APP expressed in neurons. The present description constructed a lentivirus expressing ApoE CT in neurons. ELISA results (FIG. 1, panel H) showed that overexpression of ApoE CT in neurons can reduce endogenously produced Aβ40 in primary cultured mouse hippocampal or cortical neurons to about 71% and about 73% of the control group, respectively. Likewise (FIG. 1, panel I), overexpression of ApoECT in human pluripotent stem cell-induced neurons also reduces the endogenous production of Aβ40 by human neurons to about 54% of controls.

These results indicated that in a variety of cells, ApoE2 can increase the non-amyloid metabolite α-CTF of APP through its C-terminal fragment in a cell-autonomous manner; at the same time, it can reduce the amyloidogenic pathway and reduce Aβ.

The ApoE CT amino acid sequence used in each experiment of the present description is as follows:

ARMEEMGSRTRDRLDEVKEQVAEVRAKLEEQAQQIRLQAEAFQARLKSWFE
PLVEDMQRQWAGLVEKVQAAVGTSAAPVPSDNH (SEQ ID NO: 33).

### 3.2 ApoE2 and ApoE CT secreted in the culture medium do not affect the cleavage of APP

HEK 293T cells expressing ApoE2 or ApoE CT were cultured to obtain culture medium, and the results showed that the culture medium contains secreted ApoE2 or ApoE CT (FIG. 2, panel A). HEK 293T cells expressing APP were co-incubated with this medium containing ApoE2 or ApoE CT. HEK 293T cells expressing APP were co-incubated with culture medium containing GFP as a negative control. The results showed that ApoE2 and ApoE CT in co-incubated cultures does not significantly alter α-CTF levels compared to GFP control (Fig. 2, panels B-C). Immunofluorescence results (Fig. 2, panels D-E) showed that even after prolonged co-incubation, ApoE2 and ApoE CT in the culture medium mainly adhered to the cell surface and hardly entered the cell. However, ApoE2 and ApoE CT expressed in HEK 293T cells (FIG. 2, panel D) or mouse primary cultured neurons (FIG. 2, panel E) can exist in large quantities in the cells.

This result indicated that ApoE2 and ApoE CT in cell culture medium can not change the production of α-CTF and Aβ. ApoE2 and ApoE CT must be in a cell-autonomous manner, that is, they must be in the same cell as APP, in order to regulate the cleavage of APP, so as to achieve the effect of increasing α-CTF and reduce the production of Aβ. There are many ways to allow ApoE2 and ApoE CT to enter the target cells, specifically including: liposomes transferred DNA or RNA encoding such proteins. Unless otherwise specified, the present description used liposomes to transfect plasmids encoding corresponding proteins into cells to express ApoE and ApoE CT; the invasion of cells by lentiviruses and adeno-associated viruses can lead to the expression of ApoE2 and ApoE CT in cells expressing APP. The present description used lentivirus or adeno-associated virus expression systems to express ApoE2 or ApoE CT in neurons; cell-penetrating peptide, enhanced cell endocytosis, and other methods can enable ApoE2 and ApoE CT proteins to enter target cells expressing APP. The present description further explored the role of cell-penetrating peptide in the following text.

### 3.3 the core sequence of ApoE CT that attenuates APP amyloidogenic pathway

In order to explore which amino acids on ApoE CT are the core sequences for changing APP metabolism, the present description added 1, 2, 3, 4 or 5 original amino acid residues of ApoE to the N-terminus of ApoE CT to construct plasmids expressing CT NA1, CT NA2, CT NA3, CT NA4, and CT NA5, respectively; deleted 1, 2, 3, 4, or 5 amino acids at the N-terminus of ApoE CT to construct plasmids expressing CT ND1, CT ND2, CT ND3, CT ND4, and CT ND5, respectively: the original 5 and 10 amino acids of ApoE were deleted at the C-terminus of ApoE CT, and plasmids expressing CT CD5 and CT CD10 were constructed. The amino acid sequences of the above CT NA1, CT NA2, CT NA3, CT NA4, CT NA5, CT ND1, CT ND2, CT ND3, CT ND4, CT ND5, CT CD5 and CT CD10 are respectively as shown by SEQ ID NOs: 13-24. When expressed in cells, the N-terminus of all constructed proteins were further linked with the signal peptide of ApoE itself (SEQ ID NO: 66).
SEQ ID NO: 13 (CT NA1): 
RARMEEMGSRTRDRLDEVKEQVAEVRAKLEEQAQQIRLQAEAFQARLKSWF
EPLVEDMQRQWAGLVEKVQAAVGTSAAPVPSDNH;
SEQ ID NO: 14 (CT NA2): 
LRARMEEMGSRTRDRLDEVKEQVAEVRAKLEEQAQQIRLQAEAFQARLKS
WFEPLVEDMQRQWAGLVEKVQAAVGTSAAPVPSDNH;
SEQ ID NO: 15 (CT NA3): 
RLRARMEEMGSRTRDRLDEVKEQVAEVRAKLEEQAQQIRLQAEAFQARLKS
WFEPLVEDMQRQWAGLVEKVQAAVGTSAAPVPSDNH;
SEQ ID NO: 16 (CT NA4): 
ERLRARMEEMGSRTRDRLDEVKEQVAEVRAKLEEQAQQIRLQAEAFQARLK
SWFEPLVEDMQRQWAGLVEKVQAAVGTSAAPVPSDNH;
SEQ ID NO: 17 (CT NA5): 
**GERLR**ARMEEMGSRTRDRLDEVKEQVAEVRAKLEEQAQQIRLQAEAFQARL
KSWFEPLVEDMQRQWAGLVEKVQAAVGTSAAPVPSDNH;
SEQ ID NO: 18 (CT ND1): 
RMEEMGSRTRDRLDEVKEQVAEVRAKLEEQAQQIRLQAEAFQARLKSWFEP
LVEDMQRQWAGLVEKVQAAVGTSAAPVPSDNH;
SEQ ID NO: 19 (CT ND2): 
MEEMGSRTRDRLDEVKEQVAEVRAKLEEQAQQIRLQAEAFQARLKSWFEPL
VEDMQRQWAGLVEKVQAAVGTSAAPVPSDNH;
SEQ ID NO: 20 (CT ND3): 
EEMGSRTRDRLDEVKEQVAEVRAKLEEQAQQIRLQAEAFQARLKSWFEPLVE
DMQRQWAGLVEKVQAAVGTSAAPVPSDNH;
SEQ ID NO: 21 (CT ND4): 
EMGSRTRDRLDEVKEQVAEVRAKLEEQAQQIRLQAEAFQARLKSWFEPLVE
DMQRQWAGLVEKVQAAVGTSAAPVPSDNH;
SEQ ID NO: 22 (CT ND5): 

;
SEQ ID NO: 23 (CT CD5): 
ARMEEMGSRTRDRLDEVKEQVAEVRAKLEEQAQQIRLQAEAFQARLKSWFE
PLVEDMQRQWAGLVEKVQAAVGTSAAPV;
SEQ ID NO: 24 (CT CD10):

ARMEEMGSRTRDRLDEVKEQVAEVRAKLEEQAQQIRLQAEAFQARLKSWFE
PLVEDMQRQWAGLVEKVQAAVGT.

In HEK 293T cells, human APP and β-secretase were co-expressed with each of the above plasmids, and GFP was used as a control group, and the effects of the above proteins on the production of Aβ40 were determined by ELISA. The results of ELISA (FIG. 3, panels A-B) showed that after adding one of its original amino acid residues to the N-terminus of ApoE CT, CT NA1 still reduces the production of Aβ40; but adding two or more amino acid residues, CT NA2, CT NA3, CT NA4 and CT NA5 completely lost the ability to reduce Aβ. ApoEND 1, ApoEND2, ApoEND3, ApoEND4 and ApoEND5 can still reduce Aβ40 after deletion of 1-5 amino acid residues at the N-terminus of ApoE CT. Even if 5 or 10 amino acid residues are deleted at the C-terminus of ApoE CT, CT CD5 and CT CD10 still reduce the production of Aβ. These results indicated that the N-terminus of ApoE CT is very important, and even a change of only 1 or 2 amino acids may affect the activity of ApoE CT in inhibiting the production of Aβ40. However, the influence of its C-terminal residues on the activity is relatively weak.

According to the above results, on the basis of deleting three amino acid residues (CT ND3) at the N-terminus of ApoE CT, this description constructs plasmids expressing CT ND3CD10, CT ND3CD11, CT ND3CD12, CT ND3CD13, CT ND3CD14, CT ND3CD15, CT ND3CD20, and CT ND3CD25 by deleting 10, 11, 13, 14, 15, 20, or 25 amino acid residues at the C-terminus of ApoE CT, respectively, the amino acid sequences of which are shown in SEQ ID NOs: 25-32. When expressed in cells, the N-terminus of all constructed proteins were further linked with the signal peptide of ApoE itself (SEQ ID NO: 66).
SEQ ID NO: 25 (CT ND3CD10): 
EEMGSRTRDRLDEVKEQVAEVRAKLEEQAQQIRLQAEAFQARLKSWFEPLVE
DMQRQWAGLVEKVQAAVGT;
SEQ ID NO: 26 (CT ND3CD11): 
EEMGSRTRDRLDEVKEQVAEVRAKLEEQAQQIRLQAEAFQARLKSWFEPLVE
DMQRQWAGLVEKVQAAVG;
SEQ ID NO: 27 (CT ND3CD12): 
EEMGSRTRDRLDEVKEQVAEVRAKLEEQAQQIRLQAEAFQARLKSWFEPLVE
DMQRQWAGLVEKVQAAV;
SEQ ID NO: 28 (CT ND3CD13): 
EEMGSRTRDRLDEVKEQVAEVRAKLEEQAQQIRLQAEAFQARLKSWFEPLVE
DMQRQWAGLVEKVQAA;
SEQ ID NO: 29 (CT ND3CD14): 
EEMGSRTRDRLDEVKEQVAEVRAKLEEQAQQIRLQAEAFQARLKSWFEPLVE
DMQRQWAGLVEKVQA;
SEQ ID NO: 30 (CT ND3CD15): 
EEMGSRTRDRLDEVKEQVAEVRAKLEEQAQQIRLQAEAFQARLKSWFEPLVE
DMQRQWAGLVEKVQ;
SEQ ID NO: 31 (CT ND3CD20): 
EEMGSRTRDRLDEVKEQVAEVRAKLEEQAQQIRLQAEAFQARLKSWFEPLVE
DMQRQWAGL;
SEQ ID NO: 32 (CT ND3CD25):

EEMGSRTRDRLDEVKEQVAEVRAKLEEQAQQIRLQAEAFQARLKSWFEPLVE
DMQR.

In HEK 293T cells, human APP, β-secretase were co-expressed with each of the above plasmids, and GFP was used as a control group, and the effects of the above proteins on the production of Aβ40 in cells were determined by ELISA. ELISA results (Fig. 3, panel C) showed that after deletion of 3 amino acid residues from the N-terminus of ApoE CT and deletion of 10, 11, 12, 13, 14, 15, 20 or 25 amino acids from the C-terminal, CT ND3CD10, CT ND3CD11, CT ND3CD12, CT ND3CD13, CT ND3CD14, CT ND3CD15, CT ND3CD20 or CT ND3CD25 can still significantly reduce Aβ40. These results indicated that the end of the core sequence which the ApoE CT inhibits Aβ production is between the 274th residue to the last amino acid residue of ApoE protein.

### 3.4 ApoE CT affects the cleavage of APP through γ-cleavage

In order to study the mechanism by which ApoE CT affects APP cleavage, the present description detected whether ApoE CT can change the expression level of key APP secretory enzymes. The present description used qRT-PCR and Western blot to detect the mRNA and protein expression levels of ADAM10 (main α-secretase), BACE1 (β-secretase), PS1 and PS2 (γ-secretase active subunits), respectively. qRT-PCR (FIG. 4, panel A) and Western blot (FIG. 4, panel B) results showed that overexpression of ApoE CT in cells does not change the mRNA and protein expression compared to GFP control, indicating that ApoE CT does not affect APP cleavage by changing the expression of APP metabolic enzymes.

α-CTF is a substrate of γ-secretase, and Aβ is the cleavage product of γ-secretase. Therefore, when the γ-secretase enzymatic activity changed, the production of α-CTF and Aβ will change significantly, and ApoE CT just changes the production of α-CTF and Aβ. Therefore, ApoE CT may change the activity of γ-secretase. To verify this conjecture, the present description co-expressed the active center PS1 of APP and γ-secretase in HEK293T cells. The results of Western blot (Fig. 5, panels A-B) showed that overexpression of PS1 (which can enhance the activity of γ-secretase) decreases α-CTF. Whereas, expression of ApoE CT or treatment with 1 uM γ-secretase inhibitor PF03084014 significantly increases α-CTF (Fig. 5, panels A-B). This result suggested that the effect of ApoE CT expressed in cells on APP cleavage is similar to inhibition of γ-secretase. β-CTF is the product of APP cleaved by β-secretase and also the substrate of γ-secretase. It only needs one step of γ-cleavage to generate Aβ. ELISA results (Fig. 5, panels C-D) showed that the expression of ApoE CT not only reduces Aβ40 produced by β-CTF to about 80% of the original level, but also reduces Aβ42 to about 8% of the original level. The competence of ApoE CT reduces Aβ may be achieved through regulation of γ-cleavage. The present description further co-expressed APP (APP_{ΔAICD}) without the APP intracellular C-terminal domain, BACE1 (β-secretase) and ApoE CT in HEK 293T cells. APP_{ΔAICD} does not need γ-cleavage. Only one step of β-cleavage is needed to generate Aβ. ELISA results (Fig. 5, panel E) showed that expression of ApoE CT no longer reduces Aβ produced by APP_{ΔAICD}, indicating that ApoE CT does not affect β-cleavage.

These results indicated that ApoE CT expressed in cells requires γ-cleavage to reduce Aβ, and overexpressed ApoE CT mimics the effect of γ-secretase inhibitors; ApoE CT may increase α-CTF and reduce Aβ by inhibiting γ-cleavage of APP.

### 3.5 Regulation of the cleavage of APP by ApoE CT requires the participation of γ-secretase

The active center of γ-secretase is PS1 or PS2. After knocking out PS1 and PS2 (PS 1/2 dKO), α-CTF cannot be metabolized by γ-secretase enzymatic digestion, so it accumulates in large quantities. Western blot results (Fig. 6, panels A and D) showed that in wild-type HEK 293T cell line, expressed ApoE CT significantly increases α-CTF; in PS 1/2 dKO cells expressing GFP, compared with the wild-type HEK 293T cell line, the content of α-CTF is significantly increased. Expression of ApoE CT in PS 1/2 dKO cells does not further increase α-CTF, indicating that in the absence of γ-secretase, ApoE CT can no longer increase α-CTF. ApoE CT expressed in PS1/2 dKO cells supplemented with PS1 or PS2 can significantly increase α-CTF (FIG. 6, panels B-D), indicating that ApoE CT increases α-CTF dependent on PS1 or PS2. Likewise, overexpression of ApoE CT can reduce Aβ40 in PS1/2 dKO cell lines supplemented with PS2 expression (Fig. 6, panel E). These results indicated that ApoE CT can regulate APP metabolism only in the presence of γ-secretase.

### 3.6 ApoE directly inhibits the cleavage of β-CTF by γ-secretase in the purification system

In order to further verify whether ApoE directly inhibits γ-secretase, the present description purified β-CTF, ApoE2, ApoE2NT and γ-secretase, and immunoblotting verified these purified proteins (FIG. 7, panel A). PS1, NCT and PEN2 are the constituent proteins of γ-secretase. The results of ELISA (Fig. 7, panel B) showed that co-incubation of γ-secretase and β-CTF can produce Aβ, and the addition of 10 µM γ-secretase inhibitor (PF03084014) at this time can inhibit the generation of Aβ. Addition of purified ApoE2 to this reaction inhibits Aβ40 production in a concentration-dependent manner with half-inhibitory concentration (IC50) of about 1.2 µM (FIG. 7, panel C). In addition, at the same concentration (2.5 µM), ApoE2 can significantly inhibit the production of Aβ, but ApoE2 NT cannot inhibit the production of Aβ40 ( FIG. 7 , panel D). These results indicated that ApoE2 can directly inhibit the cleavage of β-CTF by γ-secretase, and ApoE CT is necessary for this activity of ApoE, suggesting that ApoE CT is the key fragment for ApoE2 to inhibit cleavage of APP by γ-secretase.

### 3.7 ApoE CT does not inhibit γ-cleavage of APLP1

APLP1 (APP like protein 1) is a protein with a structure similar to APP, and it is also one of the substrates of α and γ secretases. Western blot results (FIG. 8) showed that the γ-secretase inhibitor PF03084014 can inhibit the γ-cleavage of APLP1 in HEK293T cells and significantly increase the α-like CTF; however, the co-expression of ApoE CT does not increase the α-like CTF of APLP1, indicating that ApoE CT does not inhibit the γ-cleavage of APLP1. This result indicated that ApoE CT has certain substrate selectivity in inhibiting the enzymatic digestion activity of γ-secretase.

### 3.8 ApoE CT expressed in neurons reduces amyloid plaques in the brains of AD model mice

Adeno-associated virus capable of expressing ApoE CT in neurons was injected into the cortex and hippocampus of 4-week-old AD model mice (5×FAD). After expressing ApoE CT (or GFP as a negative control) in neurons for six months, its effect on amyloid plaques was examined, and ApoE CT, GFP, and amyloid plaques were stained. The results showed that the area of amyloid plaques in the brains of 5 ×FAD mice expressing ApoE CT (N-terminus linked to the signal peptide of human ApoE itself (SEQ ID NO: 66)) in neurons is smaller, the fluorescence intensity is weaker, the total area of amyloid plaques accounts for a smaller proportion in the brain, and the density of amyloid plaques also decreases (FIG. 9, panels A-E). These results indicated that ApoE CT expressed in neurons inhibits the deposition of amyloid plaques in the brains of 5×FAD mice and attenuates the pathological process of AD in mice.

### 3.9 ApoE2 expressed in neurons can inhibit the production of endogenous Aβ40 in mice

The above experiments have shown that ApoE CT is the key functional domain of ApoE2 to inhibit the activity of γ-secretase, and ApoE CT can inhibit the production of Aβ40 in mouse neurons. It was next explored whether ApoE2 also inhibits the production of endogenous Aβ40 in neurons (FIG. 10). GFP or ApoE2 was expressed in primary cultured neurons of mice using a lentivirus and a neuron-specific promoter, respectively. The N-terminus of ApoE2 is further linked to the signal peptide of human ApoE itself (SEQ ID NO: 66). ELISA results (FIG. 10, panel A) demonstrated that overexpressed ApoE2 in neurons can reduce endogenously produced Aβ40 compared to GFP controls. Further, ApoE2 was expressed in neurons in the brain of wild-type mice (WT mouse) using the AAV expression system. ELISA results (FIG. 10, panel B) showed that ApoE2 expressed in mouse brain neurons also reduce endogenous Aβ40 production compared to GFP controls. These results suggested that ApoE2, like ApoE CT, can inhibit the production of endogenous Aβ40 in neurons.

### 3.10 ApoE with tagged protein added at either end still has the activity of regulating APP cleavage

In order to explore whether the tag protein added on ApoE can destroy the activity of ApoE, the present description added tag protein V5 (SEQ ID NO: 82) to the C-terminus or N-terminus of ApoE2 and ApoE CT respectively, named as ApoE2-V5 (V5 at C-terminal), V5-ApoE2 (V5 atN-terminal), ApoE CT-V5 and V5-ApoE CT. The signal peptide of human ApoE itself (SEQ ID NO: 66) was further linked to the N-terminus of all constructed proteins. These expression plasmids were co-expressed with APP in HEK 293T cells. Western blot results showed (Figure 11), whether ApoE2-V5 and V5-ApoE2 (FIG. 11, panels A-B), or ApoE CT-V5 and V5-ApoE CT (FIG. 11, panels C-D) significantly increase α-CTF , and the activity is similar to ApoE2 or ApoE CT. This showed that ApoE2 and ApoE CT with added tag proteins at both ends still have the activity of regulating APP cleavage.

### 3.11 Mutants of ApoE CT can still regulate the cleavage of APP

In order to explore whether the mutation of ApoE CT will change the activity of ApoE CT in regulating APP cleavage, the present description constructed and analyzed a series of mutants of ApoE CT. Including single site mutations: CT A20, CT A40, CT A60, CT A65, CT A72, CT A80; double site mutations: CT A36/74; multiple site mutations: CT E5, CT E10, CT E20, CT F5 , CT R10, CT LF5, CT LM5. The position of the mutation site refers to the position of ApoE CT (SEQ ID NO: 33). The amino acid sequences of the above mutants are shown in SEQ ID NOs: 46-59. The signal peptide of human ApoE itself (SEQ ID NO: 66) was further linked to the N-terminus of all constructed proteins.
SEQ ID NO: 46 (CT A20; Q20A compared to SEQ ID NO: 33; Q235A compared to SEQ ID NO: 12): 
ARMEEMGSRTRDRLDEVKEAVAEVRAKLEEQAQQIRLQAEAFQARLKSWFE
PLVEDMQRQWAGLVEKVQAAVGTSAAPVPSDNH
SEQ ID NO: 47 (CT A20; E40A compared to SEQ ID NO: 34; E255A compared to SEQ ID NO: 12): 
ARMEEMGSRTRDRLDEVKEQVAEVRAKLEEQAQQIRLQAAAFQARLKSWFE
PLVEDMQRQWAGLVEKVQAAVGTSAAPVPSDNH
SEQ ID NO: 48 (CT A60; Q60A compared to SEQ ID NO: 33; Q275A compared to SEQ ID NO: 12): 
ARMEEMGSRTRDRLDEVKEQVAEVRAKLEEQAQQIRLQAEAFQARLKSWFE
PLVEDMQRAWAGLVEKVQAAVGTSAAPVPSDNH
SEQ ID NO: 49 (CT A65; V65A compared to SEQ ID NO: 34; V280A compared to SEQ ID NO: 12): 
ARMEEMGSRTRDRLDEVKEQVAEVRAKLEEQAQQIRLQAEAFQARLKSWFE
PLVEDMQRQWAGLAEKVQAAVGTSAAPVPSDNH
SEQ ID NO: 50 (CT A72; V72A compared to SEQ ID NO: 33; V287A compared to SEQ ID NO: 12): 
ARMEEMGSRTRDRLDEVKEQVAEVRAKLEEQAQQIRLQAEAFQARLKSWFE
PLVEDMQRQWAGLVEKVQAAAGTSAAPVPSDNH
SEQ ID NO: 51 (CT A80; P80A compared to SEQ ID NO: 33; P295A compared to SEQ ID NO: 12): 
ARMEEMGSRTRDRLDEVKEQVAEVRAKLEEQAQQIRLQAEAFQARLKSWFE
PLVEDMQRQWAGLVEKVQAAVGTSAAPVASDNH
SEQ ID NO: 52 (CT A36/74; R36A+T74A compared to SEQ ID NO: 33; R251A+T289A compared to SEQ ID NO: 12): 
ARMEEMGSRTRDRLDEVKEQVAEVRAKLEEQAQQIALQAEAFQARLKSWFE
PLVEDMQRQWAGLVEKVQAAVGASAAPVPSDNH
SEQ ID NO: 53 (CT E5): 
ARMEEMGSRARDRLAEVKEAVAEVAAKLEAQAQQARLQAAAFQAALKSW
AEPLVADMQRAWAGLAEKVQAAVGTAAAPVASDNH
SEQ ID NO: 54 (CT E10): 
ARMEEMGSRARDRLDEVKEAVAEVRAKLEAQAQQIRLQAAAFQARLKSWA
EPLVEDMQRAWAGLVEKVQAAVGTSAAPVASDNH
SEQ ID NO: 55 (CT E20): 
ARMEEMGSRTRDRLDEVKEAVAEVRAKLEEQAQQIRLQAAAFQARLKSWFE
PLVEDMQRAWAGLVEKVQAAVGTSAAPVASDNH
SEQ ID NO: 56 (CT F5): 
AAAAAMGSRTRDRLDEVKEQVAEVRAKLEEQAQQIRLQAEAFQARLKSWFE
PLVEDMQRQWAGLVEKVQAAVGTSAAPVPSDNH
SEQ ID NO: 57 (CT R10; S75A+P78A+V79A+P80A+S81A+D82A+N83A+H84A compared to SEQ ID NO: 33; S290A+P293A+V294A+P295A+S296A+D297A+N298A+H299A compared to SEQ ID NO: 12): 
ARMEEMGSRTRDRLDEVKEQVAEVRAKLEEQAQQIRLQAEAFQARLKSWFE
PLVEDMQRQWAGLVEKVQAAVGTAAAAAAAAAA
SEQ ID NO: 58 (CT LF5; R11A+D12A+R13A+L14A+D15A compared to SEQ ID NO: 33; R226A+D227A+R228A+L229A+D230A compared to SEQ ID NO: 12): 
ARMEEMGSRTAAAAAEVKEQVAEVRAKLEEQAQQIRLQAEAFQARLKSWFE
PLVEDMQRQWAGLVEKVQAAVGTSAAPVPSDNH
SEQ ID NO: 59 (CT LM5; E23A+V24A+R25A+K27A compared to SEQ ID NO: 33; E238A+V239A+R240A+K242A relative to SEQ ID NO: 12):

ARMEEMGSRTRDRLDEVKEQVAAAAALEEQAQQIRLQAEAFQARLKSWFE
PLVEDMQRQWAGLVEKVQAAVGTSAAPVPSDNH

Human APP was co-expressed with each of the above plasmids in HEK 293T cells, respectively, and GFP was used as a negative control group, and ApoE CT was used as a positive control group. The results of immunoblotting showed that the single point mutation and double point mutation of the constructed ApoE CT (FIG. 12, panels A-B), CT A20, CT A40, CT A60, CT A65, CT A72, CT A80 and CT A36/74 are all significantly increase α-CTF; and compared to ApoE CT, the degree of increasing α-CTF by these mutations do not decrease (the degree of increased α-CTF by CT A72, CT A80, and CT A36/74 are even higher). Multi-site mutations CT E5, CT E10, CT E20, and CT F5 can no longer increase α-CTF; however, CT R10, CT LF5, and CT LM5 still significantly increase α-CTF, and the levels of increased α-CTF by these mutations are similar to ApoE CT(FIG. 12, panels C-D). These data suggested that mutants of ApoE CT (such as CT A20, CT A40, CT A60, CT A65, CT A72, CT A80, CT A36/74, CT R10, CT LF5, and CT LM5) also possess similar activity to the original ApoE CT in regulating APP cleavage.

### 3.12 Addition or deletion of amino acid residues inside ApoE2 can still regulate APP metabolism

This experiment explored whether the addition or deletion of amino acid residues inside ApoE2 will affect the activity of ApoE. The present description first added a tag protein, 3XFLAG, between the amino acid residues of positions 215th and 216th of ApoE2 (SEQ ID NO: 12), and named it ApoE2-M3F (SEQ ID NO: 60; 3XFLAG sequence is bold; the underline indicates sequence of SEQ ID NO: 33), the modification does not affect the ApoE CT sequence (SEQ ID NO: 33). The present description also constructed ApoE2 MD10 (SEQ ID NO: 61). ApoE2 MD10 was obtained by deleting the amino acid residues at positions 244th to 254th of ApoE2, and these deleted amino acids were located in the C-terminus region of ApoE2 that regulates APP metabolism, that is, in the sequence as shown by SEQ ID NO:33. When expressed in cells, the N-terminus of all constructed proteins were further linked with the signal peptide of human ApoE itself (SEQ ID NO: 66).
SEQ ID NO: 60 (ApoE2-M3F)
KVEQAVETEPEPELRQQTEWQSGQRWELALGRFWDYLRWVQTLSEQVQEEL LSSQVTQELRALMDETMKELKAYKSELEEQLTPVAEETRARLSKELQAAQARLGA DMEDVCGRLVQYRGEVQAMLGQSTEELRVRLASHLRKLRKRLLRDADDLQKCL AVYQAGAREGAERGLSAIRERLGPLVEQGRVRAATVGSLAGQPLQERAQAWGER LR**GSGGSDYKDHDGDYKDHDIDYKDDDDKSGSGS**ARMEEMGSRTRDRLDEVK EQVAEVRAKLEEQAQQIRLQAEAFQARLKSWFEPLVEDMQRQWAGLVEKVQAA VGTSAAPVPSDNH (SEQ ID NO: 12; the underline indicates sequence of SEQ ID NO: 33; bold indicates 3XFLAG sequence, SEQ ID NO: 77). 
SEQ ID NO: 61 (ApoE2 MD10):
KVEQAVETEPEPELRQQTEWQSGQRWELALGRFWDYLRWVQTLSEQVQEEL
LSSQVTQELRALMDETMKELKAYKSELEEQLTPVAEETRARLSKELQAAQARLGA
DMEDVCGRLVQYRGEVQAMLGQSTEELRVRLASHLRKLRKRLLRDADDLQKCL
AVYQAGAREGAERGLSAIRERLGPLVEQGRVRAATVGSLAGQPLQERAQAWGER
LRARMEEMGSRTRDRLDEVKEQVAEVRAKLEAFQARLKSWFEPLVEDMQRQWA
GLVEKVQAAVGTSAAPVPSDNH
KVEQAVETEPEPELRQQTEWQSGQRWELALGRFWDYLRWVQTLSEQVQEEL LSSQVTQELRALMDETMKELKAYKSELEEQLTPVAEETRARLSKELQAAQARLGA DMEDVCGRLVQYRGEVQAMLGQSTEELRVRLASHLRKLRKRLLRDADDLQKCL AVYQAGAREGAERGLSAIRERLGPLVEQGRVRAATVGSLAGQPLQERAQAWGER LRARMEEMGSRTRDRLDEVKEQVAEVRAKLEEQAQQIRLQAEAFQARLKSWFEP LVEDMOROWAGLVEKVQAAVGTSAAPVPSDNH (SEQ ID NO: 12; the underline indicates sequence of SEQ ID NO: 33; the box indicates deleted sequences in ApoE2 MD10).
ARMEEMGSRTRDRLDEVKEQVAEVRAKLEAFQARLKSWFEPLVEDMQRQW AGLVEKVQAAVGTSAAPVPSDNH (SEQ ID NO: 76; ApoE CT was removed from the amino acid residues of positions 244-254 of ApoE2)
GSGGSDYKDHDGDYKDHDIDYKDDDDKSGSGS (SEQ ID NO:77; 3XFLAG)

Human APP was co-expressed with ApoE2-M3F and ApoE2 MD10 in HEK 293T cells, and GFP was used as a negative control group. The results of Western blot showed (FIG. 13), both ApoE2-M3F with amino acid residues added in the middle of ApoE2 (which does not affect the ApoE CT sequence), and ApoE2 MD10 with the deletion of amino acid residues in the middle of the C-terminal functional region significantly increases α-CTF. This result indicated that ApoE2 can still regulate APP metabolism by adding or deleting certain amino acid sequences inside ApoE2.

### 3.13 Non-human ApoE CT can also regulate the cleavage of APP

In order to further explore whether non-human ApoE also has the same activity, this experiment analyzed non-human primate-derived ApoE CT (monkey CT). This sequence is highly similar in amino acid sequence to human ApoE CT, with only three amino acid substitutions: R36S+T74A+A76T (FIG. 14, panel C). The monkey ApoE CT amino acid sequence is shown below.

SEQ ID NO: 62 (monkey CT):
ARMEEMGSRTRDRLDEVKEQVAEVRAKLEEQAQQISLQAEAFQARLKSWFE
PLVEDMQRQWAGLVEKVQAAVGASTAPVPSDNH

In HEK 293T cells, human APP and non-human primate ApoE CT (when expressed in cells, the N-terminals are further linked to the signal peptide of human ApoE itself (SEQ ID NO: 66)) were co-expressed, GFP as a negative control group, human ApoE CT (the signal peptide of human ApoE itself (SEQ ID NO: 66) was further linked to the N-terminal) was used as a positive control. The results of Western blot (FIG. 14, panels A and B) showed that non-human primate ApoE CT (monkey CT) and human ApoE CT also increase α-CTF. This result indicated that ApoE CT of non-human primates also has the activity of regulating APP cleavage.

### 3.14 ApoE with cell-penetrating peptide added has the activity of regulating APP cleavage

In order to explore whether the cell-penetrating peptide changed the activity of ApoE, the present description added the cell-penetrating peptide to ApoE. The selected cell-penetrating peptide sequence is as follows:
SEQ ID NO: 63 (R₉): RRRRRRRRR
SEQ ID NO: 64(FGF4): AAVLLPVLLAAP
SEQ ID NO: 65 (RD):
   KSVRTWNEIIPSKGCLRVGGRCHPHVNGGGRRRRRRRRR

The ApoE2 mutants constructed by adding R9, FGF4, and RDP to the N-terminus of human ApoE2 were named R9-ApoE2, FGF4-ApoE2, and RDP-ApoE2, respectively. The signal peptide of human ApoE itself (SEQ ID NO: 66) was further linked to the N-terminus of all protein constructs. R9-ApoE2, FGF4-ApoE2, RDP-ApoE2 were co-expressed with human APP in HEK 293T cells, respectively, and GFP was used as a negative control group. The results of Western blot (FIG. 15) showed that although FGF4-ApoE2 does not increase α-CTF, both R9-ApoE2 and RDP-ApoE2 significantly increase α-CTF. This result indicated that ApoE2 linked with cell-penetrating peptide can still regulate the cleavage of APP.

### 3.15 The signal peptide is necessary for ApoE CT to regulate the activity of APP cleavage.

The front part of the amino acid sequence of all the corresponding polypeptides expressed in the above experiments used to explore the function of ApoE expressed in cells comprises the signal peptide of human ApoE itself (ApoE SP; SEQ ID NO: 66), and the signal peptide is located at N-terminus of ApoE or ApoE CT and each mutant of ApoE or ApoE CT as described above. In order to explore whether the signal peptide can affect the activity of ApoE CT, the present description had replaced the signal peptide of ApoE CT with the signal peptide of apolipoprotein A1 and apolipoprotein J respectively (ApoA1 SP and ApoJ SP; SEQ ID NO:67 and SEQ ID NO: 68). ApoE CT activity without a signal peptide was also analyzed.
SEQ ID NO: 66 (ApoE SP): MKVLWAALLVTFLAGCQA
SEQ ID NO: 67 (ApoAI SP): MKAAVLTLAVLFLTGSQA
SEQ ID NO: 68 (ApoJ SP): MMKTLLLFVGLLLTWESGQVLG

The ApoE CT mutant replaced with apolipoprotein A1 signal peptide was named ASP CT; the ApoE CT mutant replaced with apolipoprotein J signal peptide was named JSP CT; the ApoE CT mutant without signal peptide was named NSP CT. Human APP was co-expressed with each of the mutants of ApoE CT mentioned above in HEK 293T cells, respectively, GFP was used as a negative control group, and ApoE CT was used as a positive control group. Western blot results (FIG. 16) showed that ASPCT and JSP CT can still increase α-CTF, but the degree of increasing α-CTF is not as good as that of ApoE CT with the signal peptide of ApoE itself; after deleting the signal peptide, NSP CT no longer increases α-CTF. This result indicated that ApoE CT expressed in cells needs a signal peptide to regulate the cleavage of APP by γ-secretase. This signal peptide could be a different signal peptide, not limited to the original signal peptide of ApoE.

### 4. Experimental results

The present description discovered for the first time that ApoE2 can regulate the cleavage of APP in a cell-autonomous manner in neurons, and this process depends on the C-terminal fragment of ApoE2. The C-terminal fragment of ApoE not only increases the non-amyloid metabolite α-CTF of APP; but also weakens the amyloidogenic pathway of APP and reduces Aβ. It was also found that ApoE2 and its C-terminal fragment regulate APP metabolism. It is achieved not through the secreted ApoE protein indirectly affecting the cleavage of APP in the cell; but through ApoE2 or its C-terminal fragment that co-exists with APP in the same cell to inhibit directly the γ-cleavage of APP, which is a cell-autonomous mode of action. In experiments using purified proteins, it was also found that ApoE2 can directly inhibit the cleavage of β-CTF by γ-secretase, while ApoEN NT lacking the C-terminus cannot inhibit the cleavage of β-CTF by γ-secretase. In addition, the C-terminal fragment of ApoE cannot inhibit the γ-cleavage of APLP1, indicating that the inhibitory effect of the C-terminal fragment of ApoE on γ-cleavage is substrate-selective. The present description also found that the C-terminal fragment of ApoE specifically expressed in neurons significantly reduces the size and density of amyloid plaques in the brains of AD model mice. ApoE specifically expressed in mouse neurons can inhibit the production of endogenous Aβ40. In addition, the addition of tagged proteins at the N or C terminal does not affect the activity of ApoE2 and ApoE C-terminal fragments to inhibit γ-cleavage of APP. The present description also constructed a series of mutants of ApoE C-terminal fragments and ApoE2 internal sequences, which have the activity of inhibiting γ-cleavage of APP similar to the original ApoE CT or ApoE2. The present description also found that the addition of some cell-penetrating peptides to the N-terminus of ApoE will not affect its γ-cleavage activity of inhibiting APP, but if the N-terminus lacks a signal peptide, it would lead to the loss of ApoE's activity of inhibiting γ-cleavage of APP. The discovery of the present description is not limited to the human ApoE C-terminal fragment, and the non-human primate ApoE C-terminal fragment also has similar activity of inhibiting γ-cleavage of APP.

These results indicated that ApoE2 and its C-terminal fragment (or mutants based on ApoE2 or C-terminal fragment) can be used as a potential medicament for the prevention or treatment of AD. Such potential medicaments would need to act in a cell-autonomous manner in neurons. Liposome delivery of DNA or RNA, lentivirus and adeno-associated virus infection of cells can make ApoE2 and/or its C-terminal fragment expressed in target cells; cell-penetrating peptides, enhanced endocytosis and other methods can also make ApoE2 and/or its C-terminal fragment exists in large quantities in the target cells; then ApoE2 and/or its C-terminal fragment inhibit the γ-secretase enzymatic digestion activity, reduce Aβ, and weaken the amyloidogenic pathway of APP in a cell-autonomous manner.

The above experiments only take AD as an example to illustrate that ApoE2 and/or its C-terminal fragments inhibit the γ-secretase enzymatic digestion activity in a cell-autonomous manner. In addition, conditions such as amyloid cerebrovascular diseases are also associated with abnormal in γ-secretase function. And Down syndrome is also prone to develop into AD. Therefore, ApoE2 and/or its C-terminal fragments also have application potential in the treatment and prevention of these APP or γ-secretase related diseases.

### Example 2. ApoE's precise inhibition of γ-secretase is the basis for its prevention of Alzheimer's disease

### 1. Summary

Abnormally low γ-secretase activity is associated with most presenilin mutations that cause familial Alzheimer's disease (familial AD, fAD). However, the role of γ-secretase in the more prevalent form of sporadic AD (sAD) remains unresolved. Human apolipoprotein E (ApoE) is the most important genetic risk factor for sAD, and the present paper reported that ApoE interacts with γ-secretase and inhibits the activity of γ-secretase with substrate-specificity in a cell-autonomous manner through conserved C-terminal region (CT) of the ApoE. This ApoE CT-mediated inhibitory activity is impaired to varying degrees in different ApoE subtypes, resulting in a rank order of ApoE2>ApoE3>ApoE4 potency that is inversely proportional to its associated AD risk. Interestingly, in a mouse model of AD, ApoE CT expressed in neurons can migrate from other regions to amyloid plaques and slow down the formation of amyloid plaques. Taken together, the data from this experiment revealed that ApoE has substrate-specific γ-secretase inhibitory activity and strongly suggested that such precise inhibition of γ-secretase by ApoE may protect against the risk of sAD.

### 2. Introduction

Alzheimer's disease (AD) is the most common neurodegenerative disorder, affecting nearly 50 million people worldwide (Prince, 2015). The β-amyloid (Aβ) hypothesis attributes AD to the excessive aggregation of Aβ, Aβ is a polypeptide, produced by the sequential cleavage of amyloid precursor protein (APP) by β-secretase and γ-secretase (De Strooper and Karran, 2016 2002; De Strooper et al., 1998; Haass and Selkoe, 1993; Hardy and Selkoe, 2002; Wolfe et al., 1999). Mutations in the enzymatic catalytic centers of γ-secretase-presenilin 1 (PS1) and presenilin 2 (PS2)-cause a rare form of familial AD (fAD). Based on these evidences, γ-secretase is considered as a drug target in AD. Unfortunately, treatment with conventional γ-secretase inhibitors causes severe side effects (Panza et al., 2019; Xia, 2019). Recent studies have found that presenilin mutations associated with fAD are often associated with reduced overall activity of γ-secretase (Shen and Kelleher, 2007; Sun et al., 2017; Walker et al., 2005; Xia et al., 2015 ), which contradicts the predicted role of γ-secretase based on the Aβ hypothesis. Given the dismal results of Aβ-based drug development efforts (Karran and De Strooper, 2016), there is an urgent need to unravel the role of γ-secretase in the more prevalent form of sporadic AD (sAD).

sAD has no disease-causing genes, but its risk is affected by a variety of genetic factors. Among them, apolipoprotein E4 (ApoE4) is the most influential factor (Corder et al., 1993; Rhinn et al., 2013). The ApoE gene encodes three isoforms with only a single amino acid difference in their N-terminal region (NT): ApoE2, 3, and 4 (Kanekiyo et al., 2014). ApoE4 not only increases the risk of AD, but also leads to an earlier age of onset and aggravates the severity of AD (Belloy et al., 2019; Li et al., 2020). In contrast, ApoE2 reduced AD risk. In the rodent brain, under normal physiological conditions, ApoE is usually detected only in glial cells (Kanekiyo et al., 2014), whereas Aβ is produced by neurons. However, ApoE is also present in human neurons (Aoki et al., 2003; Han et al., 1994; Metzger et al., 1996; Strittmatter et al., 1993; Xu et al., 1999) and in injured rodent neurons (Xu et al., 2006). The metabolism of ApoE in the human brain is more similar to that of ApoE expressed in neurons than in glial cells (Brecht et al., 2004). A recent study using neurons derived from human induced pluripotent stem cells (hiPSCs) carrying different ApoE isoforms demonstrated increased Aβ production in ApoE4-positive neurons, suggesting that ApoE in neurons can regulate metabolism of APP in a cell-autonomous manner (Wang et al., 2018). This evidence points to a crucial physiological role for ApoE in neurons, but this has never been fully studied.

This experiment reported that ApoE directly interacts with γ-secretase and specifically inhibits the γ-cleavage of APP through its C-terminal region. It is exciting that this inhibitory activity is impaired in different ApoE subtypes, which is negatively correlated with the risk of AD corresponding to the ApoE subtype. Furthermore, ApoE CT expressed in neurons accumulates around amyloid plaques, with the potential to achieve local y suppression within this amyloidogenic hotspot (Jordà-Siquier et al., 2022). Taken together, the data from this experiment demonstrated that precise inhibition of γ-secretase by ApoE protects against the risk of sAD and identify ApoE CT as a novel γ-secretase inhibitor with unprecedented specificity.

### 3. Experimental results

### 3.1. Endogenous ApoE and APP are co-expressed in human cortical neurons with overlapping subcellular distribution patterns

ApoE is mainly expressed by glial cells in the rodent brain, and Aβ is produced by the metabolism of APP expressed in neurons. Therefore, most studies have focused on how ApoE secreted by glia affects extracellular amyloid plaque formation (Huynh et al., 2017; Huynh et al., 2017; Liu et al., 2017). However, a recent study showed that ApoE can regulate Aβ production in a cell-autonomous manner (Wang et al., 2018). Before exploring this potential mechanism, this study first used immunofluorescence to stain human brain samples in order to verify that ApoE was indeed expressed in neurons. The experimental results are consistent with previously reported human brain data (Aoki et al., 2003; Belonwu et al., 2022; Han et al., 1994; Zalocusky et al., 2021), ApoE was detected both in human brain neurons and glial cells. Specifically, for young controls (young, 16-23 years; FIG. 17, panel A; FIG. 25, panel B; and Fig. 32 information on human brain donors), age-matched controls of non-AD patients (old, > 60 years) and AD patients (AD, >60 years; FIG. 25, panels A and B), 30-50% of cortical neurons were ApoE-positive; meanwhile, 40-62% of ApoE-positive cells were human cortical neurons in samples (FIG. 17, panel A; FIG. 25, panels A and C). In addition, human cortical astrocytes also express ApoE (FIG. 25, panel D). In monkey cortex, most detectable ApoE signals were localized in neurons (FIG. 17, panel A). A strong ApoE staining signal was detectable in amyloid plaques in the brains of human AD patients, but no ApoE signal was detectable in the same regions in control brain samples without AD (FIG. 25, panel E). Importantly, immunostaining revealed that endogenous ApoE and APP co-exist in human cortical neurons with overlapping subcellular signaling patterns (FIG. 17, panel B).

### 3.2. ApoE2 increases α-CTF and reduces Aβ in a cell-autonomous manner

In order to investigate whether ApoE can regulate the metabolism of APP in a cell-autonomous manner, APP and ApoE were co-expressed in mouse neuron cell line (N2A; FIG. 17, panel C) and HEK 293T cells (FIG. 26, panel C) in this experiment. α-CTF is an enzymatic cleavage product of the non-amyloid metabolic pathway of APP, whose production is known to primarily depend on ADAM10 (FIG. 26, panels A and B), an α-secretase (Esch et al., 1990; Haass et al. people, 1992; Mucke and Selkoe, 2012). In N2A and HEK293T cells, ApoE2 or ApoE3 significantly increased the α-CTF levels produced by co-expressing APP (FIG. 17, panels C and D, N2A cells; FIG. 26, panels C and D, HEK 293T cells). In addition, ApoE2 also significantly reduced Aβ40 levels produced by co-expressing APP (FIG. 17, panel E). Interestingly, co-expression of ApoE4 and APP failed to alter α-CTF levels (FIG. 17, panels C and D) and Aβ40 levels (FIG. 17, panel E). These results suggested that ApoE2 increases α-CTF in a cell-autonomous manner.

### 3.3. The C-terminal region of ApoE2 is more active than full-length ApoE2 in increasing α-CTF levels

ApoE comprises three regions: an N-terminal region (ApoE NT), a hinge region and a conserved C-terminal region (ApoE CT) (FIG. 33) (Chen et al., 2011). The only sequence differences between the three ApoE isoforms lie within their NTs. Experiments using HEK 293T cells with ApoE subtype truncated mutants showed that NT from ApoE2, 3 or 4 does not significantly alter the α-CTF produced by co-expressing APP (FIG. 26, panels E and F). In contrast, co-expression of the conserved ApoE CT resulted in a more than 6-fold increase in α-CTF levels (FIG. 26, panels G and H).

Strikingly, experiments showed that co-expression of ApoE CT together with APP resulted in the secretion of Aβ40 into the cell culture medium reduced by about 75% (FIG. 26, panel I), and Aβ40 in cell lysates has decreased by about 42% (FIG. 26, panel J), and also significantly reduced Aβ42 by about 74% (FIG. 26, panel K), but the ratio of Aβ42/Aβ40 remains unchanged (FIG. 26, panel K). However, ApoE CT co-expressed with APP and BACE1 did not alter β-CTF levels (FIG. 26, panels L and M). All ApoE NTs failed to significantly reduce Aβ40 (FIG. 26, panel I). Similarly, experiments using N2A cells showed that only co-expression of ApoE CT with APP, but not ApoE NT (FIG. 17, panels F and G), significantly increases α-CTF; in addition, total Aβ40 was also significantly reduced by ApoE CT (FIG. 17, panel H).

Experiments found that compared with ApoE2, ApoE CT also significantly increases the level of α-CTF (FIG. 17, panels I and J, N2A cells; FIG. 26, panels N and O, HEK 293T cells), which indicated that the activity of ApoE CT in regulating APP metabolism is clearly higher than that of full-length ApoE2. These results suggested that ApoE CT, a conserved region of ApoE, can increase α-CTF levels, and suggested that the NT of ApoE may somehow impede this activity of ApoE CT.

In order to investigate whether secreted ApoE also affects APP cleavage, HEK 293T cells expressing APP alone were cultured in culture medium comprising secreted ApoE or ApoE CT (FIG. 27). Experiments showed that free ApoE2 or ApoE CT in culture medium does not alter the α-CTF produced by APP in cells (FIG. 27, panels A, β and C). It is worth noting that ApoE2 can exist stably in the conditioned medium at 37°C for 48 hours (FIG. 27, panel D), which can exclude the possible interfering factors caused by the degradation of ApoE. For HEK 293T cells and primary cultured rat neurons treated with conditioned medium, ApoE2 or ApoE CT was mainly detected outside the cell membrane (FIG. 27, panels E and F). In contrast, ApoE CT expressed by cells or neurons is distributed within cells or neurons (FIG. 27, panels E and F). Comparing the relative amount of ApoE2 in the cell lysate and ApoE2 secreted in the culture medium, both contained similar amounts of ApoE2 (FIG. 27, panel G). Furthermore, small amounts of ApoE from conditioned medium could be endocytosed by cells or neurons after co-incubation (FIG. 27, panels E and F). Therefore, ApoE regulation of APP metabolism is more likely to be mediated by intracellular ApoE in a cell-autonomous manner rather than by secreted ApoE.

### 3.4. ApoE CT expressed in neurons reduces endogenous Aβ.

In addition to human APP, ApoE CT also increases α-CTF levels in HEK 293T cells transiently expressing mouse APP (mAPP) (FIG. 26, panels P and Q). Experimental results in primary cultured neurons showed a 30% reduction in endogenous Aβ40 after overexpression of ApoE CT (lentiviral infection) in mouse cortical and hippocampal neurons compared to GFP controls (FIG. 17, panel K ). In hiPSC-induced neurons, ApoE CT (lentiviral infection) resulted in an approximately 46% reduction in Aβ40 production by human neurons (FIG. 17, panel L). Collectively, these results suggested that ApoE CT can slow endogenous Aβ in rodent and human neurons. In addition, expression of ApoE CT also slows production of Aβ40 in HEK 293T cells expressing a pathogenic Swedish APP mutant (APPsw) (Citron et al., 1992): Aβ₄₀ in the medium was reduced by approximately 76% (FIG. 26, Panel R), Aβ₄₀ in cell lysates was reduced by approximately 43% (FIG. 26, panel S), indicating that ApoE CT reduces production of Aβ by APPsw.

Another apolipoprotein co-expressed with APP, apolipoprotein A1 (ApoA1), failed to alter the levels of α-CTF and Aβ (FIG. 26, panels T, U and V), suggesting that ApoE regulating the activity of APP cleavage is not a non-specific activity of any apolipoprotein.

### 3.5. ApoE2 interacts with APP and γ-secretase through CT.

In N2A cells, ApoE2 or ApoE CT has a perinuclear subcellular distribution pattern very similar to co-expressed APP-FLAG (FIG. 18, panel A). In cultured rat hippocampal neurons (FIG. 18, panel B) and hiPSC-derived neurons (FIG. 18, panel C), the expression of ApoE2-V5 or ApoE CT-V5 has a subcellular distribution pattern similar to the endogenously expressed APP in neurons (V5 is tagged protein). These results suggested that ApoE and ApoE CT have a subcellular distribution pattern similar to APP.

To detect potential interactions between ApoE and APP or APP secretase, this experiment used the protein proximity labeling technique (Hung et al., 2016). When co-expressed in HEK 293T cells, ApoE2 and ApoE CT were biotinylated by APP-APEX2 (ascorbate peroxidase 2, linked to the C-terminus of APP; FIG. 18, panels D and E). Meanwhile, FL-APP (full-length APP) and α-CTF could also be biotinylated by ApoE2-APEX2 in the same system (FIG. 18, panel F). In primary cultured rat neurons, ApoE2-APEX2 biotinylates endogenously expressed APP (FIG. 18, panel G). Furthermore, ApoE2-APEX2 biotinylated the co-expressed ADAM10 (α-secretase), PS1 and PS2 (catalytic activity of γ-secretase), but not the β-secretase BACE1 (FIG. 18, panel H). Furthermore, ApoE2 and ApoE CT, but not ApoE2 NT, were able to co-immunoprecipitate with β-CTF-FLAG when co-expressed in HEK293T cells (FIG. 18, panel I). Similarly, ApoE2 and ApoE CT (but not ApoE2 NT) were co-immunoprecipitated with components of γ-secretase (APH, NCT and PEN2) when co-expressed in HEK 293T cells (Figure 18, panel J; using anti-FLAG- PEN2 for immunoprecipitation). Interestingly, in human brain samples, endogenous γ-secretases (NCT, PS1, PS2 and PEN2) co-immunoprecipitated with endogenous ApoE (FIG. 18, panel K). The above results indicated that only ApoE2 and ApoE CT (not ApoE NT) can interact with APP, γ-secretase and α-secretase, but they do not interact with BACE1, and there is endogenous ApoE and γ-secretase in the human brain interaction.

### 3.6. ApoE CT reduces γ-cleavage of APP.

To further investigate the underlying mechanism, this study further explored the interaction between AD-related APP mutants and ApoE CT. ApoE CT increased α-CTF produced by all tested APP mutants (FIG. 28). Interestingly, in cells expressing APP variants with fAD mutations near the y cleavage site (T714I, V717F, and L723P) compared to wild-type APP (WT APP) (Kumar-Singh et al., 2000; Kwok et al., 2000; Murrell et al., 1991), the ability of ApoE CT to increase α-CTF levels is significantly reduced (FIG. 28). For WT APP and APP isoforms with fAD mutations near the β- or α-cleavage site (KM670/671NL, E693K, E693A) (Bugiani et al., 2010; Citron et al., 1992; Tomiyama et al., 2008 Xia et al., 2021), no statistically significant difference in the effect of ApoE CT on α-CTF levels was detected (FIG. 28). For co-expression of the APP variant having the protective Icelandic mutation (A673T) that can reduce the incidence of AD (Xia et al., 2021), no difference in the effect of ApoE CT on α-CTF levels was detected (FIG. 28). Taken together, when mutations occur near the γ-site of APP, the activity of ApoE CT is destroyed, which suggested that γ-cleavage is related to the activity of ApoE CT in regulating APP metabolism. Since α-CTF is degraded by γ-cleavage (De Strooper et al., 2012; De Strooper et al., 1998; Haass et al., 1992), these results suggested that ApoE CT may affect α-CTF degradation.

Next, this study conducted an in-depth exploration based on the role of γ-secretase in the metabolism of APP. Consistent with previous reports of α-CTF cleavage by γ-cleavage (De Strooper et al., 2012; De Strooper et al., 1998; Haass et al., 1992), overexpression of PS1 significantly reduces the level of α-CTF to 50% (FIG. 19, panels A and B); overexpressed PS1 no longer decreases α-CTF when treated with γ-secretase inhibitor PF03084014 (1 µM), PF03084014 also increases α-CTF; when treated with γ-secretase inhibitor, the level of α-CTF is similar to that in HEK 293T cells co-expressing ApoE CT (FIG. 19 , panels A, B, C and D). Interestingly, ApoE CT failed to further increase α-CTF in the presence of PF03084014 (FIG. 19, panels C and D), suggesting that ApoE CT activity can be blocked by inhibiting γ-secretase. Furthermore, the use of the ADAM10 inhibitor GI254023X (1 µM) can reduce α-CTF to about 45% (FIG. 29, panels A and B). In the presence of GI254023X, ApoE CT can still increase α-CTF (approximately 4-fold; FIG. 29, panels A and B) and decrease Aβ40 (FIG. 29, panels C and D). Furthermore, when β-secretase was inhibited by its inhibitor AZD3839 (5 µM), ApoE CT still increases α-CTF (FIG. 29, panels E and F). ApoE CT is also able to increase α-CTF produced by APP_{M596V} (FIG. 29, panels G and H), an APP mutation that cannot conduct β-cleavage (Citron et al., 1995). In addition, ApoE CT also significantly decreases Aβ₄₀ (FIG. 19, panel E) and Aβ₄₂ (FIG. 19, panel F) produced by β-CTF, indicating that ApoE CT reduces Aβ production by inhibiting γ-cleavage. Only β-cleavage is needed for the γ-cleavage product of APP, APP_{ΔAICD}, to generate Aβ₄₀. Interestingly, ApoE CT failed to reduce Aβ₄₀ produced by APP_{ΔAICD} (FIG. 19, panels G and H). Together, these results supported the hypothesis that ApoE CT inhibits γ-cleavage of APP, but does not directly regulate α- or β-cleavage.

Furthermore, neither ApoE2 nor ApoE CT alter the mRNA or protein expression levels of ADAM10, BACE1, PS1 or PS2 (FIG. 29, panels I and J), indicating that the expression levels of APP secretase are not altered. Furthermore, ApoE CT did not alter APP expression on the cell surface (FIG. 29, panel K).

### 3.7 ApoE CT regulates the metabolism of APP and requires the participation of γ-secretase.

To determine whether γ-secretase is required for ApoE CT regulation of APP metabolism, PS1 and/or PS2 knockout (KO) HEK 293T cell lines (Lu et al., 2014) were constructed in this study (FIG. 20, panel A). α-CTF levels were elevated in PS1 KO cells, which could be reversed by compensatory expression of PS1 (FIG. 20, panel B). PF03084014 treatment further increased α-CTF levels in PS1 KO cells (FIG. 20, panel B). Co-expressed ApoE CT was still able to increase α-CTF in PS1 KO cells (FIG. 20, panels C and D) and PS2 KO cells (FIG. 20, panels E and F). Therefore, knockdown of PS1 or PS2 alone is not sufficient to completely abolish the activity of ApoE CT.

In PS1/2 double knockout cells (PS1/2 dKO), α-CTF levels were also significantly increased compared with WT cells (FIG. 20, panel G); ApoE CT co-expressed in PS 1/2 dKO cells (FIG. 20, panels G and J) or ApoE2 (FIG. 20, panels K and L) did not further increase α-CTF levels. Complementary expression of PS1 or PS2 rescued the activity of ApoE CT, which again significantly increased α-CTF (FIG. 20, panels H, I and J). Meanwhile, ApoE CT also significantly reduced Aβ₄₀ produced by β-CTF in PS1/2 dKO cells supplemented with PS2 (FIG. 20, panel M). These results collectively indicated that either ApoE CT or ApoE2 requires γ-secretase to increase α-CTF.

### 3.8. ApoE endogenously expressed in mouse neurons regulates γ-cleavage of endogenous APP.

In order to explore the physiological activity of endogenous ApoE in neurons, shRNA of mouse ApoE was constructed (shApoE_1, shApoE_2, shScramble were used as controls). ShApoE_1 and shApoE_2 expressed in mouse neurons using the lentivirus successfully reduced the protein expression of endogenous ApoE in primary cultured mouse neurons (FIG. 21, panel A). Treatment with the γ-secretase inhibitor PF03084014 significantly increased the level of the ~14kD CTF (C-terminal fragment, CTF_{14kD}) produced by APP metabolism in neurons, suggesting that this CTF_{14kD} can also be cleaved by γ-secretase (FIG. 21, panel B). ShApoE_1 or shApoE_2 significantly reduced the basal level of CTF_{14kD} band in mouse neurons (FIG. 21, panels C and D), indicating that endogenous ApoE increases CTF_{14kD}. In the presence of the γ-secretase inhibitor PF03084014, these two shRNAs of ApoE could no longer reduce CTF_{14kD} (FIG. 21, panel E), indicating that this activity of ApoE requires the presence of active γ-secretase. In addition, knockdown of ApoE also resulted in increased levels of Aβ produced by neurons (FIG. 21, panel F). These results collectively indicated that endogenous ApoE in neurons regulates the γ-cleavage of endogenous APP.

### 3.9. ApoE CT inhibits γ-cleavage in a substrate-specific manner.

The present description goes on to test whether ApoE CT also inhibits the γ-cleavage of NOTCH. NOTCH is a substrate of γ-secretase (Xia, 2019; Yang et al., 2019). In HEK 293T cells, co-expressed γ-secretase reduced N100 (NOTCH fragment), and treatment with PF03084014 reversed this reduction (FIG. 30, panel A). Interestingly, co-expressed ApoE CT failed to suppress this γ-secretase-dependent reduction of N100 (FIG. 30, panel A). However, when co-expressed with γ-secretase, ApoE CT still inhibited γ-cleavage of APP by γ-secretase (FIG. 30, panel B). APP-like protein 1 (APLP1) is another substrate of α and y secretases (Eggert et al., 2004). Treatment with PF03084014 decreased APLP1 levels while enhancing its cleavage product (α-like CTF); however, α-like CTF was not altered by co-expression of ApoE CT with APLP1 (FIG. 30, panel C). These results demonstrated that ApoE CT does not inhibit the γ-cleavage of NOTCH and APLP1.

### 3.10. ApoE2 directly inhibits the γ-cleavage of APP.

To determine whether ApoE directly inhibits γ-cleavage of β-CTF, the present description purified β-CTF, ApoE2, ApoE2 NT, and γ-secretase (Lu et al., 2014) (FIG. 22, panel A; FIG. 31) for test further. Incubation of purified γ-secretase with β-CTF resulted in the production of Aβ₄₀; in the absence of γ-secretase, β-CTF did not produce Aβ₄₀; and incubation of γ-secretase with β-CTF produced Aβ₄₀, which could be complete blocked by PF03084014 (FIG. 22, panel B). Purified ApoE2 inhibited Aβ₄₀ production in a dose-dependent manner with an IC50 of 1.2 µM (FIG. 22, panel C). In contrast, ApoE2 NT failed to inhibit Aβ40 production (FIG. 22, panel D). These results suggested that ApoE2 directly inhibits the γ-cleavage of β-CTF through its CT.

### 3.11. Neuronally expressed ApoE CT migrates to amyloid plaques and reduces plaque burden in 5×FAD mice.

In order to investigate whether ApoE CT can delay the generation of amyloid plaques in the AD mouse model, ApoE CT was expressed in neurons in the cortex and hippocampus of 4-week-old 5×FAD mice through AAV vector (FIG. 23, panel A). After 10 weeks, compared with the GFP control, the average plaque size, percentage of total amyloid plaque area and plaque density in the subiculum brain region of ApoE CT-expressing 5×FAD mice were significantly reduced (FIG. 23, panels B, C, D and E).

Interestingly, in these 5×FAD mice, although ApoE CT expression was restricted to neurons in the cortex, dentate gyrus (DG) and CA3 regions of the hippocampus, it migrated to the subiculum and accumulated in amyloid plaques around the block (FIG. 23, panel B); whereas neurons expressing GFP failed to do so. As shown by the present description (FIG. 25, panel E) and data reported by others (Metzger et al., 1996), ApoECT expressed in neurons can migrate to amyloid plaques to achieve locally regulating γ-secretase among these amyloidogenic hotspots (Jordà-Siquier et al., 2022). When ApoE2 and ApoE CT were expressed in 5×FAD mice with lentiviral vectors, a similar distribution pattern was detected around amyloid plaques (Figure 24, panel A, arrows, ApoE in amyloid plaques; sessile arrow, ApoE in neurons); whereas GFP and ApoE2 NT failed to accumulate around plaques (FIG. 24, panel A). In 1-month-old 5×FAD mice (FIG. 24, panel B) and WT mice (FIG. 24, panel C) that did not develop amyloid plaques, ApoE CT was only present in neuronal somata and partially co-localized with the subcellular distribution of the expressed human APP (hAPP). These results suggested that CT is required for ApoE accumulation around amyloid plaques.

### 4. Discussion

Efforts to target Aβ to treat AD have continued to fail, and the role of Aβ in AD is questioned (Liu et al., 2019; Panza et al., 2019; Roberson and Mucke, 2006). Inevitably, this failure also challenges many fundamental understandings of AD itself. For example, although it remains unquestionable that dysfunction of γ-secretase causes fAD, this links Aβ dysregulation to fAD with strong human genetic evidence (Selkoe, 2001). However, the evidence supporting a role for Aβ in sAD is much weaker. The possibility that sAD is a distinct disease that happens to share the same pathological features as fAD cannot be completely ruled out (Armstrong, 2013; De Strooper and Karran, 2016). In fact, fAD and sAD also differ in other aspects besides underlying genetic factors, including age of onset, detailed pathological abnormalities and symptoms, etc. (Dorszewska et al., 2016; Roher et al., 2016).

ApoE is the most important genetic factor of sAD, and the data from this experiment show for the first time that ApoE appears to reduce the risk of sAD by selectively inhibiting γ-cleavage of APP, a key step in amyloidogenesis. These results of the present description explain why ApoE4 carriers produce a higher amyloid load and have fewer metabolites from non-amyloidogenic pathways than ApoE2 carriers (Corder et al., 1993; Hashimoto et al., 2012; Olsson et al., 2003; Schmechel et al., 1993). Taken together, the data from this experiment support that precise inhibition of γ-secretase can prevent the risk of sAD, and therefore it is reasonable to treat sAD by inhibiting γ-secretase. Notably, recent studies have found that presenilin mutations in most fADs lead to decreased γ-secretase activity (Sun et al., 2017; Xia et al., 2015). This suggests that although γ-secretase dysfunction plays a central role in the pathogenesis of sAD and fAD, changes in γ-secretase activity are opposite in these two different forms of AD. From this perspective, γ-secretase inhibition may not be applicable to fAD with presenilin loss-of-function mutations. Interesting questions are: why both higher and lower γ-secretase activity lead to AD, and whether fAD should be divided into different groups based on how presenilin mutations affect γ-secretase activity.

Also, considering the broad-spectrum γ-substrates that have not been tested (Güner and Lichtenthaler, 2020), APP may not be the only substrate whose cleavage is inhibited by ApoE, so the effect of ApoE on γ-cleavage of APP may not be the only reason for the sAD risk, but still plausible. Therefore, inhibition of γ-secretase may not result in the same effect as Aβ clearance. This is of particular concern as it may help explain why Aβ clearance strategies alone do not yield satisfactory clinical results. By analyzing which substrates of γ-secretase are affected by ApoE, the study of the present description will provide a unique opportunity to investigate the role of substrates of γ-secretase other than Aβ in the etiology of AD.

In mouse models, ApoE is mainly expressed in glial cells (Xu et al., 2006). Since Aβ is produced in neurons, most studies have focused on how ApoE secreted by glial cells affects extracellular amyloid progression (Holtzman et al., 1999; Huang et al., 2017; Huynh et al., 2017; Liu et al., 2017; Yu et al., 2014). Indeed, many papers from different laboratories have independently shown that both the mRNA and protein of ApoE are detected in human neurons (Aoki et al., 2003; Han et al., 1994; Metzger et al., 1996; Strittmatter et al., 1993; Xu et al., 1999). Recent single-nucleus RNA-seq data have also shown that ApoE can be detected in neurons, with up to 28% of AD patient neurons expressing ApoE at high levels (Belonwu et al., 2022; Zalocusky et al., 2021), which is consistent with our observation, approximately 30-40% of neurons express ApoE in the human cortex. Furthermore, the metabolism of ApoE in the human brain is more similar to that of ApoE expressed in neurons than in glial cells (Brecht et al., 2004). It has also been reported that human neurons carrying the ApoE4 subtype produce more Aβ than those with other ApoE subtypes (Wang et al., 2018), strongly supporting that endogenously expressed ApoE in human neurons can regulate metabolism of endogenous APP. However, the role of ApoE in neurons remains largely poorly understood. The present description found that the protective subtype ApoE2 directly inhibits γ-cleavage of APP in a cell-autonomous manner through its CT, while ApoE4 has no such activity. Importantly, the present description also found that endogenous ApoE in mouse neurons significantly inhibited the γ-cleavage of endogenously expressed APP; and in the human brain, there was an interaction between endogenous ApoE and endogenously expressed γ-secretase. Furthermore, AD mouse models recapitulate many of the key pathological features of AD but not the amyloid deposition of ApoE (Oakley et al., 2006). Surprisingly, the present inventors found that ApoE expressed in neurons of 5×FAD mice migrated and accumulated around amyloid plaques, which is consistent with previous reports that ApoE is present in amyloid plaques in the brains of AD patients (Han et al., 1994; Strittmatter et al, 1993), suggesting that ApoE in amyloid plaques has a neuronal origin. These findings emphasize the crucial role of ApoE in neurons in AD and provide a new mechanistic explanation for the differential contributions of ApoE2 and ApoE4 to AD pathogenesis.

γ-secretase is a potential drug target in AD. However, inhibition of γ-secretase activity was found to lead to severe side effects in previous drug development, which may be due to the poor selectivity of γ-secretase inhibitors among different substrates (Panza et al., 2019; Roberson and Mucke, 2006; Xia, 2019). Broad, non-specific inhibitors, while inhibiting γ-secretase to block Aβ production, also prevent the normal processing of γ-secretase's other substrates (Panza et al., 2019; Xia, 2019), leading to side effects. Developing γ-secretase modifiers to selectively inhibit γ-cleavage of APP is a promising new strategy (Xia, 2019). The present description found that ApoE CT inhibits the γ-cleavage of APP, but does not affect the γ-cleavage of NOTCH and APLP1, indicating that the inhibitory effect of ApoE CT on γ-cleavage has substrate specificity. As previously reported, small-molecule drugs inhibit the activity of γ-secretase by occupying the same position on the β-chain on PS1 that interacts with its substrates, such as APP or Notch (Yang et al., 2021). ApoE CT, as a molecule structurally distinct from traditional small molecule inhibitors, may act on some allosteric sites, affecting only the binding of some substrates to γ-secretase, but not other substrates, thereby achieving substrate selectivity. However, the exact mechanism remains to be resolved. Nevertheless, ApoE CT may serve as a new strategic treatment approach with potentially fewer side effects compared with conventional γ-secretase inhibitors. In summary, the present description not only provides a potential mechanistic understanding of the role of neuronal ApoE isoforms in the pathogenesis of sAD; but also identifies the ApoE CT as a potential treatment for AD by selectively inhibiting γ-cleavage of AD related γ-secretase substrates.

These results supported that precise inhibition of γ-secretase protects against the risk of sAD, in contrast to fAD, which is usually associated with decreased γ-secretase activity. The present description also identifies ApoE CT as a effective γ-secretase inhibitor with rare substrate specificity that can act locally by migrating from distant sites to amyloidogenic hotspots around plaques.

### 5. Experimental materials and methods

### Animals

Human APP and PS 1 with five fAD mutations are expressed in neurons of 5×FAD mice: K670N/M671L+I716V+V717I mutations in APP and M146L+L286V mutations in PS1 (Oakley et al., 2006). All mice were housed under a 12 h light-dark cycle (light from 8 am to 8 pm) with ad libitum access to food and water. All mouse procedures were performed in accordance with the Guide for the Care and Use of Laboratory Animals of the Chinese Academy of Sciences and the National Institutes of Health.

### Human and monkey brain tissues

All human brain tissues were obtained from the China Brain Bank (CBBC) in Hubei and Zhejiang University School of Medicine, and the Zhejiang National Health And Disease Human Brain Center. FIG. 32 summarizes the donor information. Human brain tissue was stored at -80°C before the experiment, fixed overnight in 4% PFA, and frozen overnight in 30% sucrose before sectioning. The following steps are the same as for IHC performed on mouse brain slices.

Fixed brain slices from adult rhesus monkeys were obtained from the Center for Excellence in Brain Science and Intelligence Technology (CEBSIT), Chinese Academy of Sciences, Shanghai, China (Wu et al., 2021).

### Culture of primary neurons

Primary isolated neuronal cultures were prepared as previously described (Chen et al., 2014). Whole hippocampus or cortex mice (C57BL/6) or rats (SD) were removed from day 16 wild-type embryos and treated with papain (Worthington) for 10 min at 37°C and dissociated. Isolated cells were seeded in B27 neuron growth medium at 150,000 cells/well (24-well plate). Neurons were cultured in an incubator at 37°C and 5% CO₂, and fresh medium was added once a week.

### Construction of PS1KO, PS2KO and PS1/2dKO cell lines

HEK 293T cells were cultured in DMEM medium (Gibco) supplemented with 10% bovine serum albumin (Life Technology) and 1% penicillin/streptomycin (Life Technology). To generate PS1, PS2 single knockout or PS1/2 double knockout HEK 293T cell lines, the CRISPR/Cas9 genome editing method was employed (Ran et al., 2013). Guide RNA (gRNA) targeting PS1 exon 4: 5'-ATTATCTAATGGACGACCCC-3' (SEQ ID NO: 91) and gRNA targeting PS2 exon 4: 5'-CAAATACGGAGCGAAGCACG-3' (SEQ ID NO: 92) subcloned into pSpCas9(BB)-2A-puro backbone (PS1gRNA, PS2gRNA) respectively. The plasmid comprising the gRNA was transfected into HEK 293T cells using Lipofectamine2000 (Invitrogen Technology). Eighteen hours after transfection of PS1 gRNA or PS2 gRNA, HEK 293T cells were treated with 3 µg/ml puromycin (Selleck). After the HEK 293T cells transfected with the blank plasmid were completely dead, the HEK 293T cells transfected with PS1 gRNA or PS2 gRNA were cultured in a 10 cm culture dish at a dilution ratio of 1:400 to ensure that each cell was monoclonal. After individual cells grow into cell clusters, genome-edited cell clusters were selected and verified by DNA sequencing and Western blot. For the construction of PS1/2dKO cell line PS2 knockout cells were transfected with PS1 gRNA and subjected to a second round of selection.

### Western blot (WB)

Plasmids were transfected in HEK 293T or N2A cells for 36-48 hours and then lysed in cell lysis buffer [10% 1M tris-HCl (pH=6.8), 4% SDS, 20% glycerol, 0.01% bromophenol blue]. Lysates were shaken at room temperature for 10 min, then boiled for 10 min, and centrifuged at 12,000 × g for 10 min. Proteins were run on 8% or 12% Tris-glycine SDS-PAGE and transferred to PVDF membranes (Life Technologies). Samples were incubated overnight at 4°C with primary antibody diluted in 5% BSA buffer (in 1×TBST). After washing 3 times with 1×TBST, they were incubated with secondary antibody diluted in 5% BSA buffer (in 1×TBST) for 2 hours at room temperature. Finally, after washing 3 times with 1×TBST, the signal was detected by ECL Western blotting substrate (Solarbio, PE0010).

Brain tissue samples were lysed with TissueLyserII (QIAGEN, 85300) in RIPA buffer (Solarbio, R0010). The lysate was boiled for 10 minutes and then centrifuged at 12,000 × g for 10 minutes. The operation steps of Western blot are the same as those of cell samples.

### Preparation of APEX-tagged proteins for Western blot

A method for detecting protein interactions in living cells by targeting APEX2 was applied as previously described (Hung et al., 2016). Approximately 30 hours after transfection with APEX2 plasmids via Lipofectamine2000 (Invitrogen Technology), HEK 293T cells were incubated in 1 ml of complete medium with or without 250 uM biotinphenol (BP) for 30 minutes at 37°C. Cells were treated with 1 mM H₂O₂ for 1 min and cells were washed 3 times in quencher solution (10 mM sodium ascorbate, 5 mM Trolox and 10 mM sodium azide solution in 1X DPBS). Cells were lysed in RIPA lysis buffer (1X protein inhibitor cocktail, 1 mM PMSF and 10 mM sodium ascorbate, 5 mM Trolox and 10 mM sodium azide). The lysate was sonicated and centrifuged on ice (12,000 x g, 4°C, 10 min), and the supernatant was incubated with streptavidin magnetic beads overnight at 4°C. The beads were collected using a magnetic rack and washed with a series of buffers (twice with RIPA buffer, once with 1M KCl, once with 0.1M Na₂CO₃, twice with 2 M urea in 10 mM Tris-HCl, pH 8.0, and twice with RIPA lysis buffer. Biotinylated proteins were eluted from the beads by boiling each sample in loading buffer comprising 2 mM biotin and 20 mM DTT for 10 min, and the eluates were collected. Samples proceed to Western blot.

### Co-immunoprecipitation (co-IP)

HEK 293T cells transfected and plasmids were treated with Lipofectamine2000 (Invitrogen Technology) for 48 hours, HEK 293T cells were lysed in 1% CHAPSO (SIGMA), 25 mM HEPES (pH7.4) (Gibco), 150 mMNaCl (comprising 1× protease inhibitor cocktail), centrifuged at 14,000×g for 20 min, and the supernatant were collected. The supernatant was incubated with anti-FLAG (SIGMA) or anti-IgG (Santacruz) antibody at 4°C for 3 hours, and pulled down using ProteinG Sepharose beads (Gen Script), and then washed in 0.1% digitonin-HEPES buffer for 3 hours, 15 minutes each time, and collected in cell lysis buffer [10% 1 M tris-HCl (pH=6.8), 4% SDS, 20% glycerol, 0.01% bromophenol blue]. Samples proceed to Western blot.

### Immunofluorescence staining and immunohistochemistry (IHC)

Neurons and HEK 293T cells cultured on coverslips in 24-well plates were washed with 1×PBS (2.5 mM MgCl₂ and 0.5 mM CaCl₂) and fixed with 4% PFA+4% sucrose in 1×PBS for 10 min at room temperature. Next, the wells were punched with 0.15% Triton-X in 1×PBS for 15 minutes, and blocked with 5% BSA in 1×PBS for 30 minutes at room temperature, and the samples were incubated with the primary antibody diluted in 5% BSA buffer (in 1×PBS) overnight at 4°C. Samples were then washed once with 0.5 M NaCl in 1×PBS and three times with 1×PBS. Samples were then incubated with secondary antibody diluted in 5% BSA (in 1×PBS) buffer for 1 h at room temperature. After three final wash steps with 1X PBS, samples were mounted with Prolong Gold Antifade Mountant (Life Technology). All images were taken and analyzed blinded.

Mice were anesthetized with isoflurane and perfused with 4% PFA. Dissected mouse brains were fixed overnight in 4% PFA and cryoprotected overnight in 30% sucrose. A cryostat (Leica CM3050S) was used to obtain frozen brain slices (40 µM) from fixed and cryoprotected mouse brains. Floating slices were washed 3 times with 1×PBS and then permeabilized with 0.5% Triton-X in 1×PBS for 30 minutes. After blocking with 5% BSA in 1×PBS at room temperature for 1 h, the slices were incubated overnight at 4° C in 5% BSA (in 1×PBS) buffer comprising the primary antibody. Slices were washed 3 times with 1×PBS and incubated with secondary antibody diluted in 5% BSA (1×PBS) buffer for 1 hour at room temperature. Samples were then washed 3 times with 1×PBS and mounted with Prolong Gold Antifade Mountant (Life Technology).

### THS staining

Mice were anesthetized with isoflurane and perfused with 4% PFA. Dissected mouse brains were fixed overnight in 4% PFA and cryoprotected overnight in 30% sucrose. A cryostat (Leica, CM3050S) was used to obtain frozen brain slices (40 µM) from fixed and cryoprotected mouse brains. Floating slices were washed 3 times with ddH₂O and then incubated with 1% THS (SIGMA) in water for 5 minutes at room temperature. Next, the slices were washed twice with 80% ethanol and once with 95% ethanol for 3 minutes each. The samples were washed with ddH₂O and 3 times with 1×PBS before IHC.

### Quantitative real-time PCR (qRT-PCR)

Total RNA was extracted from HEK 293T cells using TRIzol reagent (Invitrogen). Reverse transcription was performed using the QuantiTect SYBR-Green RT-PCR (Reverse Transcription-PCR) Kit (Qiagen). GAPDH was used as an internal control. ADAM10 mRNA forward primer: 5'-AGAGCAACATCTGGGGACAAA-3' (SEQ ID NO: 93) and reverse primer: 5'-TCCACAAATAGGTTGGCCAGAT-3' (SEQ ID NO: 94), BACE1 mRNA forward primer: 5'-AACGAATTGGCTTTGCTGTC ( SEQ ID NO:95) and reverse primer: 5'-AGGCTATGGTCATGAGGGTTG-3' (SEQ ID NO:96), forward primer: 5'-TGACTCTCTGCATGGTGGTG-3' (SEQ ID NO:97) and reverse primer for PS1 mRNA: 5'-TCAGAATTGAGTGCAGGGCT-3' (SEQ ID NO: 98), forward primer for PS2 mRNA: 5'-TTTGAGCCTCCCTTGACTGG-3' (SEQ ID NO: 99) and reverse primer: 5'-GGTATTCCAGTCCCGCTG-3' (SEQ ID NO: 100) for qRT-PCR. Quantification was tested using comparative threshold cycle measurements of the SYBR green fluorescence signal (Applied Biosystems from Thermo Fisher Scientific).

### Enzyme-linked immunosorbent assay (ELISA)

48 hours after transfection of HEK 293T cells and N2A cells, or 4.5 days after lentivirus infection of neurons, media and lysates were collected. Aβ40 and Aβ42 in the culture medium and cell lysates were detected by an Aβ ELISA kit as indicated in the protocol. (Invitrogen Technology; KHB3481 for human Aβ40, KHB3441 for human Aβ42 and KMB3481 for mouse Aβ40).

### Protein purification

The human γ-secretase complex was purified as previously described (Lu et al., 2014), and the cDNA encoding the human γ-secretase complex together with the expression vector referred to Lu et al., 2014.

Purification of human ApoE protein. 48 hours after transfection of cells with human ApoE plasmid (ApoE2-3xFLAG or ApoE2NT-3xFLAG), transfected cells were washed once in cold 1xPBS, harvested and centrifuged at 800 g. The cell pellet was then resuspended in 5 ml lysate buffer containing 25 mM HEPES, pH 7.4, 150 mM NaCl, 5 mM MgCl₂, 1 mM dithiothreitol (DTT), 0.05% (v/v) Tween-20, and 1× Protease Inhibitor Cocktail. Cell lysis was achieved by sonication and cell debris was separated by centrifugation (12,000 xg, 4°C, 15 minutes). The supernatant was incubated with anti-FLAGM2 affinity gel (SIGMA) at 4°C for 90 min. The resin was then washed 3 times with a buffer containing 25 mM HEPES, pH 7.4, 150 mM NaCl, 5 mM MgCl₂, 1 mM DTT and 0.05% (v/v) Tween-20. ApoE protein was eluted with a buffer containing 25 mM HEPES, pH 7.4, 150 mM NaCl and 500 µg/ml 3×FLAG peptide (SIGMA). SDS-PAGE and Western blot were used to visualize purified proteins.

Purification of β-CTF E. coli cells were transformed with human β-CTF-Myc-6×His plasmid, and when OD260 reached 0.6-0.8, E. coli cells were induced with 0.5 µM IPTG (Solarbio) at 37° C for 4 hours. E. coli cells were lysed in this buffer (30 mM Tris-HCl, pH 7.5, 150 mM NaCl, 1 mM EDTA) and sonicated on ice. The lysate was centrifuged at 25,000×g for 10 min, dissolved in urea buffer (30 mM Tris-HCl, pH 8.0, 300 mM NaCl, 1 mM EDTA, 6 M urea, 1% TritonX-100 and 1 mM CaCl₂, 1× protease inhibitor mixture) and rotated slowly for 3 hours. The resuspended lysate was centrifuged twice at 40,000 xg for 30 minutes, and the supernatant was incubated with Ni-NTA beads (GE healthcare) for 2 hours at room temperature. The above mixture was washed with 10 column volumes (CV) of urea buffer 1 (30 mM Tris-HCl, pH 8.0; 300 mM NaCl, 1 mM EDTA, 6 M urea, 1% TritonX-100, 1 mM CaCl₂, 1×cocktail), 20 CV of washing buffer 2 (30 mM Tris-HCl, pH 8.0, 300 mMNaCl, 0.1% TrionX-100) and 20CV of washing buffer 3 (50 mM Tris-HCl, pH 8.0, 300 mM NaCl, 0.1% Sharpsol). Bound material was eluted with wash buffer (50 mM Tris-HCl, pH 8.0, 300 mM NaCl, 0.1% CHAPSO) with an imidazole gradient of 0-300 mM. SDS-PAGE and Western blot were used to visualize purified proteins.

### Stereotaxic injection of virus

After being deeply anesthetized with isoflurane, mice (5 weeks old) were placed in a stereotaxic apparatus (RWD Instruments). The zero point (x, y, z, 0, 0, 0) is Bregma. Lentivirus (lentivirus-GFP, lentivirus-ApoE and lentivirus-ApoE-IRES-GFP) and AAV (AAV2/9-hSyn-EGFP-WPRE-pA and AAV2/9-hSyn-ApoECT; purchased from Shanghai Taitool Bioscience Co. Ltd.) were injected into the cortex (x, y, z, +/-1.6, 0, -1) or hippocampus (x, y, z, +/-1.5, -2, -1.5), injected 1ul on each injection site. The mice were then returned to their cages for rehabilitation after surgery.

### Quantification and statistical analysis

Data were analyzed by GraphPad Prism software (v6) using unpaired, paired, or one-sample t-tests. Aβ40 and Aβ42 levels were normalized to the corresponding GFP controls. α-CTF levels were normalized to FL-APP and corresponding GFP controls. All data are represented as mean ± SEM. Statistical significance was defined as p<0.05. One, two, three and four stars (*) in the graph indicate p-values <0.05, <0.01, <0.001 and <0.0001, respectively.

### 6. References

Aoki, K., Uchihara, T., Nakamura, A., Komori, T., Arai, N., and Mizutani, T. (2003). Expression of apolipoprotein E in ballooned neurons-comparative immunohistochemical study on neurodegenerative disorders and infarction. Acta Neuropathol 106, 436-440.
Armstrong, R.A. (2013). What causes alzheimer's disease? Folia Neuropathol 51, 169-188.
Belloy, M.E., Napolioni, V., and Greicius, M.D. (2019). A Quarter Century of APOE and Alzheimer's Disease: Progress to Date and the Path Forward. Neuron 101, 820-838.
Belonwu, S.A., Li, Y., Bunis, D., Rao, A.A., Solsberg, C.W., Tang, A., Fragiadakis, G.K., Dubal, D.B., Oskotsky, T., and Sirota, M. (2022). Sex-Stratified Single-Cell RNA-Seq Analysis Identifies Sex-Specific and Cell Type-Specific Transcriptional Responses in Alzheimer's Disease Across Two Brain Regions. Molecular neurobiology 59, 276-293T.
Brecht, W.J., Harris, F.M., Chang, S., Tesseur, I., Yu, G.Q., Xu, Q., Dee Fish, J., Wyss-Coray, T., Buttini, M., Mucke, L., et al. (2004). Neuron-specific apolipoprotein e4 proteolysis is associated with increased tau phosphorylation in brains of transgenic mice. The Journal of neuroscience: the official journal of the Society for Neuroscience 24, 2527-2534.
Bugiani, O., Giaccone, G., Rossi, G., Mangieri, M., Capobianco, R., Morbin, M., Mazzoleni, G., Cupidi, C., Marcon, G., Giovagnoli, A., et al. (2010). Hereditary cerebral hemorrhage with amyloidosis associated with the E693K mutation of APP. Archives of neurology 67, 987-995.
Chen, J., Li, Q., and Wang, J. (2011). Topology of human apolipoprotein E3 uniquely regulates its diverse biological functions. Proceedings of the National Academy of Sciences of the United States of America 108, 14813-14818.
Chen, Y., Wang, Y., Modrusan, Z., Sheng, M., and Kaminker, J.S. (2014). Regulation of neuronal gene expression and survival by basal NMDA receptor activity: a role for histone deacetylase 4. The Journal of neuroscience: the official journal of the Society for Neuroscience 34, 15327-15339.
Citron, M., Oltersdorf, T., Haass, C., McConlogue, L., Hung, A.Y., Seubert, P., Vigo-Pelfrey, C., Lieberburg, I., and Selkoe, D.J. (1992). Mutation of the beta-amyloid precursor protein in familial Alzheimer's disease increases beta-protein production. Nature 360, 672-674.
Citron, M., Teplow, D.B., and Selkoe, D.J. (1995). Generation of amyloid beta protein from its precursor is sequence specific. Neuron 14, 661-670.
Corder, E.H., Saunders, A.M., Strittmatter, W.J., Schmechel, D.E., Gaskell, P.C., Small, G.W., Roses, A.D., Haines, J.L., and Pericak-Vance, M.A. (1993). Gene dose of apolipoprotein E type 4 allele and the risk of Alzheimer's disease in late onset families. Science 261, 921-923.
De Strooper, B., Iwatsubo, T., and Wolfe, M.S. (2012). Presenilins and gamma-secretase: structure, function, and role in Alzheimer Disease. Cold Spring Harbor perspectives in medicine 2, a006304.
De Strooper, B., and Karran, E. (2016). The Cellular Phase of Alzheimer's Disease. Cell 164, 603-615.
De Strooper, B., Saftig, P., Craessaerts, K., Vanderstichele, H., Guhde, G., Annaert, W., Von Figura, K., and Van Leuven, F. (1998). Deficiency of presenilin-1 inhibits the normal cleavage of amyloid precursor protein. Nature 391, 387-390.
Dorszewska, J., Prendecki, M., Oczkowska, A., Dezor, M., and Kozubski, W. (2016). Molecular Basis of Familial and Sporadic Alzheimer's Disease. Current Alzheimer research 13, 952-963.
Eggert, S., Paliga, K., Soba, P., Evin, G., Masters, C.L., Weidemann, A., and Beyreuther, K. (2004). The proteolytic processing of the amyloid precursor protein gene family members APLP-1 and APLP-2 involves alpha-, beta-, gamma-, and epsilon-like cleavages: modulation of APLP-1 processing by n-glycosylation. J Biol Chem 279, 18146-18156.
Esch, F.S., Keim, P.S., Beattie, E.C., Blacher, R.W., Culwell, A.R., Oltersdorf, T., McClure, D., and Ward, P.J. (1990). Cleavage of amyloid beta peptide during constitutive processing of its precursor. Science 248, 1122-1124.
Güner, G., and Lichtenthaler, S.F. (2020). The substrate repertoire of γ-secretase/presenilin. Seminars in cell & developmental biology 105, 27-42.
Haass, C., Schlossmacher, M.G., Hung, A.Y., Vigo-Pelfrey, C., Mellon, A., Ostaszewski, B.L., Lieberburg, I., Koo, E.H., Schenk, D., Teplow, D.B., et al. (1992). Amyloid beta-peptide is produced by cultured cells during normal metabolism. Nature 359, 322-325.
Haass, C., and Selkoe, D.J. (1993). Cellular processing of beta-amyloid precursor protein and the genesis of amyloid beta-peptide. Cell 75, 1039-1042.
Han, S.H., Hulette, C., Saunders, A.M., Einstein, G., Pericak-Vance, M., Strittmatter, W.J., Roses, A.D., and Schmechel, D.E. (1994). Apolipoprotein E is present in hippocampal neurons without neurofibrillary tangles in Alzheimer's disease and in age-matched controls. Experimental neurology 128, 13-26.
Hardy, J., and Selkoe, D.J. (2002). The amyloid hypothesis of Alzheimer's disease: progress and problems on the road to therapeutics. Science 297, 353-356.
Hashimoto, T., Serrano-Pozo, A., Hori, Y., Adams, K.W., Takeda, S., Banerji, A.O., Mitani, A., Joyner, D., Thyssen, D.H., Bacskai, B.J., et al. (2012). Apolipoprotein E, especially apolipoprotein E4, increases the oligomerization of amyloid beta peptide. The Journal of neuroscience: the official journal of the Society for Neuroscience 32, 15181-15192.
Holtzman, D.M., Bales, K.R., Wu, S., Bhat, P., Parsadanian, M., Fagan, A.M., Chang, L.K., Sun, Y., and Paul, S.M. (1999). Expression of human apolipoprotein E reduces amyloid-beta deposition in a mouse model of Alzheimer's disease. The Journal of clinical investigation 103, R15-r21.
Huang, Y.A., Zhou, B., Wernig, M., and Sudhof, T.C. (2017). ApoE2, ApoE3, and ApoE4 Differentially Stimulate APP Transcription and Abeta Secretion. Cell 168, 427-441 e421.
Hung, V., Udeshi, N.D., Lam, S.S., Loh, K.H., Cox, K.J., Pedram, K., Carr, S.A., and Ting, A.Y. (2016). Spatially resolved proteomic mapping in living cells with the engineered peroxidase APEX2. Nature protocols 11, 456-475.
Huynh, T.V., Liao, F., Francis, C.M., Robinson, G.O., Serrano, J.R., Jiang, H., Roh, J., Finn, M.B., Sullivan, P.M., Esparza, T.J., et al. (2017). Age-Dependent Effects of apoE Reduction Using Antisense Oligonucleotides in a Model of beta-amyloidosis. Neuron 96, 1013-1023 e1014.
Jordà-Siquier, T., Petrel, M., Kouskoff, V., Smailovic, U., Cordelières, F., Frykman, S., Müller, U., Mulle, C., and Barthet, G. (2022). APP accumulates with presynaptic proteins around amyloid plaques: A role for presynaptic mechanisms in Alzheimer's disease? Alzheimer's & dementia: the journal of the Alzheimer's Association.
Kanekiyo, T., Xu, H., and Bu, G. (2014). ApoE and Abeta in Alzheimer's disease: accidental encounters or partners? Neuron 81, 740-754.
Karran, E., and De Strooper, B. (2016). The amyloid cascade hypothesis: are we poised for success or failure? Journal of neurochemistry 139 Suppl 2, 237-252.
Kumar-Singh, S., De Jonghe, C., Cruts, M., Kleinert, R., Wang, R., Mercken, M., De Strooper, B., Vanderstichele, H., Löfgren, A., Vanderhoeven, I., et al. (2000). Nonfibrillar diffuse amyloid deposition due to a gamma (42)-secretase site mutation points to an essential role for N-truncated A beta(42) in Alzheimer's disease. Human molecular genetics 9, 2589-2598.
Kwok, J.B., Li, Q.X., Hallupp, M., Whyte, S., Ames, D., Beyreuther, K., Masters, C.L., and Schofield, P.R. (2000). Novel Leu723Pro amyloid precursor protein mutation increases amyloid beta42(43) peptide levels and induces apoptosis. Ann Neurol 47, 249-253.
Li, Z., Shue, F., Zhao, N., Shinohara, M., and Bu, G. (2020). APOE2: protective mechanism and therapeutic implications for Alzheimer's disease. Molecular neurodegeneration 15, 63.
Liu, C.C., Zhao, N., Fu, Y., Wang, N., Linares, C., Tsai, C.W., and Bu, G. (2017). ApoE4 Accelerates Early Seeding of Amyloid Pathology. Neuron 96, 1024-1032 e1023.
Liu, P.P., Xie, Y., Meng, X.Y., and Kang, J.S. (2019). History and progress of hypotheses and clinical trials for Alzheimer's disease. Signal transduction and targeted therapy 4, 29.
Lu, P., Bai, X.C., Ma, D., Xie, T., Yan, C., Sun, L., Yang, G., Zhao, Y., Zhou, R., Scheres, S.H.W., et al. (2014). Three-dimensional structure of human γ-secretase. Nature 512, 166-170.
Metzger, R.E., LaDu, M.J., Pan, J.B., Getz, G.S., Frail, D.E., and Falduto, M.T. (1996). Neurons of the human frontal cortex display apolipoprotein E immunoreactivity: implications for Alzheimer's disease. Journal of neuropathology and experimental neurology 55, 372-380.
Mucke, L., and Selkoe, D.J. (2012). Neurotoxicity of amyloid beta-protein: synaptic and network dysfunction. Cold Spring Harb Perspect Med 2, a006338.
Murrell, J., Farlow, M., Ghetti, B., and Benson, M.D. (1991). A mutation in the amyloid precursor protein associated with hereditary Alzheimer's disease. Science 254, 97-99.
Oakley, H., Cole, S.L., Logan, S., Maus, E., Shao, P., Craft, J., Guillozet-Bongaarts, A., Ohno, M., Disterhoft, J., Van Eldik, L., et al. (2006). Intraneuronal beta-amyloid aggregates, neurodegeneration, and neuron loss in transgenic mice with five familial Alzheimer's disease mutations: potential factors in amyloid plaque formation. The Journal of neuroscience: the official journal of the Society for Neuroscience 26, 10129-10140.
Olsson, A., Höglund, K., Sjögren, M., Andreasen, N., Minthon, L., Lannfelt, L., Buerger, K., Möller, H.-J., Hampel, H., Davidsson, P., et al. (2003). Measurement of α- and β-secretase cleaved amyloid precursor protein in cerebrospinal fluid from Alzheimer patients. Experimental Neurology 183, 74-80.
Panza, F., Lozupone, M., Logroscino, G., and Imbimbo, B.P. (2019). A critical appraisal of amyloid-beta-targeting therapies for Alzheimer disease. Nature reviews Neurology 15, 73-88.
Prince, M. (2015). World Alzheimer report 2015: the global impact of dementia.
Ran, F.A., Hsu, P.D., Wright, J., Agarwala, V., Scott, D.A., and Zhang, F. (2013). Genome engineering using the CRISPR-Cas9 system. Nature protocols 8, 2281-2308.
Rhinn, H., Fujita, R., Qiang, L., Cheng, R., Lee, J.H., and Abeliovich, A. (2013). Integrative genomics identifies APOE epsilon4 effectors in Alzheimer's disease. Nature 500, 45-50.
Roberson, E.D., and Mucke, L. (2006). 100 years and counting: prospects for defeating Alzheimer's disease. Science 314, 781-784.
Roher, A.E., Maarouf, C.L., and Kokjohn, T.A. (2016). Familial Presenilin Mutations and Sporadic Alzheimer's Disease Pathology: Is the Assumption of Biochemical Equivalence Justified? J Alzheimers Dis 50, 645-658.
Schmechel, D.E., Saunders, A.M., Strittmatter, W.J., Crain, B.J., Hulette, C.M., Joo, S.H., Pericak-Vance, M.A., Goldgaber, D., and Roses, A.D. (1993). Increased amyloid beta-peptide deposition in cerebral cortex as a consequence of apolipoprotein E genotype in late-onset Alzheimer disease. Proc Natl Acad Sci U S A 90, 9649-9653.
Selkoe, D.J. (2001). Alzheimer's disease: genes, proteins, and therapy. Physiological reviews 81, 741-766.
Shen, J., and Kelleher, R.J., 3rd (2007). The presenilin hypothesis of Alzheimer's disease: evidence for a loss-of-function pathogenic mechanism. Proceedings of the National Academy of Sciences of the United States of America 104, 403-409.
Strittmatter, W.J., Saunders, A.M., Schmechel, D., Pericak-Vance, M., Enghild, J., Salvesen, G.S., and Roses, A.D. (1993). Apolipoprotein E: high-avidity binding to beta-amyloid and increased frequency of type 4 allele in late-onset familial Alzheimer disease. Proceedings of the National Academy of Sciences of the United States of America 90, 1977-1981.
Sun, L., Zhou, R., Yang, G., and Shi, Y. (2017). Analysis of 138 pathogenic mutations in presenilin-1 on the in vitro production of Aβ42 and Aβ40 peptides by γ-secretase. Proc Natl Acad Sci U S A 114, E476-e485.
Tomiyama, T., Nagata, T., Shimada, H., Teraoka, R., Fukushima, A., Kanemitsu, H., Takuma, H., Kuwano, R., Imagawa, M., Ataka, S., et al. (2008). A new amyloid beta variant favoring oligomerization in Alzheimer's-type dementia. Ann Neurol 63, 377-387.
Walker, E.S., Martinez, M., Brunkan, A.L., and Goate, A. (2005). Presenilin 2 familial Alzheimer's disease mutations result in partial loss of function and dramatic changes in Abeta 42/40 ratios. Journal of neurochemistry 92, 294-301.
Wang, C., Najm, R., Xu, Q., Jeong, D.E., Walker, D., Balestra, M.E., Yoon, S.Y., Yuan, H., Li, G., Miller, Z.A., et al. (2018). Gain of toxic apolipoprotein E4 effects in human iPSC-derived neurons is ameliorated by a small-molecule structure corrector. Nat Med 24, 647-657.
Wolfe, M.S., Xia, W., Ostaszewski, B.L., Diehl, T.S., Kimberly, W.T., and Selkoe, D.J. (1999). Two transmembrane aspartates in presenilin-1 required for presenilin endoproteolysis and gamma-secretase activity. Nature 398, 513-517.
Wu, S.-H., Li, X., Qin, D.-D., Zhang, L.-H., Cheng, T.-L., Chen, Z.-F., Nie, B.-B., Ren, X.-F., Wu, J., Wang, W.-C., et al. (2021). Induction of core symptoms of autism spectrum disorder by in vivo CRISPR/Cas9-based gene editing in the brain of adolescent rhesus monkeys. Science Bulletin 66, 937-946.
Xia, D., Watanabe, H., Wu, B., Lee, S.H., Li, Y., Tsvetkov, E., Bolshakov, V.Y., Shen, J., and Kelleher, R.J., 3rd (2015). Presenilin-1 knockin mice reveal loss-of-function mechanism for familial Alzheimer's disease. Neuron 85, 967-981.
Xia, Q., Yang, X., Shi, J., Liu, Z., Peng, Y., Wang, W., Li, B., Zhao, Y., Xiao, J., Huang, L., et al. (2021). The Protective A673T Mutation of Amyloid Precursor Protein (APP) in Alzheimer's Disease. 58, 4038-4050.
Xia, W. (2019). gamma-Secretase and its modulators: Twenty years and beyond. Neuroscience letters 701, 162-169.
Xu, P.-T., Gilbert, J.R., Qiu, H.-L., Ervin, J., Rothrock-Christian, T.R., Hulette, C., and Schmechel, D.E. (1999). Specific Regional Transcription of Apolipoprotein E in Human Brain Neurons. The American Journal of Pathology 154, 601-611.
Xu, Q., Bernardo, A., Walker, D., Kanegawa, T., Mahley, R.W., and Huang, Y. (2006). Profile and regulation of apolipoprotein E (ApoE) expression in the CNS in mice with targeting of green fluorescent protein gene to the ApoE locus. The Journal of neuroscience: the official journal of the Society for Neuroscience 26, 4985-4994.
Yang, G., Zhou, R., Guo, X., Yan, C., Lei, J., and Shi, Y. (2021). Structural basis of γ-secretase inhibition and modulation by small molecule drugs. Cell 184, 521-533.e514.
Yang, G., Zhou, R., Zhou, Q., Guo, X., Yan, C., Ke, M., Lei, J., and Shi, Y. (2019). Structural basis of Notch recognition by human γ-secretase. Nature 565, 192-197.
Yu, J.T., Tan, L., and Hardy, J. (2014). Apolipoprotein E in Alzheimer's disease: an update. Annu Rev Neurosci 37, 79-100.
Zalocusky, K.A., Najm, R., Taubes, A.L., Hao, Y., Yoon, S.Y., Koutsodendris, N., Nelson, M.R., Rao, A., Bennett, D.A., Bant, J., et al. (2021). Neuronal ApoE upregulates MHC-I expression to drive selective neurodegeneration in Alzheimer's disease. Nature neuroscience 24, 786-798.

## Claims

1. An isolated ApoE construct comprising an ApoE polypeptide, wherein the ApoE polypeptide:
(1) comprises a fragment of the amino acid sequence as shown by SEQ ID NO: 12, the first amino acid residue at the N-terminus of the fragment being an amino acid residue at any position between positions 215-221 of SEQ ID NO: 12, the last amino acid residue at the C-terminus of the fragment being an amino acid residue at any position between positions 274-299 (preferably 279-299) of SEQ ID NO: 12; or
(2) is of amino acid sequence having at least about 70% identity with the fragment of (1), and retains at least about 50% of the inhibition of the fragment of (1) against γ-secretase enzymatic digestion activity.

2. The isolated ApoE construct according to claim 1, wherein the ApoE polypeptide or the fragment from the amino acid sequence as shown by SEQ ID NO: 12 of (1) comprises any of the following sequences:
(i) the amino acid sequence of amino acid residues of positions 215-299 of SEQ ID NO: 12;
(ii) the amino acid sequence of amino acid residues of positions 216-299 of SEQ ID NO: 12;
(iii) the amino acid sequence of amino acid residues of positions 217-299 of SEQ ID NO: 12;
(iv) the amino acid sequence of amino acid residues of positions 218-299 of SEQ ID NO: 12;
(v) the amino acid sequence of amino acid residues of positions 219-299 of SEQ ID NO: 12;
(vi) the amino acid sequence of amino acid residues of positions 220-299 of SEQ ID NO: 12;
(vii) the amino acid sequence of amino acid residues of positions 221-299 of SEQ ID NO: 12;
(viii) the amino acid sequence of amino acid residues of positions 216-294 of SEQ ID NO: 12;
(ix) the amino acid sequence of amino acid residues of positions 216-289 of SEQ ID NO: 12;
(x) the amino acid sequence of amino acid residues of positions 219-289 of SEQ ID NO: 12;
(xi) the amino acid sequence of amino acid residues of positions 219-288 of SEQ ID NO: 12;
(xii) the amino acid sequence of amino acid residues of positions 219-287 of SEQ ID NO: 12;
(xiii) the amino acid sequence of amino acid residues of positions 219-286 of SEQ ID NO: 12;
(xiv) the amino acid sequence of amino acid residues of positions 219-285 of SEQ ID NO: 12;
(xv) the amino acid sequence of amino acid residues of positions 219-284 of SEQ ID NO: 12;
(xvi) the amino acid sequence of amino acid residues of positions 219-279 of SEQ ID NO: 12;
(xvii) the amino acid sequence of amino acid residues of positions 219-274 of SEQ ID NO: 12;
(xviii) the amino acid sequence of amino acid residues of positions 220-274 of SEQ ID NO: 12;
(xix) the amino acid sequence of amino acid residues of positions 220-279 of SEQ ID NO: 12;
(xx) the amino acid sequence of amino acid residues of positions 221-274 of SEQ ID NO: 12.

3. The isolated ApoE construct according to claim 1 or 2, wherein the ApoE polypeptide or the fragment from the amino acid sequence as shown by SEQ ID NO: 12 of (1) comprises any amino acid sequence as shown by SEQ ID NOs: 13, 18-34, 78 and 87.

4. The isolated ApoE construct according to any one of claims 1-3, wherein the ApoE polypeptide or the fragment from the amino acid sequence as shown by SEQ ID NO: 12 of (1) comprises any amino acid sequence as shown by SEQ ID NOs: 31-34, 78 and 87.

5. The isolated ApoE construct according to any one of claims 1-4, wherein the ApoE polypeptide comprises mutations at one or more amino acid positions compared to the amino acid sequence as shown by SEQ ID NO: 12: R226, D227, R228, L229, D230, Q235, E238, V239, R240, K242, E244, E245, Q246, A247, Q248, Q249, I250, R251, L252, Q253, A254, E255, Q275, V280, V287, T289, S290, A291, P293, V294, P295, S296, D297, N298, H299, R251+T289, R251+T289+A291, S290+P293+V294+P295+S296+D297+N298+H299, R226+D227+R228+L229+D230, E238+V239+R240+K242, or E244+E245+Q246+A247+Q248+Q249+I250+R251+L252+Q253+A254.

6. The isolated ApoE construct according to any one of claims 1-5, wherein the ApoE polypeptide comprises mutations at one or more amino acid positions compared to the amino acid sequence as shown by SEQ ID NO: 12: R226A, D227A, R228A, L229A, D230A, Q235A, E238A, V239A, R240A, K242A, E244del, E245del, Q246del, A247del, Q248del, Q249del, I250del, R251S, L252del, Q253del, A254del, E255A, Q275A, V280A, V287A, T289A, S290A, A291T, P293A, V294A, P295A, S296A, D297A, N298A , H299A, R251A+T289A, R251S+T289A+A291T, S290A+P293A+V294A+P295A+S296A+D297A+N298A+H299A, R226A+D227A+R228A+L229A+D230A, E238A+V239A+R240A+K242A, or E244del+ E245del+Q246del+A247del+Q248del+Q249del+I250del+R251del+L252del+Q253del+A 254del.

7. The isolated ApoE construct according to any one of claims 1-6, wherein the ApoE polypeptide comprises any one of the amino acid sequences as shown by SEQ ID NOs: 46-52, 57-59, 62 and 76.

8. The isolated ApoE construct according to any one of claims 1-7, the ApoE construct further comprises at the N-terminus of the ApoE polypeptide an amino acid sequence of amino acid residues of positions 1-167 or 1-205 or 1-214 of SEQ ID NO: 12, or a sequence having at least about 70% (e.g. at least 75%, 80%, 85%, 90%, 95%, 96%, 97%, 98%, 99%, or higher) identity with amino acids of positions 1-167, or 1-205, or 1-214 of SEQ ID NO: 12.

9. The isolated ApoE construct according to claim 8, wherein the ApoE construct comprises the amino acid sequence as shown by SEQ ID NO: 60 or 61.

10. The isolated ApoE construct according to any one of claims 1-9, wherein the ApoE construct further comprises a cell-penetrating peptide at the N-terminus or C-terminus of the ApoE polypeptide.

11. The isolated ApoE construct according to claim 10, wherein the cell-penetrating peptide comprises any one of the amino acid sequences as shown by SEQ ID NOs: 35-45, 63-65, and 84-86.

12. The isolated ApoE construct according to claim 10 or 11, wherein the cell-penetrating peptide comprises the amino acid sequence as shown by SEQ ID NO: 63 or 65.

13. The isolated ApoE construct according to any one of claims 10-12, wherein, the cell-penetrating peptide is linked to the ApoE polypeptide, or the amino acid sequence comprising amino acid residues of positions 1-167, 1-205, or 1-214 of SEQ ID NO: 12, or the sequence having at least about 70% identity with amino acids of positions 1-167 or 1-205 or 1-214 of SEQ ID NO: 12 by an adapter sequence.

14. The isolated ApoE construct according to any one of claims 1-13, wherein the ApoE construct further comprises a signal peptide at the N-terminus or C-terminus of the ApoE polypeptide.

15. The isolated ApoE construct according to claim 14, wherein the signal peptide comprising any one of the amino acid sequences selected from SEQ ID NOs: 66-68.

16. An isolated nucleic acid, wherein the isolated nucleic acid encodes the protein portion of the isolated ApoE construct of any one of claims 1-15.

17. A vector, wherein the vector comprises the isolated nucleic acid according to claim 16.

18. The vector according to claim 17, wherein the vector comprises a neuron-specific promoter at the 5' end of the isolated nucleic acid.

19. The vector according to claim 18, wherein the neuron-specific promoter is selected from: a synapsin promoter, a thy-1 promoter, and a calmodulin-dependent protein kinase II-alpha promoter.

20. The vector according to any one of claims 17-19, wherein the vector is a recombinant AAV or lentivirus expression vector.

21. A host cell, wherein the host cell expresses the isolated ApoE construct according to any one of claims 1-15, the isolated nucleic acid according to claim 16, or the vector according to any one of claims 17-20.

22. The host cell according to claim 21, wherein the host cell is a neuron.

23. A pharmaceutical composition, wherein the pharmaceutical composition comprises (i) the isolated ApoE construct according to any one of claims 1-15, the isolated nucleic acid according to claim 16, the vector according to any one of claims 17-20, or the host cell according to claim 21 or 22, and (ii) a pharmaceutically acceptable carrier.

24. Use of the isolated ApoE construct according to any one of claims 1-15, the isolated nucleic acid according to claim 16, the vector according to any one of claims 17-20, the host cell according to claims 21 or 22, or the pharmaceutical composition according to claim 23 in the manufacture of a medicament for treating or preventing a disease associated with γ-secretase enzymatic digestion activity in an individual.

25. A method for treating or preventing a disease associated with γ-secretase enzymatic digestion activity in an individual, comprises administering to the individual an effective dose of the isolated ApoE construct according to any one of claims 1-15, the isolated nucleic acid according to claim 16, the vector according to any one of claims 17-20, the host cell according to claims 21 or 22, or the pharmaceutical composition according to claim 23.

26. The use according to claim 24 or the method according to claim 25, wherein the medicament or the method inhibits the γ-secretase enzymatic digestion activity, thereby treating or preventing the disease related to γ-secretase enzymatic digestion activity.

27. The use or method according to any one of claims 24-26, wherein the disease is selected from the group consisting of: a neurodegenerative disease, cancer, inflammatory disease and renal disease.

28. The use or method according to any one of claims 24-27, wherein:
(i) the neurodegenerative disease is selected from the group consisting of: Alzheimer's disease and related diseases, amyloid cerebrovascular disease, Down's syndrome, and other neurodegenerative diseases associated with aging (such as forehead temporal lobe dementia);
(ii) the cancer is selected from the group consisting of: head and neck cancer, breast cancer, liver cancer, pancreatic cancer, ovarian cancer, lung cancer, glioma, fibroma, lymphoma, osteosarcoma, gastric cancer, and bladder cancer;
(iii) the inflammatory disease is selected from the group consisting of: asthma, pneumonia, and airway inflammation; and/or
(iv) the renal disease is selected from the group consisting of: acute kidney injury, clear cell renal cell carcinoma, renal fibrosis, and obstructive nephropathy.

29. The use or method according to any one of claims 24-28, wherein the individual is a human.
